# EUROPÄISCHE PATENTSCHRIFT

(11) **EP 0 528 762 B1**
(45) Veröffentlichungstag und Bekanntmachung des Hinweises auf die Patenterteilung: **07.05.1997**
(21) Anmeldenummer: 92810603.8
(22) Anmeldetag: 06.08.1992
(51) Int. Cl.: C07D 401/10, A61K 31/33, C07D 409/10, C07D 407/10, C07D 333/60, C07D 257/04

(54) **N-Acyl-N-Heterocyclyl- oder Naphthylalkyl-Aminosäuren als Angiotensin II Antagonisten**
N-acyl-N-heterocyclyl- or naphthyl-alkyl amino acids as angiotensin II antagonists
Acides aminés N-acyl-N-hétérocyclyl- ou naphtyl-alkyl comme angiotensine II antagonistes

(30) Priorität: 15.08.1991 CH 2405/91; 07.02.1992 CH 375/92; 08.04.1992 CH 1141/92
(43) Veröffentlichungstag der Anmeldung: 24.02.1993
(73) Patentinhaber: Novartis AG, 4058 Basel (CH)
(72) Erfinder: Schmidlin, Tibur, Dr., CH-4056 Basel (CH); Zbinden, Paul, CH-4108 Witterswil (CH); Bühlmayer, Peter, Dr., CH-4144 Arlesheim (CH)

(56) Entgegenhaltungen:
- EP-A- 0 429 257
- EP-A- 0 434 249
- EP-A- 0 443 983
- EP-A- 0 490 820

## Beschreibung

Die Erfindung betrifft N-Acyl-N-heterocyclylalkyl-aminosäuren der Formel 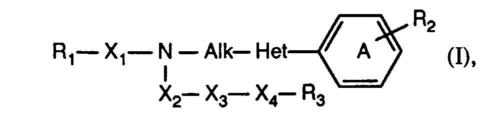 worin
R₁ C₁-C₇-Alkyl bedeutet, welches unsubstituiert oder durch Halogen oder Hydroxy substituiert ist, oder C₂-C₇-Alkenyl, C₃-C₇-Cycloalkyl, C₃-C₇-Cycloalkyloxy, C₁-C₇-Alkoxy oder C₃-C₇-Cycloalkyl-C₁-C₇-alkoxy bedeutet;
R₂ 1H-Tetrazol-5-yl, Carboxy, C₁-C₇-Alkoxy-carbonyl, SO₃H, PO₂H₂, PO₃H₂ oder Halogen-C₁-C₇-alkansulfonylamino ist;
R₃ 1H-Tetrazol-5-yl, Hydroxymethyl, C₁-C₇-Alkoxy-methyl, Formyl, Carboxy, C₁-C₇-Alkoxy Alkoxy-carbonyl, C₁-C₇-Alkoxy-C₁-C₇-alkoxy-carbonyl, Phenyl-C₁-C₄-alkoxy-carbonyl oder Carbamoyl bedeutet, dessen Aminogruppe unsubstituiert oder durch C₁-C₇-Alkyl, C₃-C₇-Alkenyl oder Phenyl-C₁-C₇-alkyl mono- oder unabhängig voneinander disubstituiert oder durch C₂-C₇-Alkylen oder C₂-C₄-Alkylenoxy-C₂-C₄-alkylen disubstituiert ist;
Alk Methylen, Ethylen oder Ethyliden ist;
Het 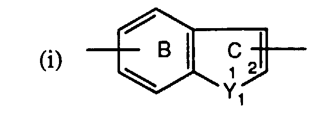 bedeutet, worin Y₁ für O, S oder N(R) steht und R Wasserstoff oder C₁-C₇-Alkyl bedeutet; oder 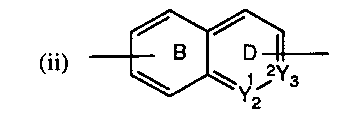 bedeutet, worin eine der Variablen Y₂ und Y₃ für C(R') und die andere für N steht oder beide Variablen jeweils für C(R') stehen; und R' Wasserstoff, Halogen, C₁-C₇-Alkyl, C₁-C₇-Alkoxy, C₂-C₇-Alkenyloxy, Phenoxy, Benzyloxy, Trifluormethyl oder S(O)ₘ-R bedeutet, worin m 0, 1 oder 2 ist; und R Wasserstoff oder C₁-C₇-Alkyl ist;
   X₁ für -CO- oder -S(O)ₘ- und der Index m für 0, 1 oder 2 steht;
   eine der Variablen X₂ und X₄ für C₁-C₄-Alkylen und die andere der Variablen X₂ und X₄ für eine Bindung steht; oder beide Variablen X₂ und X₄ jeweils für eine Bindung stehen;
   X₃ C₃-C₇-Cycloalkyliden oder das Strukturelement -C(Xₐ)(X_{b})- bedeutet und Xₐ Wasserstoff oder C₁-C₇-Alkyl ist und X_{b} C₁-C₇-Alkyl ist;
   und die Ringe A, B, C und D, bis auf die in der Formel angegebenen Substituenten, sowie aromatische Substituenten unabhängig voneinander unsubstituiert oder ein- oder mehrfach substituiert sind durch Substituenten ausgewählt aus der Gruppe bestehend aus Halogen, C₁-C₇-Alkyl, C₁-C₇-Alkoxy, C₂-C₇-Alkenyloxy, Phenoxy, Benzyloxy, Trifluormethyl und S(O)ₘ-R, worin m 0, 1 oder 2 ist und R Wasserstoff oder C₁-C₇-Alkyl ist;
   und ihre Salze; Verfahren zur Herstellung, pharmazeutische Präparate enthaltend eine Verbindung der Formel I oder ein pharmazeutisch verwendbares Salz davon sowie die Verwendung.

Die Verbindungen I können als, insbesondere pharmazeutisch verwendbare, Salze vorliegen. Weisen die Verbindungen I mindestens ein basisches Zentrum auf, können sie Säureadditionssalze bilden. Diese werden beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie gegebenenfalls, z.B. durch Halogen, substituierten C₁-C₄-Alkancarbonsäuren, z.B. Essigsäure, wie gegebenenfalls ungesättigten Dicarbonsäuren, z.B. Oxal-, Malon-, Bernstein-, Malein-, Fumar-, Phthal- oder Terephthalsäure, wie Hydroxycarbonsäuren, z.B. Ascorbin-, Glykol-, Milch-, Äpfel-, Wein- oder Zitronensäure, wie Aminosäuren, z.B. Asparagin- oder Glutaminsäure, oder wie Benzoesäure, oder mit organischen Sulfonsäuren, wie gegebenenfalls, z.B. durch Halogen, substituierten C₁-C₄-Alkan- oder Aryl-sulfonsäuren, z.B. Methan- oder p-Toluolsulfonsäure, gebildet. Entsprechende Säureadditionssalze können auch mit einem gegebenenfalls zusätzlich vorhandenen basischen Zentrum gebildet werden. Ferner können Verbindungen I mit mindestens einer aciden Gruppe (beispielsweise COOH oder 1H-Tetrazol-5-yl) Salze mit Basen bilden. Geeignete Salze mit Basen sind beispielsweise Metallsalze, wie Alkali- oder Erdalkalimetallsalze, z.B. Natrium-, Kalium- oder Magnesiumsalze, oder Salze mit Ammoniak oder einem organischen Amin, wie Morpholin, Thiomorpholin, Piperidin, Pyrrolidin, einem Mono-, Di- oder Triniederalkylamin, z. B. Ethyl-, tert.-Butyl-, Diethyl-, Diisopropyl-, Triethyl-, Tributyl- oder Dimethyl-propyl-amin, oder einem Mono-, Di- oder Trihydroxyniederalkylamin, z.B. Mono-, Di- oder Triethanolamin. Weiterhin können je nach Säure- und Basestärke der entsprechenden Gruppen gegebenenfalls innere Salze gebildet werden. Umfasst sind ferner für pharmazeutische Verwendungen nicht geeignete Salze, die beispielsweise für die Isolierung bzw. Reinigung von freien Verbindungen I oder deren pharmazeutisch verwendbaren Salzen eingesetzt werden können.

Die Ringe A, B, C und D, bis auf die in der Formel angegebenen Substituenten, sowie aromatische Substituenten, wie Phenoxy oder Benzyloxy, sind unabhängig voneinander unsubstituiert oder ein- oder in zweiter Linie mehrfach, z.B. zwei- oder dreifach, substituiert durch Substituenten ausgewählt aus der Gruppe bestehend aus Halogen, C₁-C₇-Alkyl, C₁-C₇-Alkoxy, C₂-C₇-Alkenyloxy, Phenoxy, Benzyloxy, Trifluormethyl und S(O)ₘ-R, worin der Index m 0, 1 oder 2 ist und R Wasserstoff oder C₁-C₇-Alkyl ist.

Het zugrundeliegende Heterocyclen sind Benzofuran, Benzo[b]thiophen, Indol, Chinolin und Isochinolin, Het bedeutet ebenso Naphthalin. Liegt für Het 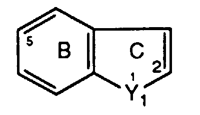 zugrunde, ist Het mit Alk über die Position 4, 6 oder 7, in erster Linie über die Position 5, und mit dem Phenylring A über die Position 3, in erster Linie über die Position 2, verbunden. Entsprechend kann der Ring C höchstens einen weiteren Substituenten aufweisen.

Liegt für Het 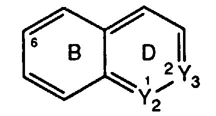 zugrunde, ist Het mit Alk über die Position 5, 7 oder 8, in erster Linie über die Position 6, und mit dem Phenylring A z.B. über die Position 3 oder 4, in erster Linie über die Position 2 (Y₃ = CR'), verbunden. Bedeutet Y₂ bzw. Y₃ CH (d.h. R'ist Wasserstoff), kann der Phenylring A über Y₂ bzw. in erster Linie über Y₃ mit dem Ring D verbunden sein.

Bevorzugtes Het ist 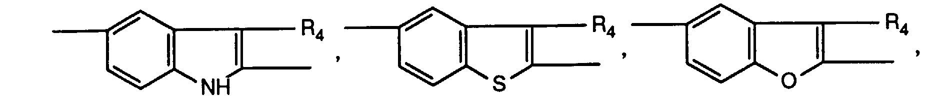 wobei R₄ Wasserstoff, Halogen, C₁-C₇-Alkyl, C₁-C₇-Alkoxy, C₂-C₇-Alkenyloxy, Phenoxy, Benzyloxy, Trifluormethyl oder S(O)ₘ-R, worin m 0, 1 oder 2 ist und R Wasserstoff oder C₁-C₇-Alkyl ist. Bevorzugtes R₄ ist Wasserstoff, Halogen, wie Brom, ferner C₁-C₇-Alkyl, C₁-C₇-Alkoxy oder Trifluormethyl; in erster Linie ist Het 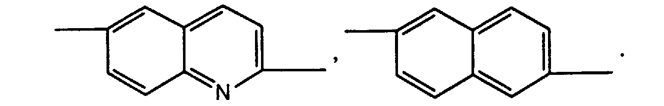 Verbindungen der Formel I, beispielsweise solche, worin Alk Ethyliden ist oder worin Xₐ und X_{b} unterschiedliche Bedeutungen haben, können je nach Anzahl, absoluter und relativer Konfiguration der asymmetrischen Kohlenstoffatome als reine Isomere, wie Antipoden und/oder Diastereomere, oder als Isomerengemische, wie Enantiomerengemische, z. B. Racemate, Diastereomerengemische oder Racematgemische, vorliegen;
entsprechende Formen sind ebenfalls Gegenstand der vorliegenden Erfindung.

Die vor- und nachstehend verwendeten Allgemeinbegriffe haben, sofern nicht abweichend definiert, die nachfolgend angegebenen Bedeutungen.

Der Ausdruck " Nieder" bedeutet, dass entsprechende Gruppen und Verbindungen jeweils 1 bis und mit 7, vorzugsweise 1 bis und mit 4, C-Atome enthalten.

C₁-C₇-Alkyl ist z.B. Methyl, Ethyl, n-Propyl, Isopropyl, n-Butyl, Isobutyl, sek.-Butyl, tert.-Butyl oder ein entsprechender Pentyl-, Hexyl- oder Heptylrest. Bevorzugt ist C₁-C₄-Alkyl.

C₁-C₇-Alkyl, welches durch Halogen substituiert ist, enthält ein oder mehrere Halogen-atom(e) und ist z.B. Trifluormethyl, 2-Chlor-1,1,2-trifluor-ethyl, Chlormethyl, 3,3,3-Trifluorpropyl, 4-Chlorbutyl oder Heptafluorpropyl. Bevorzugt ist Halogen-C₁-C₄-alkyl.

C₁-C₇-Alkyl, welches durch Hydroxy substituiert ist, ist in erster Linie einfach durch Hydroxy substituiert und bedeutet z.B. Hydroxymethyl, 2-Hydroxyethyl, 3-Hydroxypropyl oder 4-Hydroxybutyl. Bevorzugt ist Hydroxy-C₁-C₄-alkyl.

C₂-C₇-Alkenyl ist z.B. Vinyl, Propen-2-yl, Allyl oder But-1-en-3-yl,-1-en-4-yl, -2-en-1-yl oder -2-en-2-yl. Bevorzugt ist C₃-C₇-Alkenyl, in erster Linie C₃-C₅-Alkenyl.

C₃-C₇-Cycloalkyl ist Cyclopropyl, Cyclobutyl, Cyclopentyl, Cyclohexyl oder Cycloheptyl. Bevorzugt ist Cyclopropyl.

C₃-C₇-Cycloalkyloxy ist Cyclopropyloxy, Cyclobutyloxy, Cyclopentyloxy, Cyclohexyloxy oder Cycloheptyloxy. Bevorzugt ist Cyclopropyloxy und Cyclopentyloxy.

C₁-C₇-Alkoxy ist z.B. Methoxy, Ethoxy, n-Propyloxy, Isopropyloxy, n-Butyloxy, sek-Butyloxy oder tert-Butyloxy oder entsprechendes Pentyloxy, Hexyloxy oder Heptyloxy. Bevorzugt ist C₁-C₄-Alkoxy.

C₃-C₇-Cycloalkyl-C₁-C₇-alkoxy ist z.B. C₃-C₇-Cycloalkyl-C₁-C₂-alkoxy, wie Cyclopropyl-methoxy oder -ethoxy, Cyclobutyl-methoxy oder -ethoxy, Cyclopentyl-methoxy oder -ethoxy, Cyclohexyl-methoxy oder -ethoxy oder Cycloheptyl-methoxy oder -ethoxy. Bevorzugt ist Cyclopropylmethoxy.

C₁-C₇-Alkoxy-carbonyl ist z.B. Methoxy-, Ethoxy-, n-Propyloxy-, Isopropyloxy-, n-Butyloxy- oder tert-Butyloxy-carbonyl. Bevorzugt ist C₁-C₄-Alkoxycarbonyl.

Halogen ist insbesondere Halogen mit einer Atomnummer bis und mit 35, d. h. Fluor, Chlor oder Brom, und umfasst ferner Iod.

Halogen-C₁-C₇-alkansulfonylamino ist z.B. Trifluormethan-, Difluormethan-, 1, 1,2-Trifluorethan- oder Heptafluorpropan-sulfonylamino. Bevorzugt ist Halogen-C₁-C₄-alkansulfonylamino.

C₁-C₇-Alkoxy-C₁-C₇-alkoxycarbonyl ist insbesondere C₁-C₄-Alkoxy-C₁-C₄-alkoxy-carbonyl, wie 2-Methoxy-ethoxy-carbonyl, 2-Ethoxy-ethoxy-carbonyl, 3-Methoxy-propyloxycarbonyl oder 3-Ethoxy-propyloxy-carbonyl.

Phenyl-C₁-C₄-alkoxycarbonyl ist insbesondere Benzyloxy-, 1- oder 2-Phenylethoxy-carbonyl. Bevorzugt ist Phenyl-C₁-C₂-alkoxycarbonyl.

Phenyl-C₁-C₇-alkyl ist z. B. Benzyl oder 1- oder 2-Phenethyl. Bevorzugt ist insbesondere Phenyl-C₁-C₄-alkyl.

C₂-C₇-Alkylen ist geradkettig oder verzweigt und bedeutet insbesondere Eth-1,2-ylen, Prop-1,3-ylen, But-1,4-ylen, Pent-1,5-ylen, Prop-1,2-ylen, 2-Methylprop-1,3-ylen oder 2,2-Dimethylprop-1,3-ylen. Bevorzugt ist C₂-C₅-Alkylen.

C₂-C₄-Alkylenoxy ist z.B. Allyloxy, But-2-en-1-yloxy oder But-3-en-1-yloxy.

C₂-C₄-Alkylenoxy-C₂-C₄-alkylen ist bevorzugt Ethylenoxyethylen.

C₁-C₄-Alkylen ist z.B. Methylen, Ethylen, Propylen oder Butylen.

C₃-C₇-Cycloalkyliden ist Cyclopropyliden, Cyclobutyliden, Cyclopentyliden, Cyclohexyliden oder Cycloheptyliden. Bevorzugt sind Cyclopentyliden und Cyclohexyliden.

C₂-C₇-Alkenyloxy ist z.B. Allyloxy oder But-2-en- oder But-3-enyloxy. Bevorzugt ist C₃-C₅-Alkenyloxy.

Vorzugsweise sind ungesättigte Reste nicht über das Atom gebunden, von dem die Mehrfachbindung ausgeht.

Ausgedehnte pharmakologische Untersuchungen haben ergeben, dass die Verbindungen I und ihre pharmazeutisch verwendbaren Salze z. B. ausgeprägte Angiotensin-II-antagonisierende Eigenschaften aufweisen.

Bekanntlich hat Angiotensin-II starke vasokonstriktorische Eigenschaften und stimuliert ausserdem die Aldosteronsekretion und bewirkt somit eine deutliche Natrium/Wasser-Retention. Die Folge der Angiotensin-II-Aktivität manifestiert sich unter anderem in einer Erhöhung des Blutdrucks.

Die Bedeutung von Angiotensin-II-Antagonisten besteht darin, durch kompetitive Hemmung der Bindung von Angiotensin-II an die Rezeptoren die durch Angiotensin-II bewirkten vasokonstriktorischen und die Aldosteronsekretion-stimulierenden Effekte zu unterdrücken.

Die Angiotensin-II-antagonisierenden Eigenschaften der Verbindungen I und ihrer pharmazeutisch verwendbaren Salze können im Angiotensin-II-Bindungstest erfasst werden. Dabei werden glatte Muskelzellen der Ratte aus homogenisierter Rattenaorta verwendet. Das feste Zentrifugat wird in 50 mM Tris-Puffer (pH 7.4) unter Einsatz von Peptidaseinhibitoren suspendiert. Die Proben werden 60 Minuten bei 25°C mit ¹²⁵I-Angiotensin-II (0.175 nM) und einer variierenden Konzentration an Angiotensin-II oder an Testsubstanz inkubiert. Die Inkubation wird dann durch Zugabe von mit eiskaltem Phosphat gepuffertem Kochsalz beendet und es wird durch Whatman GF/F Filter filtriert. Die γ-Strahlungsaktivität der Filter wird mit einem Gamma-Zähler gezählt. Aus der Dosis-Wirkungs-Kurve werden die IC₅₀-Werte bestimmt. Für die Verbindungen I und ihre pharmazeutisch verwendbaren Salze werden IC₅₀-Werte ab etwa 10 nM ermittelt.

Zur Bestimmung der Angiotensin-II induzierten Vasokonstriktion können Untersuchungen an dem isolierten Kaninchen-Aortaring herangezogen werden. Hierzu werden von jeder Brust Aortaringe präpariert und zwischen 2 parallelen Klammern bei einer anfänglich bestehenden Spannung von 2 g fixiert. Anschliessend werden die Ringe bei 37°C in 20 ml eines Gewebebades getaucht und mit einem Gemisch aus 95 % O₂ und 5 % CO₂ begast. Die isometrischen Reaktionen werden gemessen. In 20-minütigen Intervallen werden die Ringe abwechselnd mit 10 nM Angiotensin-II (Hypertensin-CIBA) und 5 nM Noradrenalinchlorid stimuliert. Anschliessend werden die Ringe mit ausgewählten Konzentrationen der Testsubstanzen vor der Behandlung mit den Agonisten inkubiert. Die Daten werden mit einem Buxco Digitalcomputer analysiert. Die Konzentrationen, die eine 50%-ige Hemmung der Anfangskontrollwerte bewirken, werden als IC₅₀-Werte angebeben. Für die Verbindungen I und ihre pharmazeutisch verwendbaren Salze werden IC₅₀-Werte ab etwa 10 nM bestimmt.

Dass die Verbindungen I und ihre pharmazeutisch verwendbaren Salze durch Angiotensin-II induzierten Bluthochdruck reduzieren können, kann im Testmodell der normotensiven, narkotisierten und despinalisierten Ratte verifiziert werden. Zwei Stunden nach Behandlung mit der Testsubstanz wird die Ratte narkotisiert, und der Blutdruck wird direkt in der Halsschlagader gemessen und mit einem on-line Datenerfassungssystem aufgezeichnet (Buxco). Noradrenalin (1 µg/kg i.v.) bzw. Angiotensin-II (0.4 µg/kg i.v.) werden durch Bolusinjektion intravenös injiziert. Die Spezifität des Angiotensin-II-Antagonismus wird angezeigt durch die selektive Hemmung des von Angiotensin-II, nicht aber des durch Noradrenalin hervorgerufenen Druckeffektes. In diesem Testmodell zeigen die Verbindungen I und ihre pharmazeutisch verwendbaren Salze ab einer Dosis von etwa 3 mg/kg p.o. einen hemmenden Effekt.

Auch im Testmodell der renalen hypertensiven Ratte kann die antihypertensive Aktivität der Verbindungen I und ihrer pharmazeutisch verwendbaren Salze manifestiert werden. Bei männlichen Ratten wird durch Verengung einer renalen Arterie gemäss der Goldblatt-Methode Bluthochdruck erzeugt. Den Ratten werden mittels einer Magensonde Dosen der Testsubstanz verabreicht. Kontrolltiere erhalten ein äquivalentes Volumen an Lösungsmittel. Blutdruck und Herzschlag werden indirekt an wachen Tieren nach der Schwanzklemm-Methode von Gerold et al. [Helv. Physiol. Acta 24 (1966), 58] vor Verabreichung der Testsubstanz bzw. des Lösungsmittels sowie während des Verlaufs der Experimente in Intervallen gemessen. Der ausgeprägte antihypertensive Effekt kann ab einer Dosis von etwa 30 mg/kg p.o. nachgewiesen werden.

Dementsprechend können die Verbindungen der Formel I und ihre Salze erfindungsgemäss bei der Prophylaxe und insbesondere der Behandlung von Krankheitserscheinungen eingesetzt werden, die durch Angiotensin II beeinflusst bzw. verursacht werden können.

Die erfindungsgemässen Verbindungen können ferner zur Behandlung von Glaukom bzw. zur Reduktion des Augenüberdruckes und zur Förderung des retinalen Blutflusses verwendet werden. Weitere Verwendungsmöglichkeiten der Angiotensin-II-Antagonisten der Formel I und ihrer Salze bestehen in der Behandlung von sekundärem Hyperaldosteronismus, diabetischer Neuropathie bzw. diabetischer Retinopathie, akutem bzw. chronischem Nierenversagen, Schlaganfall, erhöhtem Harnsäurespiegel, Antiangiogenese, myokardischer Ischaemi (Angina), Myokardinfarkt, Hypertrophie des linken Ventrikels, myokardischer Fibrose, Insuffizienz der Aorta, Alzheimer Krankheit, Wahrnehmungsdisfunktion, Lernschwierigkeiten, senile Demenz, schizophrene Polydipsia, Depressionen, gastrointestinaler Motilität, Magensekretion, intestinaler Absorption sowie zur Prophylaxe und zur Behandlung von Restenose nach percutaneous transluminal coronary angioplasy (PTCA).

Dementsprechend können die Verbindungen I und ihre pharmazeutisch verwendbaren Salze z.B. als Wirkstoffe in Antihypertensiva verwendet werden, welche z.B. zur Behandlung von Bluthochdruck sowie von Herzinsuffizienz, und ebenfalls zur Behandlung von Glaukom bzw. zur Reduktion des Augenüberdruckes und zur Förderung des retinalen Blutflusses eingesetzt werden. Ein Erfindungsgegenstand ist somit die Verwendung der Verbindungen I und ihrer pharmazeutisch verwendbaren Salze zur Herstellung von entsprechenden Arzneimitteln und zur therapeutischen Behandlung von Erkrankungen, die durch Angiotensin II beeinflusst bzw. verursacht werden, insbesondere von Bluthochdruck sowie von Herzinsuffizienz und ebenfalls zur Behandlung von Glaukom bzw. zur Reduktion des Augenüberdruckes und zur Förderung des retinalen Blutflusses. Bei der Herstellung der Arzneimittel ist auch die gewerbsmässige Herrichtung der Wirksubstanzen eingeschlossen.

Bevorzugt sind Verbindungen Formel I, worin R₁ C₁-C₇-Alkyl bedeutet, welches unsubstituiert oder durch Halogen oder Hydroxy substituiert ist, oder C₂-C₇-Alkenyl, C₃-C₇-Cycloallkyl oder C₁-C₇-Alkoxy bedeutet; und ihre Salze.

Bevorzugt sind Verbindungen der Formel I, worin
R₁ C₂-C₇-Alkyl ist oder C₁-C₇-Alkyl bedeutet, welches durch Halogen oder Hydroxy substituiert ist, oder C₃-C₇-Alkenyl, C₃-C₆-Cycloalkyl oder C₁-C₇-Alkoxy bedeutet;
R₂ 1H-Tetrazol-5-yl, Carboxy, C₁-C₄-Alkoxy-carbonyl oder Halogen-C₁-C₄-alkansulfonylamino ist;
R₃ 1H-Tetrazol-5-yl, Carboxy, C₁-C₄-Alkoxy-carbonyl, C₁-C₄-Alkoxy-C₁-C₄-alkoxy-carbonyl, Phenyl-C₁-C₄-alkoxycarbonyl oder Carbamoyl bedeutet, dessen Aminogruppe durch C₁-C₄-Alkyl mono- oder unabhängig voneinander disubstituiert oder durch C₄-C₆-Alkylen oder Ethylenoxyethylen disubstituiert ist;
Alk Methylen, Ethylen oder Ethyliden ist;
Het 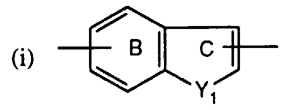 bedeutet, worin Y₁ für O, S oder N(R) steht und R Wasserstoff oder C₁-C₄-Alkyl bedeutet;
   oder 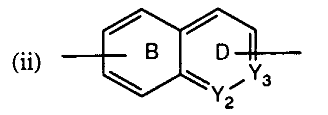 bedeutet, worin eine der Variablen Y₂ und Y₃ für CH und die andere für N steht oder beide Variablen jeweils für CH stehen;
   X₁ für -CO- oder -S(O)ₘ- und der Index m für 0, 1 oder 2 steht;
   eine der Variablen X₂ und X₄ für C₁-C₄-Alkylen und die andere der Variablen X₂ und X₄ für eine Bindung steht; oder beide Variablen X₂ und X₄ jeweils für eine Bindung stehen;
   X₃ C₃-C₆-Cycloalkyliden oder das Strukturelement -C(Xₐ)(X_{b})- bedeutet und Xₐ Wasserstoff oder C₁-C₇-Alkyl und X_{b} C₁-C₇-Alkyl bedeuten;
   und die Ringe A, B, C und D, bis auf die in der Formel angegebenen Substituenten, sowie aromatische Substituenten unabhängig voneinander unsubstituiert oder ein- oder mehrfach substituiert sind durch Substituenten ausgewählt aus der Gruppe bestehend aus Halogen, C₁-C₄-Alkyl, C₁-C₄-Alkoxy, C₃-C₅-Alkenyloxy, Phenoxy, Benzyloxy, Trifluormethyl und S(O)ₘ-R, worin m 0, 1 oder 2 ist und R Wasserstoff oder C₁-C₄-Alkyl ist;
   und ihre Salze.

Bevorzugt sind Verbindungen der Formel I, worin
R₁ C₂-C₇-Alkyl ist oder C₁-C₄-Alkyl bedeutet, welches durch Halogen oder Hydroxy substituiert ist, oder C₃-C₇-Alkenyl, C₃-C₆-Cycloalkyl oder C₁-C₇-Alkoxy bedeutet;
R₂ 1H-Tetrazol-5-yl, Carboxy oder C₁-C₄-Alkoxy-carbonyl ist;
R₃ 1H-Tetrazol-5-yl, Carboxy, C₁-C₄-Alkoxy-carbonyl, C₁-C₄-Alkoxy-C₁-C₄-alkoxy-carbonyl oder Phenyl-C₁-C₂-alkoxycarbonyl bedeutet;
Alk Methylen, ferner Ethylen oder Ethyliden ist;
Het 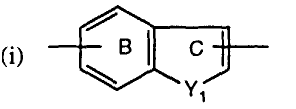 bedeutet, worin Y₁ für O, S oder NH steht; oder 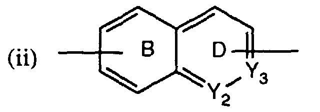 bedeutet, worin eine der Variablen Y₂ und Y₃ für CH und die andere für N steht oder beide Variablen jeweils für CH stehen;
   in erster Linie 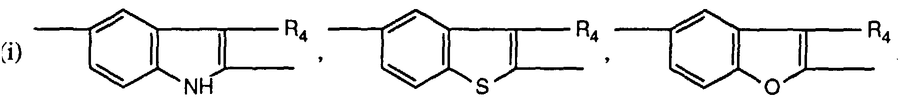 bedeutet und R₄ Wasserstoff, Halogen, wie Brom, ferner C₁-C₇-Alkyl, C₁-C₇-Alkoxy oder Trifluormethyl bedeutet; oder 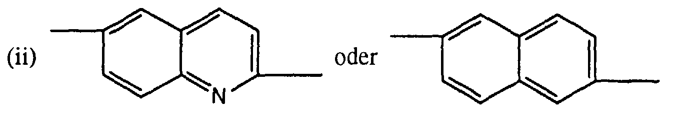 bedeutet,
   X₁ für -CO- steht;
   eine der Variablen X₂ und X₄ für C₁-C₂-Alkylen und die andere der Variablen X₂ und X₄ für eine Bindung steht; oder beide Variablen X₂ und X₄ jeweils für eine Bindung stehen;
   X₃ C₅-C₆-Cycloalkyliden oder das Strukturelement -C(Xₐ)(X_{b})- bedeutet und Xₐ Wasserstoff oder C₁-C₅-Alkyl und X_{b} C₁-C₅-Alkyl bedeuten;
   in erster Linie

(i) X₂ C₁-C₂-Alkylen ist; X₄ für eine Bindung steht; X₃ C₅-C₆-Cycloalkyliden bedeutet;
   oder
(ii) X₂ und X₄ jeweils für eine Bindung stehen; und X₃ für das Strukturelement -C(Xₐ)(X_{b})- steht und Xₐ Wasserstoff oder C₁-C₅-Alkyl und X_{b} C₁-C₅-Alkyl bedeuten; und die Ringe A, B, C und D, bis auf die in der Formel angegebenen Substituenten, unabhängig voneinander unsubstituiert oder ein- oder mehrfach substituiert sind durch Substituenten ausgewählt aus der Gruppe bestehend aus Halogen, C₁-C₄-Alkyl, C₁-C₄-Alkoxy und Trifluormethyl;
und ihre Salze.

Bevorzugt sind Verbindungen der Formel jeweils der vorstehend definierten Art, worin eine der Variablen Y₂ und Y₃ für C(R') steht und die andere für N oder C(R') steht, wobei R' Halogen, C₁-C₄-Alkyl, C₁-C₄-Alkoxy oder Trifluormethyl bedeutet, und ihre Salze.

Bevorzugt sind Verbindungen der Formel I, worin
R₁ C₂-C₇-Alkyl, wie n-Propyl oder n-Butyl, oder C₃-C₆-Cycloalkyl, wie Cyclopropyl, oder C₁-C₄-Alkoxy, wie Methoxy, Ethoxy, Propyloxy oder Butyloxy, bedeutet;
R₂ 1H-Tetrazol-5-yl, Carboxy oder C₁-C₄-Alkoxy-carbonyl, wie Methoxy- oder Ethoxy-carbonyl, ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 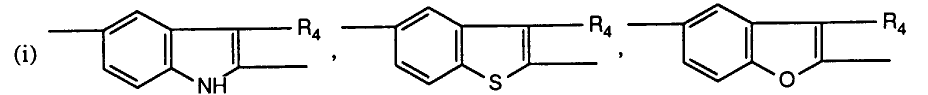 bedeutet und R₄ Wasserstoff, Halogen, wie Brom, ferner C₁-C₄-Alkyl, wie Methyl, C₁-C₄-Alkoxy, wie Methoxy, oder Trifluormethyl bedeutet; oder 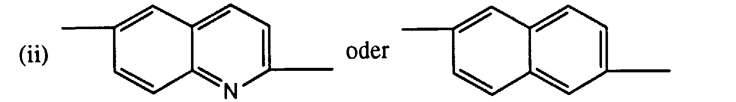 bedeutet;
   X₁ für -CO- steht;
   (i) X₂ C₁-C₂-Alkylen, insbesondere Methylen, ist; X₄ für eine Bindung steht; X₃ C₅-C₆-Cycloalkyliden bedeutet; oder
   (ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie Ethyl oder Isopropyl, bedeuten;
und ihre Salze.

Bevorzugt sind Verbindungen der Formel und ihre Salze jeweils der vorstehend definierten Art, worin R₃ Hydroxymethyl, C₁-C₄-Alkoxy-methyl oder Formyl bedeutet;
die übrigen Variablen die jeweils vorstehend angegebenen Bedeutungen haben.

Bevorzugt sind Verbindungen der Formel I, worin
R₁ C₂-C₅-Alkyl, wie n-Propyl oder n-Butyl;
R₂ 1H-Tetrazol-5-yl oder Carboxy ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 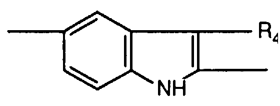 bedeutet und R₄ Wasserstoff, Halogen, wie Brom, oder C₁-C₄-Alkyl, wie Methyl, ist;
   X₁ für -CO- steht;
   (i) X₂ C₁-C₂-Alkylen, insbesondere Methylen, ist; X₄ für für eine Bindung steht; X₃ C₅-C₆-Cycloalkyliden bedeutet; oder
   (ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie Ethyl, Isopropyl oder 3-Butyl, bedeuten;
und ihre Salze.

Bevorzugt sind Verbindungen der Formel I, worin
R₁ C₂-C₅-Alkyl, wie n-Propyl oder n-Butyl, bedeutet;
R₂ 1H-Tetrazol-5-yl oder Carboxy ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 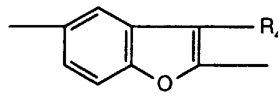 bedeutet und R₄ Wasserstoff, Halogen, wie Brom, oder C₁-C₄-Alkyl, wie Methyl, ist;
   X₁ für -CO- steht;
   (i) X₂ C₁-C₂-Alkylen, insbesondere Methylen, ist; X₄ für für eine Bindung steht; X₃ C₅-C₆-Cycloakyliden bedeutet; oder
   (ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie Ethyl, Isopropyl oder 3-Butyl, bedeuten;
und ihre Salze.

Bevorzugt sind Verbindungen der Formel I, worin
R₁ C₂-C₅-Alkyl, wie n-Propyl oder n-Butyl, bedeutet;
R₂ 1H-Tetrazol-5-yl oder Carboxy ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 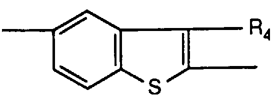 bedeutet, worin R₄ Wasserstoff, Halogen, wie Brom, oder C₁-C₄-Alkyl, wie Methyl, ist;
   X₁ für -CO- steht;
   (i) X₂ C₁-C₂-Alkylen, insbesondere Methylen, ist; X₄ für für eine Bindung steht; X₃ C₅-C₆-Cycloalkyliden, wie Cyclopentyliden oder Cyclohexyliden, bedeutet; oder
   (ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie Ethyl, Isopropyl oder 3-Butyl, bedeuten;
und ihre Salze.

Bevorzugt sind Verbindungen der Formel I, worin
R₁ C₂-C₅-Alkyl, wie n-Propyl oder n-Butyl, bedeutet;
R₂ 1H-Tetrazol-5-yl oder Carboxy ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 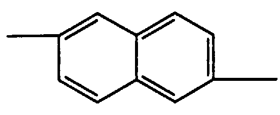 bedeutet;
   X₁ für -CO- steht;
   (i) X₂ C₁-C₂-Alkylen, insbesondere Methylen, ist; X₄ für für eine Bindung steht; X₃ C₅-C₆-Cycloalkyliden bedeutet; oder
   (ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie, Ethyl, Isopropyl oder 3-Butyl, bedeuten;
und ihre Salze.

Bevorzugt sind Verbindungen der Formel I, worin
R₁ C₂-C₅-Alkyl, wie n-Propyl oder n-Butyl, bedeutet;
R₂ 1H-Tetrazol-5-yl oder Carboxy ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 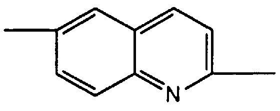 bedeutet;
   X₁ für -CO- steht;
   (i) X₂ C₁-C₂-Alkylen, insbesondere Methylen, ist; X₄ für für eine Bindung steht; X₃ C₅-C₆-Cycloalkyliden bedeutet; oder
   (ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie Ethyl, Isopropyl oder 3-Butyl, bedeuten;
und ihre Salze.

Bevorzugt sind Verbindungen der Formel I, worin
R₁ C₂-C₅-Alkyl, wie Ethyl, n-Propyl oder n-Butyl, bedeutet;
R₂ 1H-Tetrazol-5-yl oder Carboxy ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 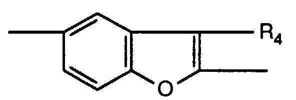 bedeutet; und R₄ Wasserstoff oder Halogen mit Atomnummer bis und mit 35, wie Brom, ist;
   X₁ für -CO- steht;
   (i) X₂ C₁-C₂-Alkylen, insbesondere Methylen, ist; X₄ für für eine Bindung steht; X₃ C₅-C₆-Cycloalkyliden bedeutet; oder
   (ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie Ethyl, Isopropyl oder 3-Butyl, bedeuten;
und ihre Salze.

Namentlich bevorzugt sind die in den Beispielen genannten Verbindungen der Formel I, in freier Form oder in Salzform.

Ein weiterer Gegenstand der Erfindung ist ein Verfahren zur Herstellung der Verbindungen I und ihrer Salze, z.B. dadurch gekennzeichnet, dass man
a) in einer Verbindung der Formel 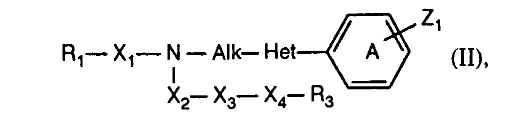 worin Z₁ einen in R₂ überführbaren Rest bedeutet, oder einem Salz davon, Z₁ in R₂ überführt; oder
b) eine Verbindung der Formel 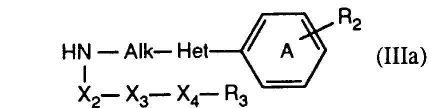 mit einer Verbindung der Formel R₁-X₁-OH (IIIb), einem reaktionsfähigen Derivat davon oder ein Salz davon, umsetzt;
und jeweils, wenn erwünscht, eine verfahrensgemäss oder auf andere Weise erhältliche Verbindung I, in freier Form oder in Salzform, isoliert, eine verfahrensgemäss oder auf andere Weise erhältliche Verbindung I in eine andere Verbindung I überführt, ein verfahrensgemäss erhältliches Gemisch von Isomeren auftrennt und das gewünschte Isomere isoliert und/oder eine verfahrensgemäss erhältliche freie Verbindung I in ein Salz oder ein verfahrensgemäss erhältliches Salz einer Verbindung I in die freie Verbindung I oder in ein anderes Salz überführt.

Salze von Ausgangsmaterialien, die mindestens ein basisches Zentrum aufweisen, sind entsprechende Säureadditionssalze, während Salze von Ausgangsstoffen, die mindestens eine acide Gruppe aufweisen, Salze mit Basen sind, jeweils wie in Zusammenhang mit entsprechenden Salzen von Verbindungen I vorstehend aufgeführt.

In die Variable R₂ überführbare Reste Z₁ stellen beispielsweise Cyano, Mercapto, Halogen, die Gruppe -N₂⁺ A⁻, in der A⁻ ein von einer Säure abgeleitetes Anion bedeutet, wie ein Halogenid, Amino sowie von COOH, SO₃H, PO₃H₂ oder PO₂H₂ verschiedene funktionell abgewandelte Formen sowie N-geschütztes 1H-Tetrazol-5-yl.

Reaktionsfähige Derivate von Verbindungen der Formel IIIb sind beispielsweise davon abgeleitete aktivierte Ester oder reaktionsfähige Anhydride, ferner reaktionsfähige cyclische Amide.

Die vor- und nachstehend in den Varianten beschriebenen Umsetzungen werden in an sich bekannter Weise durchgeführt, z.B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -80°C bis zur Siedetemperatur des Reaktionsmediums, vorzugsweise von etwa -10° bis etwa +200°C, und, falls erforderlich, in einem geschlossenen Gefäss, unter Druck, in einer Inertgasatmosphäre und/oder unter wasserfreien Bedingungen arbeitet. Einzelheiten zu entsprechenden Verfahrensweisen und Reaktionsbedingungen können insbesondere auch den Beispielen entnommen werden.

### Verfahrensvariante a):

In 1H-Tetrazol-5-yl R₂ überführbare Reste Z₁ sind beispielsweise Cyano oder geschütztes 1H-Tetrazol-5-yl.

Zur Herstellung von Verbindungen der Formel 1, worin R₂ 1H-Tetrazol-5-yl bedeutet, geht man beispielsweise von Ausgangsmaterial der Formel II aus, worin Z₁ Cyano bedeutet, und setzt dieses mit einem Azid, wie HN₃ oder insbesondere einem Salz, wie Alkalimetallsalz, davon oder mit dem Ammoniumazid, einem Organozinnazid, wie Tri(nieder)alkylammonium-, Tri(nieder)alkyl- oder Triarylzinnazid, um. Bevorzugte Azide sind beispielsweise Natrium- und Kaliumazid sowie Tri-C₁-C₄-alkyl-ammonium-, Tri-C₁-C₄-alkylazid, z.B. Triethylammoniumazid, Triethyl- oder Tributylzinnazid, und Triphenylzinnazid. Die Azide können in an sich bekannter Weise z.T. in situ gebildet werden. Bevorzugt wird die Tetrazol-5-yl-Bildung mit solchen Verbindungen der Formel 11 durchgeführt, worin R₂ von Carboxy verschieden ist.

Als Schutzgruppen von geschütztem 1H-Tetrazol-5-yl kommen die üblicherweise in der Tetrazolchemie verwendeten Schutzgruppen in Frage, insbesondere Triphenylmethyl, gegebenenfalls, z.B. durch Nitro, substituiertes Benzyl, wie 4-Nitrobenzyl, Niederalkoxymethyl, wie Methoxy- und Ethoxymethyl, ferner 1-Ethoxyethyl, Niederalkylthiomethyl, wie Methylthiomethyl, Silyl, wie Triniederalkylsilyl,z.B. Dimethyl-tert-butyl- und Triisopropyl-silyl, sowie 2-Cyanoethyl, ferner Niederalkoxyniederalkoxymethyl, wie 2-Methoxyethoxymethyl, Benzyloxymethyl sowie Phenacyl.

Die Abspaltung der Schutzgruppen erfolgt in Anlehnung an bekannte Methoden, beispielsweise wie in J. Green, Protective Groups in Organic Synthesis, Wiley-Interscience (1980) beschrieben. So wird z.B. die Triphenylmethylgruppe üblicherweise durch Hydrolyse, insbesondere in Gegenwart einer Säure, oder Hydrogenolyse in Gegenwart eines Hydrierungskatalysators, rungskatalysators, 4-Nitrobenzyl z.B. durch Hydrogenolyse in Gegenwart eines Methoxy- oder Ethoxymethyl z.B. durch Behandeln mit einem Triniederalkyl-, wie Triethyl- oder Tributyl-zinn-bromid, Methylthiomethyl z.B. durch Behandeln mit Trifluoressigsäure, Silylreste z.B. durch Behandeln mit Fluoriden, wie Tetraniederalkylammoniumfluoriden, z.B. Tetrabutylammoniumfluorid, oder Alkalimetallfluoriden, z.B. Natriumfluorid, oder 2-Cyanoethyl z.B. durch Hydrolyse, beispielsweise mit Natronlauge, 2-Methoxyethoxymethyl z.B. durch Hydrolyse, z.B. mit Salzsäure, Benzyloxymethyl und Phenacyl z.B. durch Hydrogenolyse in Gegenwart eines Hydrierungskatalysators abgespalten.

Ein in R₂= SO₃H überführbarer Rest ist beispielsweise die Mercaptogruppe. Eine solche Gruppe aufweisende Ausgangsverbindungen der Formel II werden beispielsweise durch an sich bekannte Oxidationsverfahren zu solchen Verbindungen der Formel I oxidiert, worin R₂ SO₃H ist. Als Oxidationsmittel kommen beispielsweise anorganische Persäuren, wie Persäuren von Mineralsäuren, z.B. Periodsäure oder Perschwefelsäure, organische Persäuren, wie entsprechende Percarbon- oder Persulfonsäuren, z.B. Perameisen-, Peressig-, Trifluorperessig- bzw. Perbenzoesäure oder p-Toluolpersulfonsäure, oder Gemische aus Wasserstoffperoxid und Säuren, z.B. Gemisch aus Wasserstoffperoxid mit Essigsäure, in Betracht.

Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Übergangsmetalloxide, wie Oxide von Elementen der VII. Nebengruppe, z.B. Vanadium-, Molybdän- oder Wolframoxid, zu nennen sind. Die Oxidation wird unter milden Bedingungen, z.B. bei Temperaturen von etwa -50° bis etwa +100°C, durchgeführt.

Unter einer in R₂= PO₃H₂ überführbaren Gruppe ist beispielsweise eine Gruppe N₂⁺ A⁻ zu verstehen, wobei A⁻ für ein Anion einer Säure, wie Mineralsäure, steht. Derartige Diazonium verbindungen werden beispielsweise in an sich bekannter Weise mit einem P(III)-Halogenid, wie PCl₃ oder PBr₃, umgesetzt und hydrolytisch aufgearbeitet, wobei solche Verbindungen der Formel I erhältlich sind, worin R₂ PO₃H₂ ist.

Als in Halogen-C₁-C₇-alkansulfonylamino R₂ überführbarer Rest Z₁ kommt beispielsweise primäres Amino in Frage.

Zur Herstellung von Verbindungen der Formel I, worin R₂ Halogen-C₁-C₇-alkansulfonylamino bedeutet, setzt man beispielsweise entsprechende Aniline mit einer üblicherweise reaktionsfähig derivatisierten, z.B.veresterten, Halogen-C₁-C₇-alkansulfonsäure um, wobei gegebenenfalls in Gegenwart einer Base gearbeitet wird. Als bevorzugte reaktionsfähig veresterte Halogensulfonsäure kommt das entsprechende Halogenid, wie Chlorid oder Bromid, in Frage.

Ein in R₂ = COOH überführbarer Rest Z₁ steht beispielsweise für funktionell abgewandeltes Carboxy, wie Cyano, verestertes oder amidiertes Carboxy, Hydroxymethyl oder Formyl.

Verestertes Carboxy ist beispielsweise mit einem gegebenenfalls substituierten aliphatischen, cycloaliphatischen oder aromatischen Alkohol verestertes Carboxy. Ein aliphatischer Alkohol ist beispielsweise ein Niederalkanol, wie Methanol, Ethanol, Propanol, Isopropanol, n-Butanol, sec- oder tert.-Butanol, während als cycloaliphatischer Alkohol beispielsweise ein 3- bis 8-gliedriges Cycloalkanol, wie Cyclopentanol, -hexanol oder -heptanol, in Frage kommt. Ein aromatischer Alkohol ist beispielsweise ein Phenol oder heterocyclischer Alkohol, welche jeweils gegebenenfalls substituiert sein können, insbesondere Hydroxypyridin, z.B. 2-, 3- oder 4-Hydroxypyridin. Carboxy kann ebenfalls mit einem silyliertem Alkohol verestert sein und bedeutet insbesondere Tri-(C₁-C₄-)-alkylsilyl-(C₁-C₄-)alkoxy-carbonyl, insbesondere Trimethylsilylethoxycarbonyl.

Amidiertes Carboxy ist beispielsweise Carbamoyl, durch Hydroxy, Amino, oder gegebenenfalls substituiertes Phenyl monosubstituiertes, durch Niederalkyl mono- oder disubstituiertes oder durch 4- bis 7-gliedriges Alkylen bzw. 3-Aza-, 3-Niederalkylaza-, 3-Oxo- oder 3-Thiaalkylen disubstituiertes Carbamoyl. Als Beispiele sind Carbamoyl, N-Mono- oder N,N-Diniederalkylcarbamoyl, wie N-Methyl-, N-Ethyl-, N,N-Dimethyl-, N,N-Diethyl- oder N,N-Dipropylcarbamoyl, Pyrrolidino- oder Piperidinocarbonyl, Morpholino-, Piperazino- bzw. 4-Methylpiperazino- sowie Thiomorpholinocarbonyl, Anilinocarbonyl oder durch Niederalkyl, Niederalkoxy und/oder Halogen substituiertes Anilinocarbonyl zu nennen.

Bevorzugtes funktionell abgewandeltes Carboxy ist beispielsweise Tri-(C₁-C₄-)-alkylsilyl-(C₁-C₄-)alkoxy-carbonyl, insbesondere Trimethylsilylethoxycarbonyl, oder Cyano. Verbindungen der Formel I, worin R₂ Carboxy ist, können beispielsweise ausgehend von Verbindungen der Formel II, worin Z₁ funktionell abgewandeltes Carboxy bedeutet, in an sich bekannter Weise, beispielsweise durch Hydrolyse, insbesondere in Gegenwart einer Base, im Falle von entsprechenden
Tri-(C₁-C₄-)alkylsilyl-(C₁-C₄-)alkoxy-carbonylderivaten z.B. durch Behandeln mit einem Ammoniumfluorid, wie Tetraniederalkylammonium-, z.B. Tetra-n-butylammonium-fluorid, oder im Falle von Benzyloxycarbonylderivaten durch Hydrogenolyse in Gegenwart eines Hydrierungskatalysators, bzw. ausgehend von solchen Verbindungen der Formel II, worin Z₁ Hydroxymethyl oder Formyl bedeutet, unter Verwendung üblicher Oxidationsmittel, durch Oxidation hergestellt werden.

Die Oxidation erfolgt beispielsweise in einem inerten Lösungsmittel, wie einer Niederalkancarbonsäure z.B. Essigsäure, einem Keton, z.B. Aceton, einem Ether, z.B. Tetrahydrofuran, einem heterocyclischen Aromaten, z.B. Pyridin, oder Wasser oder einem Gemisch davon, erforderlichenfalls unter Kühlen oder Erwärmen, z.B. von etwa 0° bis etwa 150°C. Als Oxidationsmittel kommen beispielsweise oxidierende Übergangsmetallverbindungen, insbesondere solche mit Elementen der I., VI., oder VIII. Nebengruppe, in Frage. Als Beispiele seien genannt: Silberverbindungen, wie Silbernitrat, -oxid oder -picolinat, Chromverbindungen, wie Chromtrioxid oder Kaliumdichromat, Manganverbindungen, wie Kaliumpermanganat, Tetrabutylammonium- oder Benzyl(triethyl)ammoniumpermanganat. Weitere Oxidationsmittel sind beispielsweise geeignete Verbindungen mit Elementen der 4. Hauptgruppe, wie Bleidioxid, oder Halogen-Sauerstoff-Verbindungen, wie Natriumperiodat oder Kaliumperiodat.

So wird beispielsweise Hydroxymethyl und Formyl zu Carboxy R₂ oxidiert.

Vorzugsweise eignet sich diese Variante zur Herstellung solcher Verbindungen der Formel I, worin die Variablen Bedeutungen haben, die von ungesättigten Resten verschieden sind.

Als Basen kommen beispielsweise Alkalimetall-hydroxide, -hydride, -amide, -alkanolate, -carbonate, -triphenylmethylide, -diniederalkylamide, -aminoalkylamide oder -niederalkylsilylamide, Naphthalinamine, Niederalkylamine, basische Heterocyclen, Ammoniumhydroxide, sowie carbocyclische Amine in Frage. Beispielhaft seien Natriumhydroxid, -hydrid, -amid, Natriummethylat, -ethylat, Kalium-tert-butylat, -carbonat, Lithium-triphenylmethylid, -diisopropylamid, Kalium-3-(aminopropyl)-amid, -bis-(tri-methylsilyl)-amid, Dimethylaminonaphthalin, Di- oder Triethylamin, oder Ethyl-diisopropylamin, N-Methyl-piperidin, Pyridin, Benzyltrimethyl-ammoniumhydroxid, 1,5-Diazabicyclo [4.3.0]non-5-en (DBN) sowie 1,8-Diaza-bicyclo[5.4.0]undec-7-en (DBU) genannt.

Das Ausgangsmaterial der Formel II kann in an sich bekannter Weise hergestellt werden.

Beispielsweise ist das Ausgangsmaterial der Formel II zugänglich, indem man eine Verbindung der Formel 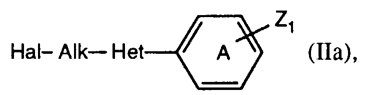 worin Hal z.B. Halogen bedeutet, mit einer Verbindung der Formel H₂N-X₂-X₃-X₄-R₃ (IIb) oder einem Salz davon erforderlichenfalls in Gegenwart einer Base umsetzt und eine so erhältliche Verbindung der Formel 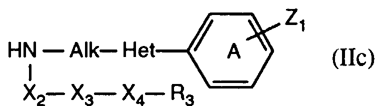 mit einer Verbindung der Formel R₁-X₁-OH (IIIb), einem reaktionsfähigen Derivat davon oder ein Salz davon, erforderlichenfalls in Gegenwart einer Base umsetzt.

Die Herstellung von Verbindungen der Formel IIa erfolgt in an sich bekannte Weise. Näheres kann den Ausführungsbeispielen entnommen werden.

### Verfahrensvariante b):

Aktivierte Ester von Verbindungen der Formel IIIb sind insbesondere am Verknüpfungskohlenstoffatom des veresternden Restes ungesättigte Ester, z.B. vom Vinylester-Typ, wie Vinylester (erhältlich z.B. durch Umesterung eines entsprechenden Esters mit Vinylacetat; Methode des aktivierten Vinylesters), Carbamoylvinylester (erhältlich z.B. durch Behandeln der entsprechenden Säure mit einem Isoxazoliumreagens; 1,2-Oxazolium- oder Woodward-Methode) oder 1-Niederalkoxyvinylester (erhältlich z.B. durch Behandeln der entsprechenden Säure mit einem Niederalkoxyacetylen; Ethoxyacetylen-Methode), oder Ester vom Amidinotyp, wie N,N'-disubstituierte Amidinoester (erhältlich z.B. durch Behandeln der entsprechenden Säure mit einem geeigneten N,N'-disubstituierten Carbodiimid, z.B. N,N'-Dicyclohexylcarbodiimid; Carbodiimid-Methode) oder N,N-disubstituierte Amidinoester (erhältlich z.B. durch Behandeln der entsprechenden Säure mit einem N,N-disubstituierten Cyanamid; Cyanamid-Methode), geeignete Arylester, insbesondere durch elektronenanziehende Substituenten substituierte Phenylester (erhältlich z.B. durch Behandeln der entsprechenden Säure mit einem geeignet substituierten Phenol, z.B. 4-Nitrophenol, 4-Methylsulfonylphenol, 2,4,5-Trichlorphenol, 2,3,4,5,6-Pentachlorphenol oder 4-Phenyldiazophenol, in Gegenwart eines Kondensationsmittels, wie N,N'-Dicyclohexylcarbodiimid; Methode der aktivierten Arylester), Cyanmethylester (erhältlich z.B. durch Behandeln der entsprechenden Säure mit Chloracetonitril in Gegenwart einer Base; Cyanmethylester-Methode), Thioester, insbesondere gegebenenfalls, z.B. durch Nitro, substituierte Phenylthioester (erhältlich z.B. durch Behandeln der entsprechenden Säure mit gegebenenfalls, z.B. durch Nitro, substituierten Thiophenolen, u.a. mit Hilfe der Anhydrid- oder Carbodiimid-Methode; Methode der aktivierten Thiolester) oder insbesondere Amino- oder Amidoester (erhältlich z.B. durch Behandeln der entsprechenden Säure mit einer N-Hydroxyamino- bzw. N-Hydroxyamido-Verbindung und deren aktivierten Derivaten, z.B. N-Hydroxysuccinimid, N-Hydroxypiperidin, N-Hydroxyphthalimid, N-Hydroxy-5-norbornen- oder norbonan-2,3-dicarbonsäureimid, 1-Hydroxybenzotriazol bzw. Benzotriazol-1-yloxy-phosphoniumsalzen oder Benzotriazol-1-yluroniumsalzen, oder 3-Hydroxy-3,4-dihydro- 1,2,3-benzotriazin-4-on, z.B. nach der Anhydrid- oder Carbodiimid-Methode; Methode der aktivierten N-Hydroxyester).

Anhydride von Säuren können symmetrische oder vorzugsweise gemischte Anhydride dieser Säuren sein, z.B. Anhydride mit anorganischen Säuren, wie Säurehalogenide, insbesondere Säurechloride (erhältlich z.B. durch Behandeln der entsprechenden Säure mit Thionylchlorid, Phosphorpentachlorid oder Oxalylchlorid; Säurechloridmethode), Azide (erhältlich z.B. aus einem entsprechenden Säureester über das entsprechende Hydrazid und dessen Behandlung mit salpetriger Säure; Azidmethode), Anhydride mit Kohlensäurehalbestern, z.B. Kohlensäureniederalkylhalbestern (erhältlich z.B. durch Behandeln der entsprechenden Säure mit Chlorameisensäureniederalkylestern oder mit einem 1-Niederalkoxycarbonyl-2-niederalkoxy- 1,2-dihydrochinolin, z.B. 1-Ethoxycarbonyl-2-ethoxy-1, 2-dihydrochinolin; Methode der gemischten O-Alkylkohlensäureanhydride), Anhydride mit dihalogenierter, insbesondere dichlorierter Phosphorsäure (erhältlich z.B. durch Behandeln der entsprechenden Säure mit Phosphoroxychlorid; Phosphoroxychloridmethode), Anhydride mit anderen Phosphorsäurederivaten (z.B. solchen, die man mit Phenyl-N-phenylphosphoramidochloridat erhalten kann) oder mit Phosphorigsäurederivaten, oder Anhydride mit organischen Säuren, wie gemischte Anhydride mit organischen Carbonsäuren (erhältlich z.B. durch Behandeln der entsprechenden Säure mit einem gegebenenfalls substituierten Niederalkan- oder Phenylniederalkancarbonsäurehalogenid, z.B. Phenylessigsäure-, Pivalinsäure- oder Trifluoressigsäurechlorid; Methode der gemischten Carbonsäureanhydride) oder mit organischen Sulfonsäuren (erhältlich z.B. durch Behandeln eines Salzes, wie eines Alkalimetallsalzes, der entsprechenden Säure mit einem geeigneten organischen Sulfonsäurehalogenid, wie Niederalkan- oder Aryl-, z. B. Methanoder p-Toluolsulfonsäurechlorid; Methode der gemischten Sulfonsäureanhydride), sowie symmetrische Anhydride (erhältlich z.B. durch Kondensation der entsprechenden Säure in Gegenwart eines Carbodiimids oder von 1-Diethylaminopropin; Methode der symmetrischen Anhyride).

Geeignete cyclische Amide sind insbesondere Amide mit fünfgliedrigen Diazacyclen aromatischen Charakters, wie Amide mit Imidazolen, z.B. Imidazol (erhältlich z.B. durch Behandeln der entsprechenden Säure mit N,N'-Carbonyldiimidazol; Imidazol-Methode), oder Pyrazolen, z.B. 3,5-Dimethylpyrazol (erhältlich z.B. über das Säurehydrazid durch Behandeln mit Acetylaceton; Pyrazolid-Methode).

Die Kondensation zur Herstellung der Amidbindung kann in an sich bekannter Weise durchgeführt werden, beispielsweise wie in Standardwerken, wie "Houben-Weyl, Methoden der organischen Chemie", 4. Auflage, Band 15/II, Georg Thieme Verlag, Stuttgart 1974, "The Peptides" (Herausg. E. Gross und J. Meienhofer), Band 1 und 2, Academic Press, London und New York, 1979/1980, oder M. Bodanszky, "Principles of Peptide Synthesis", Springer-Verlag, Berlin 1984, beschrieben.

Die Kondensation kann in Gegenwart eines der üblichen Kondensationsmittel durchgeführt werden. Uebliche Kondensationsmittel sind z.B. Carbodiimide, beispielsweise Diethyl-, Dipropyl-, N-Ethyl-N'-(3-dimethylaminopropyl)-carbodiimid oder insbesondere Dicyclohexylcarbodiimid, ferner geeignete Carbonylverbindungen, beispielsweise Carbonyldiimidazol, 1,2-Oxazoliumverbindungen, z.B. 2-Ethyl-5-phenyl-1,2-oxazolium-3'-sulfonat und 2-tert-Butyl-5-methylisoxazoliumperchlorat, oder eine geeignete Acylaminoverbindung, z.B. 2-Ethoxy-1-ethoxycarbonyl-1,2-dihydrochinolin, ferner aktivierte Phosphorsäurederivate, z.B. Diphenylphosphorylazid, Diethylphosphorylcyanid, Phenyl-N-phenylphosphoramidochloridat, Bis-(2-oxo-3-oxazolidinyl)-phosphinsäurechlorid oder 1-Benzotriazolyloxy-tris-(dimethylamino)-phosphonium-hexafluorophosphat.

Gewünschtenfalls wird eine organische Base zugegeben, z.B. ein Triniederalkylamin mit voluminösen Resten, z.B. Ethyldiisopropylamin, oder eine heterocyclische Base, z.B. Pyridin, 4-Dimethylaminopyridin oder bevorzugt N-Methylmorpholin.

Die Kondensation von Säureanhydriden mit Aminen kann z.B. in Gegenwart von anorganischen Carbonaten, z.B. Alkalimetallcarbonaten oder -hydrogencarbonaten, wie Natrium-oder Kaliumcarbonat oder -hydrogencarbonat (üblicherweise zusammen mit einem Sulfat), erfolgen.

Die Kondensation wird vorzugsweise in einem inerten, polaren, aprotischen, vorzugsweise wasserfreien, Lösungsmittel oder Lösungsmittelgemisch durchgeführt, beispielsweise in einem Carbonsäureamid, z.B. Formamid oder Dimethylformamid, einem halogenierten Kohlenwasserstoff, z.B. Methylenchlorid, Tetrachlorkohlenstoff oder Chlorbenzol, einem Keton, z.B. Aceton, cyclischen Ether, z.B. Tetrahydrofuran, einem Ester, z.B. Essigsäure-ethylester, oder einem Nitril, z.B. Acetonitril, oder in Mischungen davon, gegebenenfalls bei erniedrigter oder erhöhter Temperatur, z.B. in einem Temperaturbereich von etwa -40°C bis etwa +100°C, bevorzugt von etwa -10°C bis etwa +50°C, und gegebenenfalls unter Inertgas-, z.B. Stickstoffatmosphäre.

Reaktionsfähige Säurederivate können auch in situ gebildet werden.

Das Ausgangsmaterial der Formeln IIIa und IIIb ist bekannt bzw. kann in an sich bekannter Weise hergestellt werden.

Beispielsweise kann man das Ausgangsmaterial der Formel IIIa erhalten, indem man in einer Verbindung der Formel 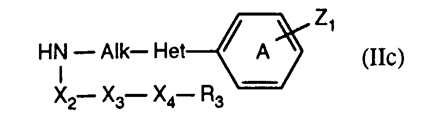 Z₁ in der in Variante a) angegebenen Weise in R₂ überführt.

In den Beispielen finden sich nähere Angaben zu Verfahren für die Herstellung entsprechender Ausgangsverbindungen bzw. ihrer Vorstufen.

Eine verfahrensgemäss oder auf andere Weise erhältliche Verbindung I kann in an sich bekannter Weise in eine andere Verbindung I überführt werden.

Eine Hydroxy aufweisende erfindungsgemässe Verbindung kann nach an sich bekannten Methoden verethert werden. Die Veretherung kann z.B. mit einem Alkohol, wie gegebenenfalls substituiertem C₁-C₇-Alkanol, oder einem reaktionsfähigen Ester desselben erfolgen. Als reaktionsfähige Ester der gewünschten Alkohole kommen beispielsweise solche mit starken anorganischen oder organischen Säuren in Frage, wie entsprechende Halogenide, Sulfate, Niederalkansulfonate oder gegebenenfalls substituierte Benzolsulfonate, z.B. Chloride, Bromide, Iodide, Methan-, Benzol- oder p-Toluol-sulfonate, in Betracht. Die Veretherung kann z.B. in Gegenwart einer Base, eines Alkalimetallhydrids, -hydroxids, -carbonats oder eines Amins, erfolgen. Umgekehrt können entsprechende Ether, wie C₁-C₇-Alkoxyverbindungen, z.B. mittels starker Säuren, wie Mineralsäuren, z.B. den Halogenwasserstoffsäuren Brom- oder Iodwasserstoffsäure, die vorteilhaft in Form von Pyridiniumhalogeniden vorliegen können, oder mittels Lewissäuren, z.B. Halogeniden von Elementen der 3. Hauptgruppe oder der entsprechenden Nebengruppen, gespalten werden. Diese Umsetzungen können, falls erforderlich, unter Kühlen oder Erwärmen, z.B. einem Temperaturbereich von etwa -20° bis etwa 100°C, in An- oder Abwesenheit eines Lösungs- oder Verdünnungsmittels, unter Inertgas und/oder unter Druck und gegebenenfalls in einem geschlossenen Gefäss, durchgeführt werden.

Hydroxymethylgruppen aufweisende erfindungsgemässe Verbindungen können beispielsweise ausgehend von entsprechenden Carboxy oder verestertes Carboxy aufweisenden Verbindungen hergestellt werden, wobei entsprechende Verbindungen in an sich bekannter Weise reduziert werden, z.B. durch Reduktion mit einem gegebenenfalls komplexen Hydrid, wie einem Hydrid gebildet aus einem Element der 1. und 3. Hauptgruppe des Periodensystems der Elemente, z.B. Boranat oder Alanat, beispielsweise Lithiumborhydrid, Lithium-, Diisobutylaluminiumhydrid (gegebenenfalls ist ein nachgelagerter Reduktionsschritt unter Verwendung von Alkalimetall-, wie Natriumcyanoborhydrid, erforderlich), ferner Diboran.

Falls ein Strukturbestandteil durch (C₁-C₇-)Alkylthio substituiert ist (in S(O)ₘ-R steht m für 0), kann man dieses auf übliche Weise zu entsprechendem (C₁-C₇-)Alkansulfinyl bzw. -sulfonyl oxidieren. Als geeignetes Oxidationsmittel für die Oxidation zur Sulfoxidgstufe kommen beispielsweise anorganische Persäuren, wie Persäuren von Mineralsäuren, z.B. Periodsäure oder Perschwefelsäure, organische Persäuren, wie entsprechende Percarbon-oder Persulfonsäuren, z.B. Perameisen-, Peressig-, Trifluorperessig- bzw. Perbenzoesäure oder p-Toluolpersulfonsäure, oder Gemische aus Wasserstoffperoxid und Säuren, z.B. Gemisch aus Wasserstoffperoxid mit Essigsäure, in Betracht.

Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Übergangsmetalloxide, wie Oxide von Elementen der VII. Nebengruppe, z.B. Vanadium-, Molybdän- oder Wolframoxid, zu nennen sind. Die Oxidation wird unter milden Bedingungen, z.B. bei Temperaturen von etwa -50° bis etwa +100°C, durchgeführt.

Die Oxidation zur Sulfonstufe kann man auch mit Distickstofftetroxid als Katalysator in Gegenwart von Sauerstoff bei tiefen Temperaturen entsprechend durchführen, ebenso wie die direkte Oxidation des (Nieder-)Alkylthio zum (Nieder-)Alkansulfonyl. Jedoch setzt man hierbei üblicherweise das Oxidationsmittel im Überschuss ein.

Weist eine der Variablen Amino auf, können entsprechende Verbindungen der Formel I, ihre Tautomeren oder Salze in an sich bekannter Weise N-alkyliert werden. Die (Phenyl-)-C₁-C₇-Alkylierung erfolgt z.B. mit einem reaktiven Ester eines (Phenyl-)-C₁-C₇-Alkylhalogenids, z.B. -bromid oder -iodid, (Phenyl-)C₁-C₇-Alkylsulfonat, z.B. methansulfonat oder -p-toluolsulfonat, oder einem Di-C₁-C₇-alkylsulfat, z.B. Dimethylsulfat, vorzugsweise unter basischen Bedingungen, wie in Gegenwart von Natronlauge oder Kalilauge, und vorteilhaft eines Phasentransfer- Katalysators, wie Tetrabutylammoniumbromid oder Benzyltrimethylammoniumchlorid, wobei indes stärker basische Kondensationsmittel, wie Alkalimetallamide, -hydride oder -alkoholate, z.B. Natriumamid, Natriumhydrid oder Natriumethanolat, erforderlich sein können. Ebenso kann Amino in an sich bekannter Weise, z.B. analog Variante b), acyliert werden.

In Verbindungen der Formel I, die als Substituenten eine veresterte oder amidierte Carboxygruppe aufweisen, kann man eine solche Gruppe z.B. mittels Hydrolyse, z.B. in Gegenwart eines basischen Mittels, oder eines sauren Mittels, wie einer Mineralsäure, in eine freie Carboxygruppe überführen. Tert-Butyloxycarbonyl beispielsweise kann weiterhin z.B. in an sich bekannter Weise, wie durch Behandeln mit Trihalogen-, wie Trifluoressigsäure, und Benzyloxycarbonyl z.B. durch katalytische Hydrierung in Gegenwart eines Hydrierungskatakysators, z. B. in der nachstehend beschriebenen Weise, in Carboxy überführt werden.

Ferner kann man in Verbindungen der Formel I, die als Substituenten eine Carboxygruppe aufweisen, insbesondere sofern R₃ von Carboxy verschieden ist, diese z.B. durch Behandeln mit einem Alkohol, wie einem C₁-C₇-Alkanol, in Gegenwart eines geeigneten Veresterungsmittels, wie eines sauren Reagens, z.B. einer anorganischen oder organischen Säure oder einer Lewissäure, z.B. Zinkchlorid, oder eines wasserbindenden Kondensationsmittels, z.B. eines Carbodiimids, wie N,N'-Dicyclohexyl-carbodiimid, oder durch Behandeln mit einem Diazoreagens, wie mit einem Diazoniederalkan, z.B. Diazomethan, in eine veresterte Carboxygruppe überführen. Diese kann man auch erhalten, wenn man Verbindungen der Formel I, worin die Carboxygruppe in freier Form oder in Salz-, wie Ammonium- oder Metall-, z.B. Alkalimetall-, wie Natrium- oder Kaliumsalzform vorliegt, mit einem reaktionsfähigen Ester eines (C₁-C₇-)Alkylhalogenid, z.B. Methyl- oder Ethyl-bromid oder -iodid, oder einem organischen Sulfonsäureester, wie einem entsprechenden (C₁-C₇-)Alkylester, z.B. Methansulfonsäure- oder p-Toluolsulfonsäuremethylester oder -ethylester, behandelt.

Verbindungen der Formel I, die als Substituenten eine veresterte Carboxygruppe aufweisen, kann man durch Umesterung, z.B. durch Behandeln mit einem Alkohol, üblicherweise einem höheren als dem der veresterten Carboxygruppe im Ausgangsmaterial entsprechenden Alkohol, in Gegenwart eines geeigneten Umesterungsmittels, wie eines basischen Mittels, z.B. eines Alkalimetall-(C₁-C₇-)alkanoats, -(C₁-C₇-)alkanolats oder -cyanids, wie Natriumacetat, -methanolat, -ethylat, -tert-butanolat oder -cyanid, oder eines geeigneten sauren Mittels, gegebenenfalls unter Entfernung des entstehenden Alkohols, z.B. durch Destillation, in andere Esterverbindungen der Formel I umestern. Man kann auch von entsprechenden, sogenannten aktivierten Estern der Formel I ausgehen, die als Substituenten eine aktivierte veresterte Carboxygruppe aufweisen (siehe unten), und diese durch Behandeln z.B. mit einem (C₁-C₇-)Alkanol, in einen anderen Ester umwandeln.

Man kann in Verbindungen der Formel I, die als Substituenten die Carboxylgruppe enthalten, diese auch zuerst in ein reaktionsfähiges Derivat, wie ein Anhydrid, inkl. ein gemischtes Anhydrid, wie ein Säurehalogenid, z.B. -chlorid (z.B. durch Behandeln mit einem Thionylhalogenid, z.B. -chlorid), oder ein Anyhdrid mit einem Ameisensäureester, z.B. -(C₁-C₇-)alkylester (z.B. durch Behandeln eines Salzes, wie eines Ammonium- oder Alkalimetallsalzes, mit einem Halogen-, wie Chlorameisensäureester, wie (C₁-C₇-)Alkylester), oder in einen aktivierten Ester, wie Cyanmethyl-, Nitrophenyl-, z.B. 4-Nitrophenyl-, oder Polyhalogenphenyl-, z.B. Pentachlorphenylester (z.B. durch Behandeln mit einer entsprechenden Hydroxyverbindung in Gegenwart eines geeigneten Kondensationsmittels, wie N,N'-Dicyclohexyl-carbodiimid) überführen, und ein solches reaktionsfähiges Derivat dann mit einem Amin umsetzen und so zu Amidverbindungen der Formel I gelangen, die als Substituenten eine amidierte Carboxygruppe aufweisen. Dabei kann man diese direkt oder über Zwischenverbindungen erhalten; so kann man z.B. einen aktivierten Ester, wie einen 4-Nitrophenylester, einer Verbindung der Formel I mit einer Carboxygruppe zuerst mit einem 1-unsubstituierten Imidazol umsetzen und die so entstandene 1-Imidazolylcarbonylverbindung mit einem Amin in Reaktion bringen. Man kann aber auch andere, nicht-aktivierte Ester, wie (C₁-C₇-)Alkylester von Verbindungen der Formel I, die als Substituenten z.B. (C₂-C₈-)Alkoxycarbonyl aufweisen, mit Aminen zur Reaktion bringen.

Verbindungen der Formel I, worin R₂ Carboxy ist, können beispielsweise ausgehend von solchen Verbindungen der Formel I, worin R₂ Hydroxymethyl oder Formyl bedeutet, in an sich bekannter Weise unter Verwendung üblicher Oxidationsmittel, durch Oxidation hergestellt werden. Die Oxidation erfolgt beispielsweise in einem inerten Lösungsmittel, wie einer C₁-C₇-Alkancarbonsäure z.B. Essigsäure, einem Keton, z.B. Aceton, einem Ether, z.B. Tetrahydrofuran, einem heterocyclischen Aromaten, z. B. Pyridin, oder Wasser oder einem Gemisch davon, erforderlichenfalls unter Kühlen oder Erwärmen, z.B. von etwa 0° bis etwa 150°C. Als Oxidationsmittel kommen beispielsweise oxidierende Übergangsmetallverbindungen, insbesondere solche mit Elementen der I., VI., oder VIII. Nebengruppe, in Frage. Als Beispiele seien genannt: Silberverbindungen, wie Silbernitrat, -oxid oder -picolinat, Chromverbindungen, wie Chromtrioxid oder Kaliumdichromat, Manganverbindungen, wie Kaliumpermanganat, Tetrabutylammonium- oder Benzyl(triethyl)ammoniumpermanganat. Weitere Oxidationsmittel sind beispielsweise geeignete Verbindungen mit Elementen der 4. Hauptgruppe, wie Bleidioxid, oder Halogen-Sauerstoff-Verbindungen, wie Natriumperiodat oder Kaliumperiodat.

Weist ein aromatischer Ring als Substituenten ein Wasserstoffatom auf, so kann dieses mit Hilfe eines Halogenierungsmittels in üblicher Weise durch ein Halogenatom ersetzt, z.B. mit Brom, Hypobromsäure, Acylhypobromite oder andere organische Bromverbindungen, z.B. N-Bromsuccinimid, N-Bromacetamid, N-Bromphthalimid, Pyridiniumperbromid, Dioxandibromid, 1,3-Dibrom-5,5-dimethylhydantoin, 2,4,4,6-Tetrabrom-2,5-cyclohexandien-1-on, bromiert bzw. mit elementarem Chlor, z.B. in einem halogenierten Kohlenwasserstoff, wie Chloroform, und unter Kühlen, z.B. bis auf etwa -10° bis etwa +100°C, chloriert werden.

Enthält ein aromatischer Ring in den erfindungsgemässen Verbindungen eine Aminogruppe, so kann diese in üblicher Weise diazotiert werden, z.B. durch Behandeln mit einem Nitrit, z.B. Natriumnitrit, in Gegenwart einer geeigneten Protonsäure, z.B. Mineralsäure, wobei die Reaktionstemperatur vorteilhaft unter etwa 5°C gehalten wird. Die so erhältliche, in Salzform vorliegende Diazoniumgruppe kann man nach analogen Verfahren beispielsweise wie folgt substituieren: durch die Hydroxygruppe analog der Phenolverkochung in Gegenwart von Wasser; durch eine Alkoxygruppe durch Behandeln mit einem entsprechenden Alkohol, wobei Energie zugeführt werden muss; durch das Fluoratom analog der Schiemann-Reaktion bei der Thermolyse von entsprechenden Diazoniumtetrafluorboraten; durch die Halogenatome Chlor, Brom oder Iod sowie die Cyanogruppe analog der Sandmeyer-Reaktion bei der Umsetzung mit entsprechenden Cu(I)-Salzen, zunächst unter Kühlen, z.B. auf etwa unter 5°C, und anschliessendem Erhitzen, z.B. auf etwa 60° bis etwa 150°C.

Enthalten die Verbindungen der Formel I ungesättigte Reste, wie (C₃-C₇-)Alkenylgruppierungen, können diese in an sich bekannter Weise in gesättigte Reste überführt werden. So erfolgt beispielsweise die Hydrierung von Mehrfachbindungen durch katalytische Hydrierung in Gegenwart von Hydrierungskatalysatoren, wobei hierfür z.B. Nickel, wie Raney-Nickel, sowie Edelmetalle bzw. deren Derivate, z.B. Oxide, geeignet sind, wie Palladium, Platinoxid, die gegebenenfalls auf Trägermaterialien, z.B. auf Kohle oder Calciumcarbonat, aufgezogen sein können. Die Hydrierung kann vorzugsweise bei Drucken zwischen 1 und etwa 100 at und zwischen etwa -80° bis etwa 200°C, vor allem zwischen Raumtemperatur und etwa 100°C, durchgeführt werden. Die Reaktion erfolgt zweckmässig in einem Lösungsmittel, wie Wasser, einem Niederalkanol, z.B. Ethanol, Isopropanol oder n-Butanol, einem Ether, z.B. Dioxan, oder einer Niederalkancarbonsäure, z.B. Essigsäure.

Die Erfindung betrifft insbesondere die in den Beispielen beschriebenen Verfahren.

Salze von Verbindungen I können in an sich bekannter Weise hergestellt werden. So erhält man beispielsweise Säureadditionssalze von Verbindungen I durch Behandeln mit einer geeigneten Säure oder einem geeigneten Ionenaustauscherreagens. Salze von Verbindungen I können in üblicher Weise in die freien Verbindungen I überführt werden, Säureadditionssalze z.B. durch Behandeln mit einem geeigneten basischen Mittel oder einem geeigneten Ionenaustauscherreagens.

Salze von Verbindungen I können in an sich bekannter Weise in andere Salze von Verbindungen I umgewandelt werden.

Je nach Verfahrensweise bzw. Reaktionsbedingungen können die Verbindungen I mit salzbildenden, insbesondere basischen Eigenschaften, in freier Form oder in Form von Salzen erhalten werden.

Infolge der engen Beziehung zwischen der Verbindung I in freier Form und in Form ihrer Salze sind im Vorausgegangenen und nachfolgend unter der freien Verbindung I bzw. ihren Salzen sinn- und zweckgemäss gegebenenfalls auch die entsprechenden Salze bzw. die freie Verbindung I zu verstehen.

Die Verbindungen I einschliesslich ihrer Salze von salzbildenden Verbindungen können auch in Form ihrer Solvate, wie Hydrate, erhalten werden und/oder andere, z. B. zur Kristallisation verwendete, Lösungsmittel einschliessen. Sinn- und zweckgemäss sind unter freien erfindungsgemässen Verbindungen gegebenenfalls auch die entsprechenden Sovate, wie Hydrate, zu verstehen.

Die Verbindungen I und ihre Salze können teilweise, je nach der Wahl der Ausgangsstoffe und Arbeitsweisen, in Form eines der möglichen Isomeren oder als Gemisch derselben, z.B. je nach Anzahl, absoluter und relativer Konfiguration der asymmetrischen Kohlenstoffatome als reine Isomere, wie Antipoden und/oder Diastereomere, oder als Isomerengemische, wie Enantiomerengemische, z. B. Racemate, Diastereomerengemische oder Racematgemische, vorliegen.

Erhaltene Diastereomerengemische und Racematgemische können auf Grund der physikalisch-chemischen Unterschiede der Bestandteile in bekannter Weise in die reinen Diastereomeren oder Racemate aufgetrennt werden, beispielsweise durch fraktionierte Kristallisation. Erhaltene Enantiomerengemische, wie Racemate, lassen sich nach bekannten Methoden in die optischen Antipoden zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenether, wobei nur ein Enantiomeres komplexiert wird, oder durch Überführung in diastereomere Salze, z.B. durch Umsetzung eines basischen Endstoffracemats mit einer optisch aktiven Säure, wie Carbonsäure, z.B. Wein- oder Äpfelsäure, oder Sulfonsäure, z. B. Camphersulfonsäure, oder durch Umsetzung eines sauren Endstoffracemats mit einer optisch aktiven Base, wie Cinchonidin, Cinchonin, Chinin, Phenethylamin, Dehydroabietylamin, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Antipoden verwendet oder insbesondere unter den Reaktionsgedingungen bildet.

Beim Verfahren der vorliegenden Erfindung werden vorzugsweise solche Ausgangsstoffe und Zwischenprodukte verwendet, welche zu den eingangs als besonders wertvoll geschilderten Verbindungen I führen. Neue Ausgangsstoffe und Zwischenprodukte für die Herstellung der Verbindungen I, ihre Verwendung und ein Verfahren zu ihrer Herstellung bilden ebenfalls einen Gegenstand der Erfindung, wobei die Variablen R₁, R₂, R₃, X₁, X₂, X₃, X₄, Alk und Het sowie die Ringe A, B, C und D die für die Verbindungen I angegebenen Bedeutungen haben. Insbesondere Verbindungen der Formeln II und IIa, worin Z₁ z.B. Cyano ist, bilden ebenfalls einen Gegenstand der Erfindung, wobei die übrigen Variablen die jeweils vorstehend angegebenen Bedeutungen haben.

Die Verbindungen I und ihre pharmazeutisch verwendbaren Salze können, vorzugsweise in Form von pharmazeutisch verwendbaren Zubereitungen, in einem Verfahren zur prophylaktischen und/oder therapeutischen Behandlung des tierischen oder menschlichen Körpers, insbesondere als Antihypertensiva oder als Mittel zur Behandlung von Glaukom bzw. zur Steigerung des Flusses der retinalen Aubenflüssigkeit, verwendet werden.

Die Erfindung betrifft daher gleichfalls pharmazeutische Präparate, die eine Verbindung I in freier Form oder in Form eines pharmazeutisch verwendbaren Salzes als Wirkstoff enthalten, sowie ein Verfahren zu ihrer Herstellung. Bei diesen pharmazeutischen Präparaten handelt es sich um solche zur enteralen, wie oralen, ferner rektalen oder parenteralen, ebenso zur ophthalmischen (d.h. topischen Applikation am Auge) Verabreichung an Warmblüter, wobei der pharmakologische Wirkstoff allein oder zusammen mit üblichen pharmazeutischen Hilfsstoffen enthalten ist. Die pharmazeutischen Präparate enthalten z.B. von etwa 0,1 % bis 100 %, vorzugsweise von etwa 1 % bis etwa 60 %, des Wirkstoffs. Pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren, hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragantgummi, Methylcellulose und/oder Polyvinylpyrrolidon, und, wenn erwünscht, Sprengmittel, wie die obengenannten Stärken, ferner Carboxymethylstärke, quervernetztes Polyvinylpyrrolidon, Agar oder Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Maensaftresistenten Überzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine sowie weiche, geschlossene Kapseln aus Gelatine und einem Weichmacher, wie Glycerin oder Sorbitol. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Ölen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole und höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole und Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Ethyloleat oder Triglyceride, verwendet, oder wässrige Injektionssuspensionen, welche viskositätserhöhende Stoffe, z.B. Natriumcarboxymethylcellulose, Sorbit und/oder Dextran, und gegebenenfalls auch Stabilisatoren enthalten.

Die Dosierung des Wirkstoffes kann von verschiedenen Faktoren, wie Applikationsweise, Warmblüter-Spezies, Alter und/oder individuellem Zustand, abhängen. Im Normalfall ist für einen etwa 75 kg schweren Patienten bei oraler Applikation eine ungefähre Tagesdosis von etwa 10 mg bis etwa 250 mg zu veranschlagen.

Entsprechende ophthalmische Präparate werden vorteilhaft topisch auf das Auge appliziert, insbesondere in Form einer Lösung, einer Salbe, eines Gels oder eines festen Kissens. Derartige Zusammensetzungen enthalten den Wirkstoff beispielsweise in einem Bereich von etwa 0,01 bis etwa 10,0 Gew.-%, vorzugsweise von etwa 0,5 bis etwa 5,0 Gew.-%. Dosiseinheitsformen des Wirkstoffes betragen beispielsweise von etwa 0,001 bis etwa 5,0 Gew.-%, insbesondere von etwa 0,05 bis etwa 2,0 Gew.-%, vorzugsweise con etwa 0,1 bis etwa 1,5 Gew.-%, in erster Linie von etwa 0,1 1 bis etwa 1,0 Gew.-%. Die Dosierung des Wirkstoffes kann von verschiedenen Faktoren, wie Applikationsweise, Bedarf, Alter und/oder individuellem Zustand, abhängen.

Für entsprechende ophthalmische Präparate werden übliche, dem Fachmann bekannte pharmazeutisch verwendbare Hilfs- bzw. Zusatzstoffe eingesetzt, beispielsweise solche der vorstehend aufgeführten Art. Die Herstellung solcher Präparate erfolgt in an sich bekannte Weise, so wird der Wirkstoff mit den entsprechenden Hilfs- und/oder Zusatzstoffen zu entsprechenden ophthalmischen Präparaten vermischt. Vorzugsweise wird der Wirkstoff in Form von Augentropfen verabreicht, wobei der Wirkstoff insbesondere in einer sterilen, wässrigen isotonischen Lösung gelöst wird, die gegebenenfalls auf den gewünschten pH-Wert gepuffert wird.

Die nachfolgenden Beispiele illustrieren die oben beschriebene Erfindung. Temperaturen sind in Grad Celsius angegeben. Die R_{f}-Werte werden auf Kieselgel-Schichtplatten (E.Merck - Art.-Nr. 5715), die im jeweils angegebenen Lösungsmittelsystem entwickelt werden, bestimmt.

### Beispiel 1:

Eine Lösung von 0,82 g N-[2-(2'-(1H-Tetrazol-5-yl)-phenyl)-chinolin-6-yl-methyl)]-N-valeroyl-valin-benzylester und 15 ml 2N Kalilauge in 15 ml Ethanol wird 45 Minuten auf 100° erhitzt. Die abgekühlte Lösung wird angesäuert und eingedampft. Der Rückstand liefert nach Filtration mit Methanol als Lösungsmittel durch ein Millipore-Filter und Entfernen des Lösungsmittels N-[2-(2'-(1H-Tetrazol-5-yl)-phenyl)-chinolin-6-yl-methyl]N-valeroyl-valin, R_{f} = 0,19 (CH₂Cl₂/CH₃OH (4:1)).

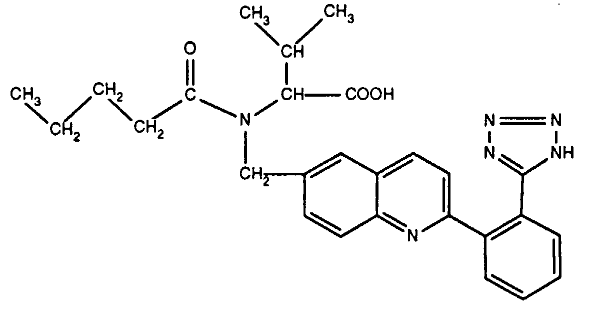

Das Ausgangsmaterial kann beispielsweise wie folgt hergestellt werden:
a) Eine Lösung von 11,94 g Trimethylzinnchlorid in 10 ml Dimethoxyethan wird zu einer bei 0° gehaltenen Mischung von 4,14 g Natrium in 40 ml Dimethoxyethan gegeben und 3 Stunden bei 0° gerührt. Die gebildete grün-gelbe Suspension wird vom überschüssigen Natrium getrennt und bei 0° tropfenweise mit einer Lösung von 8,88 g 2-Clor-6-methylchinolin (O.Fischer, Berichte 32, 1305) in 30 ml Tetrahydrofuran versetzt. Nach 3-stündigem Rühren wird auf Raumtemperatur erwärmt. Das mit 50 ml Ether verdünnte Gemisch wird mit zweimal 10 ml Wasser gewaschen. Die Etherphase wird über Na₂SO₄ getrocknet, von dem Lösungsmittel befreit und im Kugelrohr destilliert, bei 200° und 0,2 mbar geht 6-Methyl-2-trimethylstannyl-chinolin über.
b) Eine Lösung von 10,66 g 6-Methyl-2-trimethylstannyl-chinolin und 7,98 b 2-iod-benzo-nitril in 75 ml Toluol wird mit 0,40 g Tetrakis-(tripenylphosphin)-palladium (Fluka 87645) versetzt und 27 Stunden unter Rückfluss erhitzt. Das Reaktionsgemisch wird im Vakuum eingedampft und mit 90 ml Ether verdünnt und kristallisiert. Durch Nutschen wird 6-Methyl-2-(2'-cyanophenyl)-chinolin, Smp. 154- 155°, erhalten.
c) Eine Mischung von 4.89 g 6-Methyl-2-(2'-cyanophenyl)-chinolin, 4,04 g N-Bromsuccinimid und 0,09 g Azo-isobutyronitril in 100 ml Tetrachlorkohlenstoff wird 6 Stunden auf 110° erhitzt. Das abgekühlte Reaktionsgemisch wird sodann mit 20 ml 2N Natronlauge versetzt und mit 100 ml sowie zweimal mit 50 ml CH₂Cl₂ ausgezogen. Die organischen Phasen liefern nach üblicher Behandlung 6-Brommethyl-2-(2'-cyanophenyl)-chinolin, Smp. 162-166°.
d) Eine Lösung von 4,44 g Valin-benzylester-toluolsulfonsäuresalz, 5,17 g Hünig-Base und 3,23 g 6-Brommethyl-2-(2'-cyanophenyl)-chinolin in 20 ml Dimethylformamid wird 1 Stunde auf 80° erwärmt. Das Lösungsmittel wird sodann entfernt und das Rohprodukt in 200 ml Essigester gelöst. Die organische Phase wird mit zweimal 50 ml Wasser und je einmal mit 50 ml gesättigter NaHCO₃-Lösung sowie Sole gewaschen. Die Waschphasen werden mit 50 ml Essigester rückextrahiert. Die vereinigten organischen Phasen werden wie üblich behandelt und liefern N-[2-(2'-Cyanophenyl)-chinolin-6-yl-methyl]-valin-benzylester,R_{f} = 0,21 (CH₂Cl₂/CH₃OH (99:1)), der roh weiterverarbeitet wird.
e) Eine Lösung von 4,79 g N-[2-(2'-Cyanophenyl)-chinolin-6-yl-methyl]-valin-benzylester, 2,07 ml Triethylamin und 1,80 ml Valeroylchlorid in 100 ml CH₂Cl₂ wird 1 Stunde bei Raumtemperatur gerührt, dann mit zweimal 50 ml Wasser gewaschen und die Waschphasen mit 50 ml CH₂Cl₂ rückextrahiert. Die organischen Phasen liefern nach üblicher Behandlung N-[2-(2'-Cyanophenyl)-chinolin-6-yl-methyl]-N-valeroyl-valin-benzylester, R_{f} = 0,23 (CH₂Cl₂/CH₃OH (99:1)).
f) Eine Lösung von 5,33 g N-[2-(2'-Cyanophenyl)-chinolin-6-yl-methyl]-N-valeroyl-valin-benzylester und 13 g Tributylzinnazid in 100 ml Xylol wird 5 Stunden unter Rückfluss erhitzt. Die abgekühlte Lösung wird mit 50 ml 2N Natronlauge 30 Minuten gerührt. Die wässrige Phase wird angesäuert und mit dreimal 50 ml CH₂Cl₂ extrahiert. Das nach üblichem Vorgehen erhaltene Rohprodukt liefert nach Chromatographie an Kieselgel 60 (40-63µm - Merck Art.-Nr. 9385) mit CH₂Cl₂/CH₃OH (95:5) als Elutions-mittel N-[2-(2'-(1H-Tetrazol-5-yl)-phenyl)-chinolin-6-yl-methyl]-N-valeroy-valin-benzylester, R_{f} = 0,48 (CH₂Cl₂/CH₃OH (4: 1)).

### Beispiel 2:

Analog Beispiel 1 werden 0,84 g N-[2-(2'-(1H-Tetrazol-5-yl)-phenyl-chinolin-6-yl-methyl)]-N-valeroyl-1-aminomethyl-cyclopentan-1-carbonsäureethylester und 5 ml 2N Kalilauge in 10 ml Ethanol gelöst und 2,5 Stunden auf 100° erhitzt. Die Aufarbeitung erfolgt durch Trocknen mit Na₂SO₄, Filtration und Entfernen des Lösungsmittels am Rotationsverdampfer bei vermindertem Druck und liefert N-[2-(2'-(1H-Tetrazol-5-yl)-phenyl)-chinolin-6-yl-methyl]-N-valeroyl-1-aminomethyl-cyclopentan-1-carbonsäure, R_{f} = 0,14 (CH₂Cl₂/CH₃OH (4:1)).

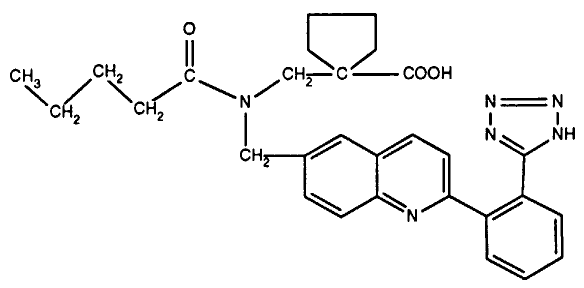

Das Ausgangsmaterial kann beispielsweise wie folgt hergestellt werden:
a) 1-Aminomethyl-cyclopentan-1-carbonsäureethylester wird erhalten durch Hydrieren von 33 g 1-Cyano-cyclopentan-1-carbonsäureethylester (Alfred Bader Chemicals) in 330 ml Ethanol, der ca. 4% Ammoniak enthält, in Gegenwart von 10 g Raney-Nickel bei 45° und unter Normaldruck. Nach Abfiltrieren vom Katalysator und Entfernen der Lösungsmittel im Vakuum wird das Produkt durch Destillation erhalten, Sdp. 71 -74° bei 0.75 mbar.
b) Eine Lösung von 1,03 g 1-Aminomethyl-cyclopentan-1-carbonsäureethylester, 0,68 ml N-Methyl-morpholin und 1,94 g 6-Brommethyl-2-(2'-cyanophenyl)-chinolin wird unter Erwärmen zur Schmelze gebracht und nach 20 Minuten mit 60 ml Essigester verdünnt. Diese Lösung wird mit zweimal 20 ml gesättigter NaHCO₃-Lösung gewaschen, über Na₂SO₄ getrocknet und von dem Lösungsmittel befreit. Das erhaltene Rohprodukt liefert nach Chromatographie an Kieselgel 60 (40-63µm) mit CH₂Cl₂/CH₃OH (98:2) als Elutionsmittel N-[2-(2'-Cyanophenyl)-chinolin-6-yl-methyl]-1-aminomethyl-cyclopentan-1-carbonsäureethylester, R_{f} = 0,28 (CH₂Cl₂/CH₃OH (95:5)).
c) Analog Beispiel 1 e) wird N-[2-(2'-Cyanophenyl)-chinolin-6-yl-methyl]-N-valeroyl-1-aminomethyl-cyclopentan-1-carbonsäureethylester, R_{f} = 0,64 (CH₂Cl₂/CH₃OH (95:5)), erhalten.
d) Analog Beispiel 1 f) wird, nach Chromatographie an Kieselgel 60 (40-63 µm) mit CH₂Cl₂/CH₃O (9:1) als Elutionsmittel N-[2-(2'-(1H-Tetrazol-5-yl)-phenyl)-chinolin-6-yl-methyl]-N-valeroyl-1-aminomethyl-cyclopentan-1-carbonsäureethylester, R_{f} = 0,43 (CH₂Cl₂/CH₃OH (4:1)), erhalten.

### Beispiel 3:

Eine Lösung von 1,73 g N-[2-(2'-(1H-Tetrazol-5-yl)-phenyl)-chinolin-6-yl-methyl]-N-valeroyl-2-aminomethyl-2-ethyl-buttersäure-tert.-butylester in 10 ml Trifluoressigsäure wird 45 Minuten bei Raumtemperatur gerührt, mit Toluol verdünnt und eingedampft. Das Rohprodukt liefert nach Chromatographie an Kieselgel 60 (40-63µm) mit CH₂Cl₂/CH₃OH (95:5) als Elutionsmittel N-[2-(2'-(1H-Tetrazol-5-yl)-phenyl)-chinolin-6-yl-methyl]-N-valeroyl-2-aminomethyl-2-ethyl-buttersäure, R_{f}=0,38 (CH₂Cl₂/CH₃OH (4: 1)).

Das Ausgangsmaterial kann beispielsweise wie folgt hergestellt werden:
a) Eine Mischung von 13,06 g Cyanoessigsäure-tert.-butylester (Lancester Synthesis Ltd., LAN-0123), 20,7 ml Bromethan, 0,96 g n-Tetrabutylammoniumbromid, 58 ml Kalilauge und 50 ml CH₂Cl₂ wird 23 Stunden bei Raumtemperatur gerührt. Die Phasen werden getrennt und die wässrige Phase dreimal mit 50 ml Ether ausgezogen. Alle organischen Phasen werden vereint und dreimal mit 50 ml Wasser gewaschen. Das nach Aufarbeitung - wie in Beispiel 2 beschrieben - erhaltene Rohprodukt liefert nach Destillation im Vakuum 2-Cyano-2-ethyl-buttersäure-tert.-butylester.
b) 29,21 g 2-Cyano-2-ethyl-buttersäure-tert.-butylester, gelöst in 300 ml Ethanol, der 4% Ammoniak enthält, werden in Gegenwart von 6 g Raney-Nickel bei 40° unter Normaldruck hydriert. Nach Abtrennen vom Katalysator wird im Vakuum eingedampft und die dabei zurückbleibende Flüssigkeit im Vakuum destilliert. Man erhält so den 2-Aminomethyl-2-ethyl-buttersäure-tert.-butylester, Siedepunkt 65° bei 0,6 mbar.
c) Eine Lösung von 1,98 g 2-Aminomethyl-2-ethyl-buttersäure-tert.-butylester, 1,4 ml N-Methyl-morpholin und 2,91 g 6-Brommethyl-2-(2'-cyanophenyl)-chinolin wird 1,5 Stunden auf 100° erhitzt. Die Mischung wird in 100 ml Essigester aufgenommen und mit je 50 ml Wasser und gesättigter NaHCO₃-Lösung gewaschen. Das nach üblichem Vorgehen erhaltene Rohprodukt liefert nach Chromatographie an Kieselgel 60 (40-63 µm) mit CH₂Cl₂/CH₃OH (98:2) als Elutionsmittel N-[2-(2'-Cyanophenyl)-chinolin-6-yl-methyl]-2-aminomethyl-2-ethyl-buttersäure-tert.-butylester, R_{f} = 0,40 (CH₂Cl₂/CH₃OH (95:5)).
d) Analog Beispiel 1 e) wird N-[2-(2'-Cyanophenyl)-chinolin-6-yl-methyl]-N-valeroyl-2-aminomethyl-2-ethyl-buttersäure-tert.-butylester, R_{f} = 0,59 (CH₂Cl₂/CH₃OH (95:5)), erhalten.
e) Analog Beispiel 1 f) wird N-[2-(2'-( 1H-Tetrazol-5-yl)-phenyl)-chinolin-6-yl-methyl]-N-valeroyl-2-aminomethyl-2-ethyl-buttersäure-tert.-butylester, R_{f} = 0,55 (CH₂Cl₂/CH₃OH (4:1)), erhalten.

### Beispiel 4:

Eine Lösung von 1,426 g N-[2-(2'-(1H-Tetrazol-5-yl)-phenyl)-5-yl-methylbenzo [b]thiophen]-N-valeroyl-2-aminomethyl-2-ethyl-buttersäure-tert.-butylester in 10 ml Trifluoressigsäure wird bei Raumtemperatur 30 Minuten gerührt. Das nach Entfernen des Lösungsmittels erhaltene Rohprodukt liefert nach Chromatographie an Kieselgel 60 (40-63 µm) mit CH₂Cl₂/CH₃OH (95:5) als Elutionsmittel N-[2-(2'-(1H-Tetrazol-5-yl)-phenyl)-benzo[b]thiophen-5-yl-methyl]-N-valeroyl-2-aminomethyl-2-ethyl-buttersäure, R_{f} =0,39(CH₂Cl₂/CH₃OH(4:1)).

Das Ausgangsmaterial kann beispielsweise wie folgt hergestellt werden:
a) Eine Lösung von 7,411 g 5-Methyl-benzo[b]thiophen (Maybridge, Chemical Co., Ltd. 11-78) in 35 ml Ether wird bei 0° zu einer Mischung von 40 ml 1,5 N Butyllithium in Hexan und 40 ml Ether getropft. Nach 30 Minuten wird eine Lösung von 9,963 g Trimethylzinnchlorid in 50 ml Ether ebenfalls bei 0° zugetropft. Nach 1-stündigem Rühren bei 0° werden 50 ml 5 %-ige Ammoniumchlorid-Lösung zugegeben. Das Produkt wird durch Extrahieren mit 100 ml und 150 ml Ether gewonnen. Destillation bei 0,15 mbar und 100-106° liefert 5-Methyl-2-trimethylstannyl-benzo[b]thiophen.
b) Eine Lösung von 12,84 g 5-Methyl-2-trimethylstannyl-benzo[b]thiophen, 9,39 g 2-iodbenzonitril und 0,464 g Tetrakis-(triphenylphosphin)-palladium in 120 ml Toluol wird 6,5 Stunden unter Rückfluss erwärmt. Nach dem Entfernen des Lösungsmittels wird mit 30 ml Ether verdünnt und durch Abnutschen 2-(2'-Cyanophenyl)-5-methyl-benzo[b]thiophen, Smp. 152- 153°, erhalten.
c) Eine Mischung von 5,00 g 2-(2'-Cyanophenyl)-5-methyl-benzo[b]thiophen, 4,04 g N-Bromsuccinimid und 0,09 g Azo-isobutyronitril in 100 ml Tetrachlorkohlenstoff wird 5 Stunden unter Rückfluss erhitzt. Nach Zugabe von 50 ml CH₂Cl₂ wird mit zweimal 20 ml 2N Natronlauge extrahiert; die alkalischen Phasen werden mit 50 ml CH₂Cl₂ rückextrahiert. Die organischen Phasen liefern nach nach Aufarbeitung gemäss Beispiel 2 5-Brommethyl-2-(2'-cyanophenyl)-benzo[b]thiophen, Smp. 108-110°.
d) Eine Mischung von 5,03 g 2-Aminomethyl-2-ethyl-buttersäure-tert.-butylester und 3,283 g 5-Brommethyl-2-(2'-cyanophenyl)-benzo[b]thiophen wird bei 100° 15 Minuten gerührt und nach dem Abkühlen in 100 ml Essigester aufgenommen. Das nach Waschen mit je 50 ml NaHCO₃-Lösung und Wasser und nach Aufarbeitung gemäss Beispiel 2 erhaltene Rohprodukt liefert nach Chromatographie an Kieselgel 60 (40-63 µm) mit CH₂Cl₂/CH₃OH (99:1) als Elutionsmittel N-[2-(2'-Cyanophenyl)-benzo[b]thiophen-5-yl-methyl]-2-aminomethyl-2-ethyl-buttersäure-tert.-butylester, R_{f}=0,48 (CH₂Cl₂/CH₃OH (95:5)).
e) Analog Beispiel 1 e) wird N-[2-(2'-Cyanophenyl)-benzo[b]thiophen-5-yl-methyl]-N-valeroyl-2-aminomethyl-2-ethylbuttersäure-tert.-butylester, R_{f}=0,64 CH₂Cl₂/CH₃OH (95:5), erhalten.
f) Analog Beispiel 1 f) wird N-[2-(2'-(1H-Tetrazol-5-yl)-phenyl)-5-yl-methyl-benzo[b]-thiophen]-N-valeroyl-2-aminomethyl-2-ethyl-buttersäure-tert.- butylester, R_{f}= 0,42 (CH₂Cl₂/CH₃OH (4:1)), erhalten.

### Beispiel 5:

Eine Lösung von 2,41 g N-[3-Brom-2-(2'-ethoxycarbonyl-phenyl)-benzo[b]-thiophen-5-yl-methyl]-N-valeroyl-valin-benzylester und 20 ml 2N Kalilauge in 20 ml Ethanol wird 22 Stunden unter Rückfluss erhitzt. Die abgekühlte Lösung wird angesäuert und eingedampft. Der Rückstand liefert nach Chromatographie an Kieselgel 60 (40-63 µm) mit CH₂Cl₂/CH₃OH (95:5) als Elutionsmittel N-[3-Brom-2-(2'-carboxy-phenyl)-benzo[b]thiophen-5-yl-methyl]-N-valeroyl-valin, R_{f}=0,44 (CH₂Cl₂/CH₃OH (4:1)).

Das Ausgangsmaterial kann beispielsweise wie folgt hergestellt werden:
a) Eine Lösung von 8,59 g 5-Methyl-2-trimethylstannyl-benzo[b] ]thiophen, 7,62 g 2-Iod-benzoesäureethylester und 0,31 g Tetrakis-(triphenylphosphin)-palladium in 70 ml Toluol wird 11 Stunden unter Rückfluss erhitzt. Der Eindampfrückstand liefert nach Chromatographie an Kieselgel 60 (40-63 µm ) mit Hexan/Essigester (9:1) als Elutionsmittel 2-(2'-Ethoxycarbonyl-phenyl)-5-methyl-benzo[b]thiophen, R_{f} = 0,33 (Hexan/Essigester (9:1)).
b) Eine Mischung von 7,97 g 2-(2'-Ethoxycarbonyl-phenyl)-5-methyl-benzo[b]thiophen, 4,58 g N-Bromsuccinimid und 0,10 g Azo-isobutyronitril in 100 ml Tetrachlorkohlenstoff wird 3 Stunden unter Rückfluss erhitzt. Die abgekühlte Mischung wird mit 50 ml CH₂Cl₂ verdünnt und mit 20 ml 2N Natronlauge ausgezogen. Die alkalische Phase wird mit 50 ml CH₂Cl₂ extrahiert. Die organischen Phasen liefern nach Aufarbeitung gemäss Beispiel 2 ein Rohprodukt, aus welchem nach Chromatographie an Kieselgel 60 (40-63 µm) mit Hexan/Essigester (9:1) 3-Brom-5-brommethyl-2-(2'-ethoxycarbonyl-phenyl)-benzo[b]-thiophen, R_{f}=0,26 (Hexan/Essigester (9:1)), erhalten wird.
c) Analog Beispiel 1 d) wird N-[3-Brom-2-(2'-ethoxycarbonyl-phenyl)-benzo[b]thiophen-5-yl-methyl]-N-valin-benzylester, R_{f} = 0,76 (CH₂Cl₂/CH₃OH (95:5)), erhalten.
d) Analog Beispiel 1 e) wird N-[3-Brom-2-(2'-ethoxycarbonyl-phenyl)-5-yl-methylbenzo [b]thiophen]-N-valeroyl-valin-benzylester, R_{f} = 0,74 (CH₂Cl₂/CH₃OH (95:5)), erhalten.

### Beispiel 6:

Eine Lösung von 1,42 g N-[2-(2'-(1H-Tetrazol-5-yl)-phenyl)-benzofuran-5-yl-methyl]-N-valeroyl-2-aminomethyl-2-ethyl-buttersäureethylester und 10 ml 2N Kalilauge in 20 ml Ethanol wird 48 Stunden bei Raumtemperatur gerührt. Nach Entfernen des Lösungsmittels wird angesäuert und mit zweimal 50 ml CH₂Cl₂ extrahiert. Das nach Aufarbeitung gemäss Beispiel 2 erhaltene Rohprodukt liefert nach Chromatographie an Kieselgel 60 (40-63 µm) mit CH₂Cl₂/CH₃OH (95:5) als Elutionsmittel N-[2-(2'-(1H-Tetrazol-5-yl)-phenyl)-benzofuran-5-yl-methyl]-N-valeroyl-2-aminomethyl-2-ethylbuttersäure , R_{f} = 0,14 (CH₂Cl₂/CH₃OH (4:1)).

Das Ausgangsmaterial kann beispielsweise wie folgt hergestellt werden:
a) Eine Mischung von 20 g 2-Hydroxy-5-methyl-benzaldehyd, 28 g α-Brom-o-tolunitril und 22 g Kaliumcarbonat in 220 ml Ethanol wird 2 Stunden unter Rückfluss erhitzt. Die abgekühlte Mischung wird filtriert und eingedampft. Der Rückstand wird in 300 ml CH₂Cl₂ gelöst und mit 80 ml gesätigter NaHCO₃-Lösung und mit zweimal 100 ml Wasser gewaschen. Die wässrigen Phasen werden mit 100 ml CH₂Cl₂ nachextrahiert. Die organischen Phasen liefern nach Aufarbeitung gemäss Beispiel 2 ein Rohprodukt, aus welchem durch Lösen in 500 ml CH₂Cl₂, Verrühren mit 100 g Kieselgel 60, Nutschen und Entfernen des Lösungsmittels 2-Formyl-3-methyl-phenyl-2'-cyano-1'-benzyl-ether, R_{f} = 0,25 (CH₂Cl₂), erhalten wird.
b) Eine Lösung von 23,3 g 2-Formyl-3-methyl-phenyl-2'-cyano-1'-benzyl-ether in 230 ml Dimethylformamid wird mit 17,7 ml einer 5,25 M Lösung von Natriummethylat in Methanol gemischt und 1,5 Stunden auf 125° erhitzt. Nach dem Abkühlen wird das Lösungsmittel entfernt, der Rückstand in 1500 ml Ether und 300 ml Wasser aufgenommen und angesäuert. Die organische Phase wird mit zweimal 200 ml Wasser gewaschen und liefert nach Aufarbeitung gemäss Beispiel 2 ein Rohprodukt, aus welchem nach Chromatographie an Kieselgel 60 (40-63 µm) mit CH₂Cl₂ als Elutionsmittel 2-(2'-Cyanophenyl)-5-methyl-benzofuran, R_{f} = 0,53 (CH₂Cl₂), erhalten wird.
c) Eine Mischung von 3,1 g 2-(2'-Cyanophenyl)-5-methyl-benzofuran, 2,6 g N-Bromsuccinimid und 0,05 g Azo-isobutyronitril in 50 ml Tetrachlorkohlenstoff wird 4 Stunden bei 110° gerührt. Die abgekühlte Mischung wird filtriert und das Filtrat wird mit 15 ml 1 N Natronlauge, 30 ml 0,1 N Salzsäure und zweimal 30 ml Wasser gewaschen. Die organische Phase liefert nach üblicher Behandlung 5-Brommethyl-2-(2'-cyanophenyl)-benzofuran, R_{f} = 0,25 (Hexan/Essigester (95:5)).
d) Eine Lösung von 3,5 g 5-Brommethyl-2-(2'-cyanophenyl)-benzofuran, 2,33 g 2-Aminomethyl methyl-2-ethyl-buttersäureethylester, 1,89 ml Triethylamin und 0,27 g 4-Dimethylaminopyridin in 50 ml Tetrahydrofuran wird 30 Stunden bei Raumtemperatur gerührt. Das Reaktionsgemisch wird filtriert und vom Lösungsmittel befreit. Der Rückstand wird in 200 ml CH₂Cl₂ aufgenommen und mit 100 ml gesättigter NaHCO₃-Lösung gewaschen. Die organische Phase liefert nach üblicher Behandlung und Chromatographie an Kieselgel 60 (40-63 µm) mit Hexan/Essigester (5:1) als Elutionsmittel N-[2-(2'-Cyanophenyl)-benzofuran-5-yl-methyl]-2-aminomethyl-2-ethyl-buttersäureethylester, R_{f} = 0,47 (CH₂Cl₂/CH₃OH (95:5)).
e) Analog Beispiel 1 e) wird N-[2-(2'-Cyanophenyl)-benzofuran-5-yl-methyl]-N-valeroyl-2-aminomethyl-2-ethyl-buttersäureethylester, R_{f} = 0,61 (CH₂Cl₂/CH₃OH (95:5)), erhalten.
f) Analog Beispiel 1 f) wird N-[2-(2'-(1H-Tetrazol-5-yl)-phenyl)-benzofuran-5-yl-methyl ]-N-valeroyl-2-aminomethyl-2-ethyl-buttersäureethylester, R_{f}=0,50 (CH₂Cl₂/CH₃OH (4:1)), erhalten.

### Beispiel 7:

Eine Lösung von 0,33 g N-[2-(2'-(1H-Tetrazol-5-yl)-phenyl)-benzofuran-5-yl-methyl]-N-valeroyl-1-aminomethyl-cyclopentan-1-carbonsäureethylester und 2,0 ml Kalilauge in 4,0 ml Ethanol wird 2,5 Stunden unter Rückfluss erhitzt. Die abgekühlte Lösung wird angesäuert, eingeengt und mit 20 ml CH₂Cl₂ und 1 ml Wasser versetzt. Ausziehen mit weiteren 15 ml CH₂Cl₂ und übliche Behandlung der organischen Phasen liefert N-[2-(2'-(1H-Tetrazol-5-yl)-phenyl)-benzofuran-5-yl-methyl]-N-valeroyl-1-aminomethyl-cyclopentan-1-carbonsäure, R_{f} = 0,30 (CH₂Cl₂/CH₃OH(4:1)).

Das Ausgangsmaterial kann beispielsweise wie folgt hergestellt werden:
a) Eine Lösung von 0,312 g 5-Brommethyl-2-(2'-cyanophenyl)-benzofuran, 0,208 g 1-Aminomethyl-cyclopentan-1-carbonsäureethylester, 0,17 ml Triethylamin und 0,024 g 4-Dimethylamino-pyridin in 5,0 ml Tetrahydrofuran wird 20 Stunden bei Raumtemperatur gerührt. Das Gemisch wird vom Lösungsmittel befreit und in 20 ml Essigester aufgenommen und mit 10 ml gesättigter NaHCO₃-Lösung gewaschen. Die organische Phase liefert nach üblicher Behandlung und Chromatographie an Kieselgel 60 (40-63 µm) mit Hexan/Essigester (9:1) als Elutionsmittel N-[2-(2'-Cyanophenyl)-benzofuran-5-yl-methyl ]-1-aminomethyl-cyclopentan-1-carbonsäureethylester, R_{f} = 0,27 (CH₂Cl₂).
b) Analog Beispiel 1 e) wird N-[2-(2'-Cyanophenyl)-benzofuran-5-yl-methyl]-N-valeroyl -1-aminomethyl-cyclopentan-1-carbonsäureethylester, R_{f} = 0,70 (CH₂Cl₂/CH₃OH (95:5)), erhalten.
c) Analog Beispiel 1 f) wird N-[2-(2'-(1H-Tetrazol-5-yl)-phenyl)-benzofuran-5-yl-methyl]-N-valeroyl-1-aminomethyl-cyclopentan-1-carbonsäureethylester, R_{f} = 0,50 (CH₂Cl₂/CH₃OH (4:1)), erhalten.

### Beispiel 8:

Eine Lösung von 0,733 g N-[2-(2'-(1H-Tetrazol-5-yl)-phenyl)-benzofuran-5-yl-methyl]-N-valeroyl-valin-benzylester und 4 ml 2N Natronlauge in 15 ml Ethanol wird 1,5 Stunden unter Rückfluss erhitzt. Die abgekühlte Lösung wird angesäuert und vom Lösungsmittel befreit. Der Rückstand liefert nach Chromatographie an Kieselgel 60 (40-63 µm) mit CH₂Cl₂/CH₃OH (9:1) als Elutionsmittel N-[2-(2'-(1H-Tetrazol-5-yl)-phenyl)-benzofuran-5-yl-methyl]-N-valeroyl-valin, R_{f} = 0,40 (CH₂Cl₂/CH₃OH (4:1)).

Das Ausgangsmaterial kann beispielsweise wie folgt hergestellt werden:
a) Eine Lösung von 4,44 g Valin-benzylester-toluolsulfonsäuresalz, 6,8 ml Hünig-Base und 3,12 g 5-Brommethyl-2-(2'-cyanophenyl)-benzofuran in 20 ml Dimethylformamid wird 1 Stunde auf 80° erwärmt. Zu Beispiel 2 a) analoge Aufarbeitung liefert N-[2-(2'-Cyanophenyl)-benzofuran-5-yl-methyl]-valin-benzylester, R_{f} = 0,20 (CH₂Cl₂/CH₃OH (99:1)).
b) Analog Beispiel 1 e) wird N-[2-(2'-Cyanophenyl)-benzofuran-5-yl-methyl]-N-valeroyl royl-valin-benzylester, R_{f} = 0,25 (CH₂Cl₂/CH₃OH (99:1)), erhalten.
c) Analog Beispiel 1 f) wird N-[2-(2'-(1H-Tetrazol-5-yl)-phenyl)-benzofuran-5-yl-methyl]-N-valeroyl-valin-benzyl-ester, R_{f} = 0,55 (CH₂Cl₂/CH₃OH (4:1)), erhalten.

### Beispiel 9:

Eine Lösung von 3,64 g N-[2-(2'-(1H-Tetrazol-5-yl)-phenyl)-benzo[b]-thiophen-5-yl-methyl]-N-valeroyl-1-aminomethyl-cyclopentan-1-carbonsäureethylester und 13,4 ml 2N Natronlauge in 33 ml Ethanol wird 17 Stunden unter Rückfluss erhitzt. Der Rückstand liefert nach Chromatographie an Kieselgel 60 (40-63 µm) mit CH₂Cl₂/CH₃OH (9:1) als Elutionsmittel N-[2-(2'-(1H-Tetrazol-5-yl)-phenyl)-5-yl-methylbenzo [b]thiophen]-N-valeroyl-1-aminomethyl-cyclopentan-1-carbonsäure, R_{f} = 0,55 (CH₂Cl₂/CH₃OH (4:1)).

Das Ausgangsmaterial kann beispielsweise wie folgt hergestellt werden:
a) Eine Mischung von 3,28 g 5-Brommethyl-2-(2'-cyanophenyl)-benzo[b]thiophen und 4,28 g 1-Aminomethyl-cyclopentan-1-carbonsäureethylester wird 30 Minuten auf 100° erwärmt. Die erkaltete Mischung wird in 100 ml Essigester aufgenommen und mit je 50 ml gesättigter NaHCO₃-Lösung und Wasser gewaschen. Die Waschphasen werden mit 50 ml Essigester rückextrahiert. Die organischen Phasen liefern nach üblicher Behandlung und Chromatographie an Kieselgel 60 (40-63 µm) mit CH₂Cl₂/CH₃OH (98:2) als Elutionsmittel N-[2-(2'-Cyanophenyl)-benzo[b]thiophen-5-yl-methyl]-1-aminomethyl-cyclopentan-1-carbonsäureethylester, R_{f} = 0,38 (CH₂Cl₂/CH₃OH (95:5)).
b) Analog Beispiel 1 e) wird N-[2-(2'-Cyanophenyl)-benzo[b]thiophen-5-yl-methyl]-N-valeroyl-1-aminomethyl-cyclopentan-1-carbonsäureethylester, R_{f} = 0,67 (CH₂Cl₂/CH₃OH (95:5)), erhalten.
c) Analog Beispiel 1 f) wird N-[2-(2'-(1H-Tetrazol-5-yl)-phenyl)-benzo[b]thiophen-5-yl-methyl]-N-valeroyl-1-aminomethyl-cyclopentan-1-carbonsäureethylester, R_{f} = 0,57 (CH₂Cl₂/CH₃OH (4:1)), erhalten.

### Beispiel 10:

Eine Lösung von 1,87 g N-[2-(2'-(1H-Tetrazol-5-yl)-phenyl)-5-yl-methylbenzo[b]thiophen]-N-valeroyl-valin-benzylester und 8 ml 2N Natronlauge in 25 ml Ethanol wird 1,5 Stunden unter Rückfluss erhitzt. Die abgekühlte Lösung wird angesäuert und eingedampft. Der Rückstand liefert nach Chromatographie an Kieselgel 60 (40-63 µm) mit CH₂Cl₂/CH₃OH (9:1) als Elutionsmittel N-[2-(2'-(1H-Tetrazol-5-yl)-phenyl)benzo[b]thiophen-5-yl-methyl]-N-valeroyl-valin, R_{f} = 0,31 (CH₂Cl₂/CH₃OH (4:1)).

Das Ausgangsmaterial kann beispielsweise wie folgt hergestellt werden:
a) Analog Beispiel 1 d) wird nach Chromatographie an Kieselgel 60 (40-63 µm) mit CH₂Cl₂/CH₃OH (9:1) als Elutionsmittel N-[2-(2'-Cyanophenyl)-benzo[b]thiophen-5-yl-methyl]-N-valeroyl-valin-benzylester, R_{f} = 0,21 (CH₂Cl₂/CH₃OH (99:1)), erhalten.
b) Analog Beispiel 1 e) wird N-[2-(2'-Cyanophenyl)-benzo[b]thiophen-5-yl-methyl]-N-valeroyl-valin-benzylester, R_{f} = 0,31 (Hexan/Essigester (2:1)), erhalten.
c) Analog Beispiel 1 f) wird N-[2-(2'-(1H-Tetrazol-5-yl)-phenyl)-5-yl-methyl-benzo[b]-thiophen]-N-valeroyl-valin-benzylester, R_{f} = 0,55 (CH₂Cl₂/CH₃OH (4:1)), erhalten.

### Beispiel 11:

Eine Lösung von 2,15 g N-[3-Brom-2-(2'-ethoxycarbonyl-phenyl)-benzo[b]-thiophen-5-yl-methyl]-N-valeroyl-1-aminomethyl-cyclopentan-1-carbonsäureethylester und 20 ml 2N Kalilauge in 20 ml Ethanol wird 2 Tage unter Rückfluss erhitzt. Aufarbeitung analog zu Beispiel 7 liefert N-[3-Brom-2-(2'-carboxyphenyl)-benzo[b]thiophen-5-yl-methyl]-N-valeroyl-1-aminomethyl-cyclopentan-1-carbonsäure, R_{f} = 0,66 (CH₂Cl₂/CH₃OH (4:1)).

Das Ausgangsmaterial kann beispielsweise wie folgt hergestellt werden:
a) Analog Beispiel 1 d) wird N-[3-Brom-2-(2'-ethoxycarbonyl-phenyl)-benzo[b]-thiophen-5-yl-methyl]-1-aminomethyl-cyclopentan- 1-carbonsäureethylester, R_{f} = 0,41 (CH₂Cl₂/CH₃OH (95:5)), erhalten.
b) Analog Beispiel 1 e) wird N-[3-Brom-2-(2'-ethoxycarbonyl-phenyl)-benzo[b]thiophen-5-yl-methyl]-N-valeroyl-1-aminomethyl-cyclopentan-1-carbonsäureethylester, R_{f} = 0,63 (CH₂Cl₂/CH₃OH (95:5)), erhalten.

### Beispiel 12:

Eine Lösung von 5,20 g N-[2-(2'-(1H-Tetrazol-5-yl)-phenyl)-benzofuran-5-yl-methyl]-N-valeroyl-1-aminomethyl-cyclohexan-1-carbonsäureethylester und 50 ml 2N Kalilauge in 100 ml Ethanol wird 18 Stunden unter Rückfluss erhitzt. Die abgekühlte Lösung wird angesäuert und eingedampft. Der Rückstand liefert nach Chromatographie an Kieselgel 60 (40-63 µm) mit CH₂Cl₂/CH₃OH (95:5) als Elutionsmittel N-[2-(2'-(1H-Tetrazol-5-yl)-phenyl)-benzofuran-5-yl-methyl]-N-valeroyl-1-aminomethyl-cyclohexan-1-carbonsäure, R_{f} = 0,50 (CH₂Cl₂/CH₃OH (4:1)).

Das Ausgangsmaterial kann beispielsweise wie folgt hergestellt werden:
a) 1-Aminomethyl-cyclohexan-1-carbonsäureethylester wird erhalten durch hydrieren von 72,08g 1-Cyano-cyclohexan-1-carbonsäureethylester (T. Kurihara et al. Tet. Lett. 1976, 2455) in 600 ml Ethanol, der ca. 4 % Ammoniak enthält, in Gegenwart von 20 g Raney-Nickel bei 45° und unter Normaldruck. Nach Entfernen des Katalysators und Lösungsmittels wird das Produkt durch Destillation erhalten, Siedepunkt 72-75° bei 0,3 mbar.
b) Analog Beispiel 9 a) wird N-[2-(2'-Cyanophenyl)-benzofuran-5-yl-methyl]-1-aminomethyl-cyclohexan-1-carbonsäureethylester, R_{f} = 0,36 (CH₂Cl₂/CH₃OH (95:5)), erhalten.
c) Analog Beispiel 1 e) wird N-[2-(2'-Cyanophenyl)-benzofuran-5-yl-methyl]-N-valeroyl-1-aminomethyl-cyclohexan-1-carbonsäureethylester, R_{f} = 0,64 (CH₂Cl₂/CH₃OH (95:5)), erhalten.
d) Analog Beispiel 1 f) wird N-[2-(2'-(1H-Tetrazol-5-yl)-phenyl)-benzofuran-5-yl-methyl]-N-valeroyl-1-aminomethyl-cyclohexan-1-carbonsäureethylester, R_{f} = 0,52 (CH₂Cl₂/CH₃OH (4:1)), erhalten.

### Beispiel 13:

In analoger Weise, beispielsweise wie in einem der Beispiele 1-12 beschrieben, kann man herstellen:
N-[2-(2'-(Tetrazol-5-yl)-phenyl)-naphthalin-6-yl-methyl]-N-valeroyl-valin;
N-[2-(2'-(Tetrazol-5-yl)-phenyl)-naphthalin-6-yl-methyl]-N-valeroyl-1-aminomethyl-cyclopentan-1-carbonsäure;
N-[2-(2'-(Tetrazol-5-yl)-phenyl)-naphthalin-6-yl-methyl]-N-valeroyl-1-amino-cyclopentan-1-carbonsäure;
N-[2-(2'-(Tetrazol-5-yl)-phenyl)-naphthalin-6-yl-methyl]-N-valeroyl-1-amino-cyclohexan-1-carbonsäure;
N-[2-(2'-Carboxy-phenyl)-naphthalin-6-yl-methyl]-N-valeroyl-1-aminomethyl-cyclopentan-1-carbonsäure;
N-[2-(2'-(Tetrazol-5-yl)-phenyl)-naphthalin-6-yl-methyl]-N-valeroyl-2-aminomethyl-2-ethyl-buttersäure;
N-[2-(2'-(Tetrazol-5-yl)-phenyl)-naphthalin-6-yl-methyl]-N-valeroyl-2-amino-propionsäure;
N-[2-(2'-(Tetrazol-5-yl)-phenyl)-naphthalin-6-yl-methyl]-N-valeroyl-2-amino-2-methylpropionsäure;
N-[2-(2'-(Tetrazol-5-yl)-phenyl)-naphthalin-6-yl-methyl]-N-valeroyl-1-aminomethyl-cyclohexan-1-carbonsäure;
N-[2-(2'-(Tetrazol-5-yl)-phenyl)-naphthalin-6-yl-methyl]-N-butyryl-1-aminomethyl-cyclopentan-1-carbonsäure;
N-[2-(2-Carboxy-phenyl)-naphthalin-6-yl-methyl]-N-butyryl-1-aminomethyl-cyclopentan-1-carbonsäure;
N-[2-(2'-(Tetrazol-5-yl)-phenyl)-chinolin-6-yl-methyl]-N-cyclopropylcarbonyl-valin;
N-[2-(2'-(Tetrazol-5-yl)-phenyl)-chinolin-6-yl-methyl]-N-cyclopropylcarbonyl-1-aminomethyl-cyclopentan-1-carbonsäure;
N-[2-(2'-(Tetrazol-5-yl)-phenyl)-chinolin-6-yl-methyl]-N-cyclopropylcarbonyl-2-aminomethyl-2-ethyl-buttersäure;
N-[2-(2'-(Tetrazol-5-yl)-phenyl)-chinolin-6-yl-methyl]-N-cyclopropylcarbonyl-1-aminomethyl-cyclohexan-1-carbonsäure;
N-[2-(2'-(Tetrazol-5-yl)-phenyl)-naphthalin-6-yl-methyl]-N-propyloxycarbonyl-valin;
N-[2-(2'-(Tetrazol-5-yl)-phenyl)-naphthalin-6-yl-methyl]-N-propyloxycarbonyl-1-aminomethyl-cyclopentan-1-carbonsäure;
N-[2-(2'-(Tetrazol-5-yl)-phenyl)-naphthalin-6-yl-methyl]-N-propyloxycarbonyl-2-aminomethyl-2-ethyl-buttersäure;
N-[2-(2-(Tetrazol-5-yl)-phenyl)-naphthalin-6-yl-methyl]-N-propyloxycarbonyl-1-aminomethyl-cyclohexan-1-carbonsäure;
N-[2-(2'-(Tetrazol-5-yl)-phenyl)-naphthalin-6-yl-methyl]-N-ethoxycarbonyl-valin;
N-[2-(2'-(Tetrazol-5-yl)-phenyl)-naphthalin-6-yl-methyl]-N-ethoxycarbonyl-1-aminomethyl-cyclopentan-1-carbonsäure;
N-[2-(2'-(Tetrazol-5-yl)-phenyl)-naphthalin-6-yl-methyl]-N-ethoxycarbonyl-2-aminomethyl-2-ethyl-buttersäure;
N-[2-(2'-(Tetrazol-5-yl)-phenyl)-naphthalin-6-yl-methyl]-N-ethoxycarbonyl-1-aminomethyl-cyclohexan-1-carbonsäure;
N-[2-(2'-(Tetrazol-5-yl)-phenyl)-chinolin-6-yl-methyl]-N-propyloxycarbonyl-valin;
N-[2-(2'-(Tetrazol-5-yl)-phenyl)-chinolin-6-yl-methyl]-N-propyloxycarbonyl-1-aminomethyl-cyclopentan-1-carbonsäure;
N-[2-(2'-(Tetrazol-5-yl)-phenyl)-chinolin-6-yl-methyl]-N-propyloxycarbonyl-2-aminomethyl-2-ethyl-buttersäure;
N-[2-(2'-(Tetrazol-5-yl)-phenyl)-chinolin-6-yl-methyl]-N-ethoxycarbonyl-1-aminomethyl-cyclohexan-1-carbonsäure;
N-[2-(2'-(Tetrazol-5-yl)-phenyl)-chinolin-6-yl-methyl]-N-ethoxycarbonyl-valin; N-[2-(2'-(Tetrazol-5-yl)-phenyl)-chinolin-6-yl-methyl]-N-ethoxycarbonyl-1-aminomethyl-cyclopentan-1-carbonsäure;
N-[2-(2'-(Tetrazol-5-yl)-phenyl)-chinolin-6-yl-methyl]-N-ethoxycarbonyl-2-aminomethyl-2-ethyl-buttersäure;
N-[2-(2'-(Tetrazol-5-yl)-phenyl)-chinolin-6-yl-methyl]-N-ethoxycarbonyl-1-aminomethyl-cyclohexan-1-carbonsäure;
N-[2-(2'-(Tetrazol-5-yl)-phenyl)-chinolin-6-yl-methyl]-N-valeroyl-1-aminomethyl-cyclohexan-1-carbonsäure;
N-[2-(2'-(Tetrazol-5-yl)-phenyl)-chinolin-6-yl-methyl]-N-valeroyl-1-amino-cyclohexan-1-carbonsäure;
N-[2-(2'-(Tetrazol-5-yl)-phenyl)-chinolin-6-yl-methyl]-N-valernyl-1-amino-cyclopentan-1-carbonsäure;
N-[2-(2'-(Tetrazol-5-yl)-phenyl)-chinolin-6-yl-methyl]-N-butyryl-1-aminomethyl-cyclopentan-1-carbonsäure;
N-[2-(2'-Carboxy-phenyl)-naphthalin-6-yl-methyl]-N-valeroyl-valin;
N-[2-(2'-(Tetrazol-5-yl)-phenyl)-naphthalin-6-yl-methyl]-N-butyryl-valin;
N-[2-(2'-Carboxy-phenyl)-naphthalin-6-yl-methyl]-N-butyryl-valin;

### Beispiel 14:

Eine Lösung von 1.675 g N-[2-(2'-Cyanophenyl)-naphthalin-6-ylmethyl]-N-valeroyl-1-aminomethyl-cyclopentan-1-carbonsäureethylester und 1.46 g Tributylzinnazid in 36 ml Xylol wird 23 Stunden unter Rückfluss erhitzt. Die abgekühlte Lösung wird mit 10 ml 2N Kalilauge 1 Stunde gerührt. Die wässrige Phase wird mit 40 ml Ether extrahiert, dann angesäuert und mit dreimal 30 ml CH₂Cl₂ ausgezogen. Das sich zwischen der wässrigen und der organischen Phase abscheidende Oel wird separat gesammelt. Dieses liefert nach Chromatographie an Kieselgel 60 (0.040 - 0.063 mm) mit CH₂Cl₂/CH₂OH (9:1) als Elutionsmittel N-[2-(2'-(1H-Tetrazol-5-yl)-phenyl)-naphthalin--6-ylmethyl] -N-valeroyl-1-amino-methyl-cyclopentan-1-carbonsäureethylester, R_{f} = 0,31 (CH₂Cl₂/CH₃OH (9:1)).

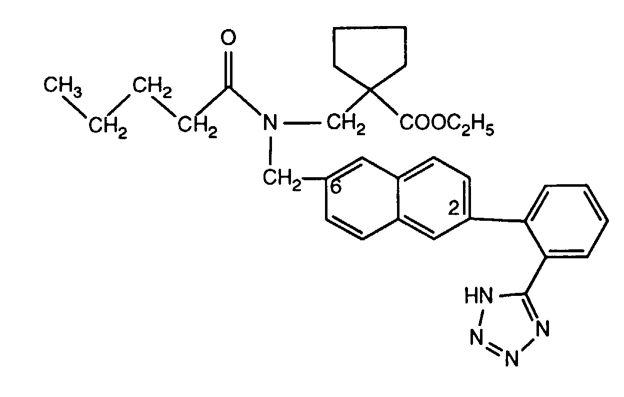

Das Ausgangsmaterial kann beispielsweise wie folgt hergestellt werden:
a) Zu einer aus 4.69 g Magnesium und 42.59 g 2-Brom-6-methylnaphthalin (R.G. Jones et al., J.Amer.Chem.Soc.70, 2843 (1948)) in 420 ml Tetrahydrofuran bereiteten Grignard-Lösung wird bei 15° eine Lösung von 4,5-Dihydro-2-(2-methoxyphenyl)-4,4-dimethyloxazol (I.A.Meyers, M.A.Hanagan & A.L.Mazzu, Heterocycles 15, 361 (1981)) in 400 ml Tetrahydrofuran zugetropft. Nach 4-stündigem Rühren bei Raumtemperatur wird auf eine Lösung von 60 g Ammoniumchlorid in 1500 ml Wasser gegossen und mit dreimal 500 ml Ether extrahiert. Die über MgSO₄ getrocknete vereinte Etherphase wird vom Lösungsmittel im Vakuum befreit. Umkristallisation aus heissem Cyclohexan gibt 4,5-Dihydro-2-[2-(6'-methylnaphth-2'-yl)-phenyl]-4,4-dimethyl-oxazol, Smp. 153 - 155°.
b) Eine Mischung von 63.8 g 4,5-Dihydro-2-[2-(6'-methylnaphth-2'-yl)-phenyl]-4,4-dimethyl-oxazol, 44.3 g Phosphoroxychlorid und 22.8 g Pyridin in 650 ml Toluol wird 2.5 Stunden zum Rückfluss erhitzt. Die auf 0° gekühlte Reaktionsmischung wird mit Na₂CO₃-Lösung versetzt (pH=8) und sodann mit dreimal 500 ml Essigester extrahiert. Die Essigester-Phasen werden mit dreimal 100 ml Wasser gewaschen, über MgSO₄ getrocknet und im Vakuum vom Lösungsmittel befreit. Das so erhaltene Rohprodukt liefert nach Chromatographie an Kieselgel 60 (0.040 - 0.063 mm) mit Hexan/CH₂Cl₂/Essigester (9:1:1) als Elutionsmittel 6-Methyl-2-(2'-cyanophenyl)-naphthalin, Smp. 100 - 104°.
c) Eine Mischung von 6.30 g 6-Methyl-2-(2'-cyanophenyl)-naphthalin, 4.60 g N-Bromsuccinimid und 0.085 g Azoisobutyronitril in 70 ml Tetrachlorkohlenstoff wird 4 Stunden auf 110° erhitzt. Das abgekühlte Reaktionsgemisch wird mit zweimal 45 ml 2N Natronlauge extrahiert. Die wässrigen Phasen werden mit je 50 ml CH₂Cl₂ rückextrahiert. Die vereinten organischen Phasen werden über MgSO₄ getrocknet. Nach Entfernen des Lösungsmittels im Vakuum wird das Rohprodukt aus Toluol/Hexan umkristallisiert und liefert 6-Brommethyl-2-(2'-cyanophenyl)-naphthalin, Smp. 171 - 172°.
d) Eine Lösung von 1.290 g 6-Brommethyl-2-(2'-cyanophenyl)-naphthalin, 0.694 g 1-Aminomethyl-cyclopentan-1-carbonsäureethylester und 0.71 ml N,N-Diiso-propyl-N-ethylamin in 10 ml Toluol wird 3 Stunden auf 80° erwärmt. Vom gebildeten Niederschlag wird mit Hilfe von 30 ml Ether abfiltriert. Das Filtrat wird mit 20 ml Ether verdünnt und mit dreimal 10 ml Wasser extrahiert. Die organische Phase gibt nach Trocknen über Na₂SO₄ und Entfernen der Lösungsmittel im Vakuum ein viskoses Oel. Das erhaltene Rohprodukt liefert nach Chromatographie an Kieselgel 60 (0.040 - 0.063 mm) mit CH₂Cl₂/CH₃OH (98:2) als Elutionsmittel N-[2-(2'-Cyanophenyl)-naphthalin--6-ylmethyl]-1-aminomethyl-cyclopentan-1-carbonsäureethylester, R_{f} = 0,33 (CH₂Cl₂/CH₃OH (95:5)).
e) Eine Lösung von 1.40 g N-[2-(2'-Cyanophenyl)-naphthalin-6-ylmethyl]-1-aminomethyl-cyclopentan-1-carbonsäureethylester, 0.49 ml Triethylamin und 0.41 ml Valeroylchlorid in 14 ml CH₂Cl₂ wird 2,5 Stunden bei Raumtemperatur gerührt, dann mit 50 ml Ether verdünnt und nacheinander mit 7 ml 1N Kalilauge, 5 ml 1N Salzsäure, 5 ml gesättigter NaHCO₃-Lösung und zweimal 5 ml Wasser gewaschen. Die über Na₂SO₄ getrocknete organische Phase wird im Vakuum von den Lösungsmittel befreit und liefert reinen N-[2-(2'-Cyanophenyl)-naphthalin-6-ylmethyl]-N-valeroyl-1-aminomethyl-cyclopentan-1-carbonsäure-ethyl-ester, R_{f} = 0,69 (CH₂Cl₂/CH₃OH (95:5)).

### Beispiel 15:

Eine Mischung von 0.888 g N-[2-(2'-(1H-Tetrazol-5-yl)-phenyl)-naphthalin-6-ylmethyl]-N-valeroyl-1-aminomethyl-cyclopentan-1-carbonsäur eethylester, 8 ml Ethanol und 5 ml 2N Kalilauge wird 2,5 Stunden zum Rückfluss erhitzt. Die abgekühlte Reaktionslösung wird neutralisiert und im Vakuum eingedampft. Der Rückstand wird in 30 ml CH₂Cl₂ aufgenommen und mit 5 ml 1N Kalilauge extrahiert. Die alkalische Phase wird mit 3 ml 4N Salzsäure auf pH = 1 gestellt und mit dreimal 25 ml CH₂Cl₂ ausgezogen. Trocknen der CH₂Cl₂-Phasen über Na₂SO₄ und Entfernen des Lösungsmittels im Vakuum liefert N-[2-(2'-(1H-Tetrazol-5-yl)-phenyl)-naphthalin-6-ylmethyl]-N-valeroyl-1-aminomethyl-cyclopentan-1-carbonsäure, R_{f} = 0,21 (CH₂Cl₂/CH₃OH (9:1)).

### Beispiel 16:

Eine Lösung von 2.30 g N-[2-(2'-Cyanophenyl)-6-ylmethyl-naphthalin]-N-valeroyl-3-amino-2,2-dimethyl-propionsäureethylester und 2.16 g Tributylzinnazid in 50 ml Xylol wird 16 Stunden unter Rückfluss erhitzt. Die abgekühlte Lösung wird mit 50 ml 2N Kalilauge während 30 Minuten verrührt. Die wässrige Phase wird abgetrennt, mit 30 ml 4N Salzsäure angesäuert und mit zweimal 25 ml CH₂Cl₂ extrahiert. Nach Trocknung über Na₂SO₄ und Entfernen des Lösungsmittels im Vakuum wird der Rückstand in 25 ml Ethanol gelöst, mit 10 ml 2N Kalilauge gemischt und 3 Stunden unter Rückfluss erhitzt. Die abgekühlte Reaktionsmischung wird mit 8 ml 4N Salzsäure sauer gestellt, im Vakuum eingedampft und liefert nach Chromatographie an Kieselgel 60 (0.040 - 0.063 mm) mit CH₂Cl₂/CH₃OH (9:1) als Elutionsmittel N-[2-(2'-(1H-Tetrazol-5-yl)-phenyl-naphthalin-6-ylmethyl]-N-valeroyl-3-amino-2,2-dimethyl-propionsäure, R_{f} = 0,55 (CH₂Cl₂/CH₃OH (4:1)).

Das Ausgangsmaterial kann beispielsweise wie folgt hergestellt werden:
a) Eine Mischung von 1.82 g 3-Amino-2,2-dimethyl-propionsäureethylester und 1.61 g 6-Brommethyl-2-(2'-cyanophenyl)-naphthalin wird während 15 Minuten auf 100° erwärmt und sodann in 100 ml Essigester aufgenommen, der mit 50 ml 1N Salzsäure und 50 ml gesättigter NaHCO₃-Lösung extrahiert wird. Die organische Phase liefert nach Trocknen über Na₂SO₄ und Entfernen des Lösungsmittels im Vakuum N-[2-(2'-Cyanophenyl)-naphthalin-6-ylmethyl]-3-amino-2,2-dimethyl-propionsäureethyl ester, R_{f} = 0,45 (CH₂Cl₂/CH₃OH (95:5)).
b) Eine Lösung von 1.90 g N-[2-(2'-Cyanophenyl)-naphthalin-6-ylmethyl]-3-amino-2,2-dimethyl-propionsäureethylester, 1.32 ml N,N-Diiso-propyl-N-ethylamin und 0.72 ml Valeroylchlorid in 50 ml Toluol wird 1 Stunde bei Raumtemperatur gerührt. Die Reaktionsmischung wird nacheinander mit zweimal 25 ml 1N Salzsäure, 25 ml Wasser, 25 ml gesättigter NaHCO₃-Lösung und 25 ml Sole gewaschen. Die organische Phase liefert nach Trocknen über Na₂SO₄ und Entfernen des Lösungsmittels im Vakuum N-[2-(2'-Cyanophenyl)-naphthalin-6-ylmethyl]-N-valeroyl-3-amino-2,2-dimethyl-propionsäureethylester, R_{f} = 0,35 (CH₂Cl₂/CH₃OH (95:5)).

### Beispiel 17:

Eine Lösung von 1.6 g N-[2-(2'-(1H-Tetrazol-5-yl)-phenyl)-naphthalin)-6-yl-methyl]-N-butyryl-valin-benzylester in 30 ml 2N NaOH und 50 ml Methanol wird 1/2 Stunde unter Rückfluss erhitzt. Die abgekühlte Lösung wird faserfrei filtriert und das Methanol am Rotavap entfernt. Nach Ansäuern mit HCl kristallisiert das N-[2-(2'-(1H-Tetrazol-5-yl)-phenyl)-naphthalin)-6-yl-methyl]-N-butyryl-valin mit einem Wassergehalt von ca. 2.5 % als farblose Kristalle. Smp: 115-125°; R_{f} = 0.16 (CH₂Cl₂/Ethanol (9:1))

Das Ausgangsmaterial kann beispielsweise wie folgt hergestellt werden:
a) Eine Lösung von 6.58 g Valin-benzylester-toluolsulfonsäuresalz, 5.9 ml Hünig-Base und 2.8 g 6-Brommethyl2-(2'-cyanophenyl)-naphthalin in 30 ml N,N-Dimethylformamid wird während 2 Stunden auf 80° erwärmt. Die abgekühlte Reaktionslösung wird auf 100 ml 1% ige NaHCO₃-Lösung ausgegossen und das Produkt mit 3 x 100 ml Essigester extrahiert. Die organischen Phasen werden mit 3 x 50 ml Wasser gewaschen, vereinigt, über Magnesiumsulfat getrocknet, filtriert und eingedampft. Das Rohprodukt von 5.8 g wird über Kieselgel 60 (0,04 - 0,063 mm) mit Hexan/Essigester (4:1) als Elutionsmittel chromatographiert. Man erhält N-[2-(2'-Cyanophenyl)-naphthalin-6-ylmethyl]-valin-benzylester als gelbes Oel. R_{f} = 0.3 (Hexan/Essigester (4:1))
b) Eine Lösung von 1.95 g N-[2-(2'-Cyanophenyl)-naphthalin-6-ylmethyl]-N-butyrylvalin-benzylester, 1.1 ml Hünig-Base und 0.55 g Buttersäurechlorid in 20 ml Essigester wird bei Raumtemperatur während 18 Stunden gerührt. Man verdünnt mit 50 ml Essigester, wäscht einmal mit 25 ml 2N Sodalösung und zweimal mit 25 ml Wasser. Die wässrigen Phasen werden zweimal mit je 50 ml Essigester rückextrahiert. Die organischen Phasen nach Trocknen und Eindampfen ergeben N-[2-(2'-Cyanophenyl)-naphthalin-6-yl-methyl]-N-butyryl-valin-benzylester als dickes gelbes Oel. R_{f} = 0.2 (Hexan/Essigester (4:1))
c) Eine Lösung von N-[2-(2'-Cyanophenyl)-naphthalin-6-ylmethyl]-N-butyryl-valinbenzylester und 2 g Tributylzinnazid in 10 ml Xylol-Isomerengemisch wird 24 Stunden am Rückfluss gekocht. Nach Abkühlen auf Raumtemperatur mit 5 ml 5N HCl in Ether versetzt und 1/2 Stunde bei Raumtemperatur gerührt. Man setzt 80 ml Cyclohexan zu und dekantiert anschliessend die überstehende Lösung. Der Rückstand wird noch 2 Mal mit je 50 ml Cyclohexan verrieben, die überstehende Lösung abdekantiert und verworfen. Der Rückstand wird über Kieselgel 60 chromatographiert. Man erhält N-[2-(2'-(1H-Tetrazol-5-yl)-phenyl)-naphthalin)-6-ylmethyl]-N-butyryl-valin-benzylester als gelben Schaum; R_{f} = 0.32 (CH₂Cl₂/Ethanol (95:5))

### Beispiel 18:

Analog Beispiel 17 werden aus N-[2-(2'-(1H-Tetrazol-5-yl)-phenyl)naphthalin)-6-ylmethyl]-N-valeroyl-valinbenzylester durch Hydrolyse mit NaOH/Methanol und anschliessende Aufarbeitung das N-[2-(2'-(1H-Tetrazol-5-yl)-phenyl)-naphthalin)-6-ylmethyl]-N-valeroyl-valin mit einem Wassergehalt von 2.5 % erhalten. Fp: 118-127°; R_{f} = 0.37 (CH₂Cl₂/Ethanol (9:1)).

### Beispiel 19:

In analober Weise, beispielsweise wie in einem der vorstehenden Beispiele beschrieben bzw. ausgehend von einer der vorstehend beschriebenen Produkte, kann man herstellen:
N-[2-(2'-(1H-Tetrazol-5-yl)-phenyl)-naphthalin-6-ylmethyl]-N-valeroyl-1-aminomethyl-1-hydroxymethyl-cyclopentan;
N-[2-(2'-(1H-Tetrazol-5-yl)-phenyl)-naphthalin-6-ylmethyl]-N-valeroyl-1-aminomethyl-1-formylcyclopentan;
N-[2-(2'-(1H-Tetrazol-5-yl)-phenyl)-chinolin-6-ylmethyl]-N-valeroyl-1-aminomethyl-1-hydroxymethyl-cyclopentan;
N-[2-(2'-(1H-Tetrazol-5-yl)-phenyl)-chinolin-6-ylmethyl]-N-valeroyl-1-aminomethyl-1-formyl-cyclopentan;
N-[2-(2'-(1H-Tetrazol-5-yl)-phenyl)-naphthalin-6-ylmethyl]-N-valeroyl-N-1-hydroxymethyl-isobutan;
N-[2-(2'-(1H-Tetrazol-5-yl)-phenyl)-naphthalin-6-ylmethyl]-N-valeroyl-N-1-formyl-isobutan;
N-[2-(2'-(1H-Tetrazol-5-yl)-phenyl)-chinolin-6-ylmethyl]-N-valeroyl-N-1-hydroxymethyl-isobutan;
N-[2-(2'-(1H-Tetrazol-5-yl)-phenyl)-chinolin-6-ylmethyl]-N-valeroyl-N-1-formylisobutan.

### Beispiel 20:

Eine Lösung 2,93 g N-(3-Brom-2-[2'-(1-trityl-tetrazol-5-yl)-phenyl]benzofuran-5-ylmethyl}-N-valeroyl-1-aminomethyl-cyclopentan-1-carbonsäureethylester und 0,5 ml konz. Salzsäure in 50 ml Methanol wird während 1,5 Stunden bei Raumtemperatur gerührt. Die abgekühlte Lösung wird mit 10 ml 2N Natronlauge alkalisch gestellt und eingedampft. Der Rückstand wird in 50 ml Wasser aufgenommen und mit 100 ml Ether extrahiert. Die wässrige Phase wird mit 15 ml 2N Salzsäure angesäuert und mit 100 sowie 50 ml Essigester extrahierL Die Essigester-Phasen liefern nach Waschen mit je 50 ml Wasser und Sole und Befreien vom Lösungsmittel im Vakuum N-{3-Brom-2-[2'-(1H-tetrazol-5-yl)-phenyl]-benzofuran-5-ylmethyl}-N-valeroyl-1-aminomethyl-cyclopentan-1-carbonsäure-ethylester, R_{f}= 0,52 (CH₂Cl₂/CH₃OH (4:1)). 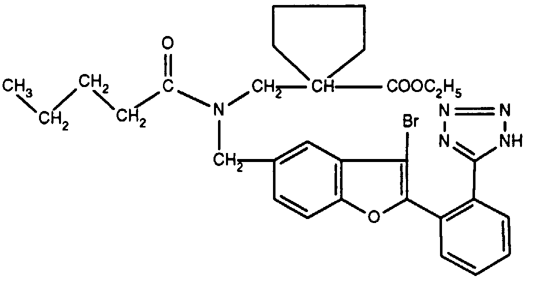 In analoger Weise kann man herstellen:
N-{3-Brom-2-[2'-(1H-tetrazol-5-yl)-phenyl]-benzofuran-5-ylmethyl}-N-butyryl-1-aminomethyl-cyclopentan-1-carbonsäure-ethylester;
N-{3-Brom-2-[2'-(1H-tetrazol-5-yl)-phenyl]-benzofuran-5-ylmethyl}-N-caproyl-1-aminomethyl-cyclopentan-1-carbonsäure-ethylester;
N-{3-Brom-2-[2'-(1H-tetrazol-5-yl)-phenyl]-benzofuran-5-ylmethyl}-N-valeroyl-valinethylester;
N-{3-Brom-2-[2'-(1H-tetrazol-5-yl)-phenyl]-benzofuran-5-ylmethyl}-N-butyryl-valinethylester;
N-{3-Brom-2-[2'-(1H-tetrazol-5-yl)-phenyl]-benzofuran-5-ylmethyl}-N-caproyl-valinethylester;
N-{3-Brom-2-[2'-(1H-tetrazol-5-yl)-phenyl]-benzofuran-5-ylmethyl}-N-valeroyl-alaninethylester.

Das Ausgangsmaterial kann beispielsweise wie folgt hergestellt werden:
a) Eine Mischung von 23,3 g 2-(2'-Cyanophenyl)-5-methyl-benzofuran und 67 g Tributylzinnazid wird langsam auf 160° erwärmt und dann 3 Stunden bei dieser Temperatur gehalten. Die abgekühlte Lösung wird in 600 ml 1N Natronlauge aufgenommen und fünfmal mit je 200 ml Ether extrahiert. Das Produkt wird aus der wässrigen Phase durch Eintropfen von 4N Salzsäure (bis pH=1) gefällt. Durch Nutschen und Trocknen des Niederschlags bei 70° im Vakuum wird 2-[2'-(1H-Tetrazol-5-yl)-phenyl]-5-methyl-benzofuran, R_{f} = 0.51 (CH₂Cl₂/CH₃OH (4:1)) erhalten.
b) Zu einer Suspension von 10,0 g 2-[2'-(1H-Tetrazol-5-yl)-phenyl]-5-methyl-benzofuran in 418 ml Dioxan wird innerhalb von 35 Minuten eine Lösung von 11,6 g Brom in 28 ml Tetrachlorkohlenstoff zugetropft. Nach 3-stündigem Rühren bei Raumtemperatur werden 12,6 ml Cyclohexen zugetropft und danach wird das Reaktionsgemisch im Vakuum eingedampft. Der Rückstand wird in 50 ml 2N Natronlauge aufgenommen und mit 100 ml Ether extrahiert. Es bilden sich drei klare Phasen. Die beiden unteren Phasen werden abgetrennt, mit 40 ml 4N Salzsäure angesäuert und dreimal mit je 100 ml Essigester extrahiert. Die vereinten Essigester-Phasen werden mit 100 ml Sole gewaschen und führen nach Befreiung vom Lösungsmittel im Vakuum zu einem gelben Oel, welches aus Toluol kristallines 3-Brom-2-[2'-(1H-tetrazol-5-yl)-phenyl]-5-methyl-benzofuran, Smp. 167- 168°, liefert.
c) Eine Suspension von 10,37 g 3-Brom-2-[2'-(1H-tetrazol-5-yl)-phenyl]-5-methyl-benzofuran in 300 ml CH₂Cl₂ wird nacheinander mit 5,89 g Triethylamin, 8,14 g Triphenylchlormethan und 0,10 g 4-N,N-Dimethylamino-pyridin versetzt und 3 Stunden bei Raumtemperatur gerührt. Die Reaktionslösung wird mit je 150 ml Wasser und Sole gewaschen, im Vakuum auf ein Volumen von 50 ml eingeengt und an Kieselgel 60 (40-63 µm) mit CH₂Cl₂ als Elutionsmittel chromatographiert. Das Produkt mit R_{f}= 0,82 (CH₂Cl₂/CH₃OH (95:5)) gibt aus Ether kristallines 3-Brom-2-[2'-(1-trityl-tetrazol-5-yl)-phenyl]-5-methyl-benzofuran, Smp. 199-200°.
d) Zu einer 50° warmen Lösung von 15,0 g 3-Brom-2-[2'-(1-trityl-tetrazol-5-yl)-phenyl]-5-methyl-benzofuran in 415 ml Tetrachlorkohlenstoff werden 4,48 g N-Bromsuccinimid und 0,22 g Dibenzoylperoxid gegeben. Die Mischung wird 3,5 Stunden zum Rückfluss erhitzt. Die auf 5° gekühlte Reaktionsmischung wird filtriert. Das Filtrat liefert nach Waschen mit je 100 ml Wasser und Sole, Trocknen über Na₂SO₄, Eindampfen im Vakuum und Aufnehmen in Ether/CH₃OH (1:1) kristallines 3-Brom-5-brommethyl-2-[2'-(1-trityl-tetrazol-5-yl-)-phenyl]-5-methyl-benzofuran, Smp. 192-193°.
e) Eine Lösung von 2,03 g 3-Brom-5-brommethyl-2-[2'-(1-trityl-tetrazol-5-yl)-phenyl]-5-methyl-benzofuran und 1,28 g 1-Aminomethyl-cyclopentan-1-carbonsäureethylester in 25 ml Toluol wird 1 Stunde auf 100° erhitzt. Die mit 25 ml Toluol verdünnte Reaktionslösung wird nacheinander mit je 25 ml 1N Salzsäure, Wasser und Sole gewaschen und liefert nach Entfernen des Lösungsmittels im Vakuum N-{3-Brom-2-[2'-(1-trityl-tetrazol-5-yl)-phenyl]-benzofuran-5-ylmethyl}-1-aminomethyl-cyclopentan-1-carbonsäure-ethylester, R_{f} 0,84 (CH₂Cl₂/CH₃OH (4:1)).
f) Eine Lösung von 2,51 g N-{3-Brom-2-[2'-(1-trityl-tetrazol-5-yl)-phenyl]-benzofuran-5-ylmethyl}-1-aminomethyl-cyclopentan-1-carbonsäureethylester, 0,79 ml Ethyldiisopropylamin und 0,43 ml Valeroylchlorid in 50 ml Toluol wird 1 Stunden bei Raumtemperatur gerührt. Das Reaktionsgemisch wird mit 50 ml Toluol verdünnt und nacheinander mit je 25 ml 1N Salzsäure (zweimal), Wasser, gesättigter NaHCO₃-Lösung und Sole gewaschen. Die wässrigen Phasen werden mit 50 ml Toluol nachextrahiert. Nach Entfernen des Lösungsmittels im Vakuum liefern die vereinten organischen Phasen N-{3-Brom-2-[2'-(1-trityl-tetrazol-5-yl)-phenyl]-benzofuran-5-ylmethyl}-N-valeroyl-1-aminomethyl-cyclopentan-1-carbonsäureethylester, R_{f} 0,23 (CH₂Cl₂/CH₃OH (99:1)).

### Beispiel 21:

Eine Lösung 0,913 g N-{3-Brom-2-[2'-(1H-tetrazol-5-yl)-phenyl]-benzofuran-5-ylmethyl}-N-valeroyl-1-aminomethyl-cyclopentan-1-carbonsäureethylester und 10 ml 2N Natronlauge in 15 ml Ethanol wird 3 Stunden auf 100° erhitzt. Die abgekühlte Lösung wird mit 8 ml 4N Salzsäure angesäuert und im Vakuum eingedampft. Der Rückstand liefert nach Chromatographie an Kieselgel 60 (40-63 µm) mit CH₂Cl₂ /CH₃OH (95:5) als Elutionsmittel reine N-{3-Brom-2-[2'-(1H-tetrazol-5-yl)-phenyl]-benzofuran-5-ylmethyl}-N-valeroyl-1-aminomethyl-cyclopentan-1-carbonsäure, R_{f}= 0,43 (CH₂Cl₂/CH₃OH (4:1)).

In analoger Weise kann man herstellen:
N-{3-Brom-2-[2'-(1H-tetrazol-5-yl)-phenyl]-benzofuran-5-ylmethyl}-N-butyryl-1-aminomethyl-cyclopentan-1-carbonsäure;
N-{3-Brom-2-[2'-(1H-tetrazol-5-yl)-phenyl]-benzofuran-5-ylmethyl}-N-caproyl-1-aminomethyl-cyclopentan-1-carbonsäure;
N-{3-Brom-2-[2'-(1H-tetrazol-5-yl)-phenyl]-benzofuran-5-ylmethyl}-N-valeroyl-valin;
N-{3-Brom-2-[2'-(1H-tetrazol-5-yl)-phenyl]-benzofuran-5-ylmethyl}-N-butyryl-valin;
N-{3-Brom-2-[2'-(1H-tetrazol-5-yl)-phenyl]-benzofuran-5-ylmethyl}-N-caproyl-valin;
N-{3-Brom-2-[2'-(1H-tetrazol-5-yl)-phenyl]-benzofuran-5-ylmethyl}-N-valeroyl-alanin.

### Beispiel 22:

0,4 g N-[2-(2'-(1H-Tetrazol-5-yl)-phenyl)-naphtalin-6-ylmethyl]-N-valeroyl-valin-methylester werden zusammen mit 20 ml einer Lösung von Methylamin in Ethanol (33 % ig) im Autoklaven während 17 Stunden auf 100° erhitzt. Das Reaktionsgemisch wird anschliessend am Rotavap zur Trockne eingedampft. Der Rückstand wird mit 50 ml Essigester und 10 ml HCl 2N im Scheidetrichter geschüttelt. Die wässreige Phase wird abgetrennt und zwei Mal mit je 20 ml Essigester extrahiert. Die organischen Phasen werden zwei Mal mit je 10 ml Wasser gewaschen, vereinigt und über Magnesiumsulfat getrocknet.Nach Filtration und Eindampfen des Filtrates wird der Rückstand über Kieselgel 60 chromatographiert. Man erhält N-[2-(2'-(1H-Tetrazol-5-yl)-phenyl)-naphthalin-6-yl-methyl]-N-valeroyl-valin-methylamid als hellgelbes Harz. R_{f} = 0,14 (CH₂Cl₂/Ethanol 95/5).

Das Ausgangsmaterial kann beispielsweise wie folgt hergestellt werden:
a) Eine Lösung von 3,39 g Valin-methylester-hydrochlorid, 9,1 ml Hünig-Base und 5,42 g 6-Brommethyl-2-(2'-cyanophenyl)-naphtalin in 50 ml N,N-Dimethylformamid wird während 3 Stunden auf 80° erwärmt. Die abgekühlte Reaktionslösung wird auf 200 ml 1 %ige NaHCO₃-Lösung ausgegossen und das Produkt 3 Mal mit je 200 ml Essigester extrahiert. Die organischen Phasen werden mit 3 Mal mit je 100 ml Wasser gewaschen, vereinigt, über Magnesiumsulfat getrocknet, filtriert und eingedampft. Das Rohprodukt von 6,8 g wird über Kieselgel 60 chromatographiert. Man erhält N-[2-(2'-Cyanophenyl)-naphtalin-6-ylmethyl]-valin-methylester als oranges Oel. R_{f}= 0,24 (Hexan/Essigester: 4/1).
b) Eine Lösung von 4,94 g N-[2-(2'-Cyanophenyl)-naphtalin-6-ylmethyl]-valin-methylester, 3,4 ml Hünig-Base und 1,29 g Valeriansäurechlorid in 50 ml Essigester wird bei Raumtemperatur während 18 Stunden gerührt. Man verdünnt mit 150 ml Essigester, wäscht einmal mit 40 ml Sodalösung 2N und zwei Mal mit je 50 ml Wasser. Die wässrigen Phasen werden zwei Mal mit je 100 ml Essigester rückextrahiert. Die organischen Phasen, nach Trocknen und Eindampfen ergeben N-[2-(2'-Cyanophenyl)-naphtalin-6-yl-methyl]-N-valeroyl-valin-methylester als oranges Oel. R_{f}= 0,18 (Hexan/Essigester 4/1).
c) Eine Lösung von 6,68 g N-[2-(2'-Cyanophenyl)-naphtalin-6-ylmethyl]-N-valeroyl-valin-methylester und 6,8 g Tributylzinnazid in 40 ml Xylol-Isomerengemisch wird 48 Stunden unter Rückfluss gekocht. Nach Abkühlen auf Raumtemperatur wird mit 5 ml HCl 5N in Ether versetzt und eine halbe Stunde bei Raumtemperatur gerührt. Man setzt 150 ml Cyclohexan zu und dekantiert anschliessend die überstehende Lösung vom ausgefällten, harzartigen Rückstand. Der Rückstand wird noch zwei Mal mit je 150 ml Cyclohexan verrieben, die überstehende Lösung abdekantiert und verworfen. Der Rückstand wird schliesslich über Kieselgel 60 chromatographiert. Man erhält N-[2-(2'-(1H-Tetrazol-5-yl)-phenyl)-naphtalin-6-ylmethyl]-N-valeroyl-valin-methylester als gelben Schaum. R_{f}= 0,30 (CH₂Cl₂/Ethanol 95/5).

### Beispiel 23:

In analoger Weise, beispielsweise wie in einem der Beispiele 1-22 beschrieben, kann man herstellen:
N-{3-Brom-2-[2'-(1H-tetrazol-5-yl)-phenyl]-benzofuran-5-ylmethyl}-N-butyryl-1-aminomethyl-cyclopentan-1-carbonsäure;
N-{3-Brom-2-[2'-(1H-tetrazol-5-yl)-phenyl]-benzofuran-5-ylmethyl}-N-caproyl-1-aminomethyl-cyclopentan-1-carbonsäure;
N-{3-Brom-2-[2'-(1H-tetrazol-5-yl)-phenyl]-benzofuran-5-ylmethyl}-N-propionyl-1-aminomethyl-cyclopentan-1-carbonsäure;
N-{3-Brom-2-[2'-(1H-tetrazol-5-yl)-phenyl]-benzofuran-5-ylmethyl}-N-ethoxycarbonyl-1-aminomethyl-cyclopentan-1-carbonsäure;
N-{3-Brom-2-[2'-(1H-tetrazol-5-yl)-phenyl]-benzofuran-5-ylmethyl}-N-propyloxycarbonyl-1-aminomethyl-cyclopentan-1-carbonsäure;
N-{3-Brom-2-[2'-(1H-tetrazol-5-yl)-phenyl]-benzofuran-5-ylmethyl}-N-cyclopropyloxycarbonyl-1-aminomethyl-cyclopentan-1-carbonsäure;
N-{3-Brom-2-[2'-(1H-tetrazol-5-yl)-phenyl]-benzofuran-5-ylmethyl}-N-cyclopentyl-carbonyl-1-aminomethyl-cyclopentan-1-carbonsäure;
N-{3-Brom-2-[2'-(1H-tetrazol-5-yl)-phenyl]-benzofuran-5-ylmethyl}-N-cyclopropyl-carbonyl-1-aminomethyl-cyclopentan-1-carbonsäure;
N-{3-Brom-2-[2'-(1H-tetrazol-5-yl)-phenyl]-benzofuran-5-ylmethyl}-N-cyclopropyl-methylcarbonyl-1-aminomethyl-cyclopentan-1-carbonsäure;
N-{3-Brom-2-[2'-(1H-tetrazol-5-yl)-phenyl]-benzothiophen-5-ylmethyl}-N-butyryl-1-aminomethyl-cyclopentan-1-carbonsäure;
N-{3-Brom-2-[2'-(1H-tetrazol-5-yl)-phenyl]-benzothiophen-5-ylmethyl}-N-caproyl-1-aminomethyl-cyclopentan-1-carbonsäure;
N-{3-Brom-2-[2'-(1H-tetrazol-5-yl)-phenyl]-benzothiophen-5-ylmethyl}-N-propionyl-1-aminomethyl-cyclopentan-1-carbonsäure;
N-{3-Brom-2-[2'-(1H-tetrazol-5-yl)-phenyl]-benzothiophen-5-ylmethyl}-N-ethoxycarbonyl-1-aminomethyl-cyclopentan-1-carbonsäure;
N-{3-Brom-2-[2'-(1H-tetrazol-5-yl)-phenyl]-benzothiophen-5-ylmethyl}-N-propyloxycarbonyl-1-aminomethyl-cyclopentan-1-carbonsäure;
N-{3-Brom-2-[2'-(1H-tetrazol-5-yl)-phenyl]-benzothiophen-5-ylmethyl}-N-cyclopropyloxycarbonyl-1-aminomethyl-cyclopentan-1-carbonsäure;
N-{3-Brom-2-[2'-(1H-tetrazol-5-yl)-phenyl]-benzothiophen-5-ylmethyl}-N-cyclopentylcarbonyl-1-aminomethyl-cyclopentan-1-carbonsäure;
N-{3-Brom-2-[2'-(1H-tetrazol-5-yl)-phenyl]-benzothiophen-5-ylmethyl}-N-cyclopropylcarbonyl-1-aminomethyl-cyclopentan-1-carbonsäure;
N-{3-Brom-2-[2'-(1H-tetrazol-5-yl)-phenyl]-benzothiophen-5-ylmethyl}-N-cyclopropyl-methylcarbonyl-1-aminomethyl-cyclopentan-1-carbonsäure;
N-{3-Brom-2-[2'-(1H-tetrazol-5-yl)-phenyl]-indol-5-ylmethyl}-N-butyryl-1-aminomethyl-cyclopentan-1-carbonsäure;
N-{3-Brom-2-[2'-(1H-tetrazol-5-yl)-phenyl]-indol-5-ylmethyl}-N-caproyl-1-aminomethyl-cyclopentan-1-carbonsäure;
N-{3-Brom-2-[2'-(1H-tetrazol-5-yl)-phenyl]-indol-5-ylmethyl}-N-propionyl-1-aminomethyl-cyclopentan-1-carbonsäure;
N-{3-Brom-2-[2'-(1H-tetrazol-5-yl)-phenyl]-indol-5-ylmethyl}-N-ethoxycarbonyl-1-aminomethyl-cyclopentan-1-carbonsäure;
N-{3-Brom-2-[2'-(1H-tetrazol-5-yl)-phenyl]-indol-5-ylmethyl}-N-propyloxy-carbonyl-1-aminomethyl-cyclopentan-1-carbonsäure;
N-{3-Brom-2-[2'-(1H-tetrazol-5-yl)-phenyl]-indol-5-ylmethyl}-N-cyclopropyloxycarbonyl-1-aminomethyl-cyclopentan-1-carbonsäure;
N-{3-Brom-2-[2'-(1H-tetrazol-5-yl)-phenyl]-indol-5-ylmethyl}-N-cyclopentylcarbonyl-1-aminomethyl-cyclopentan-1-carbonsäure;
N-{3-Brom-2-[2'-(1H-tetrazol-5-yl)-phenyl]-indol-5-ylmethyl}-N-cyclopropylcarbonyl-1-aminomethyl-cyclopentan-1-carbonsäure;
N-{3-Brom-2-[2'-(1H-tetrazol-5-yl)-phenyl]-indol-5-ylmethyl}-N-cyclopropylmethylcarbonyl-1-aminomethyl-cyclopentan-1-carbonsäure;
N-{3-Brom-2-[2'-carboxy-phenyl]-benzofuran-5-ylmethyl}-N-butyryl-1-aminomethyl-cyclopentan-1-carbonsäure;
N-{3-Brom-2-[2'-carboxy-phenyl]-benzofuran-5-ylmethyl}-N-caproyl-1-aminomethyl-cyclopentan-1-carbonsäure;
N-{3-Brom-2-[2'-carboxy-phenyl]-benzofuran-5-ylmethyl}-N-propionyl-1-aminomethyl-cyclopentan-1-carbonsäure;
N-{3-Brom-2-[2'-carboxy-phenyl]-benzofuran-5-ylmethyl}-N-ethoxycarbonyl-1-aminomethyl-cyclopentan-1-carbonsäure;
N-{3-Brom-2-[2'-carboxy-phenyl]-benzofuran-5-ylmethyl}-N-propyloxycarbonyl-1-aminomethyl-cyclopentan-1-carbonsäure;
N-{3-Brom-2-[2'-carboxy-phenyl]-benzofuran-5-ylmethyl}-N-cyclopropyloxycarbonyl-1-aminomethyl-cyclopentan-1-carbonsäure;
N{3-Brom-2-[2'-carboxy-phenyl]-benzofuran-5-ylmethyl}-N-cyclopentylcarbonyl-1-aminomethyl-cyclopentan-1-carbonsäure;
N-{3-Brom-2-[2'-carboxy-phenyl]-benzofuran-5-ylmethyl}-N-cyclopropylcarbonyl-1-aminomethyl-cyclopentan-1-carbonsäure;
N-{3-Brom-2-[2'-carboxy-phenyl]-benzofuran-5-ylmethyl}-N-cyclopropylmethylcarbonyl-1-aminomethyl-cyclopentan-1-carbonsäure;
N-{3-Brom-2-[2'-carboxy-phenyl]-benzothiophen-5-ylmethyl}-N-butyryl-1-aminomethyl-cyclopentan-1-carbonsäure;
N-{3-Brom-2-[2'-carboxy-phenyl]-benzothiophen-5-ylmethyl}-N-caproyl-1-aminomethyl-cyclopentan-1-carbonsäure;
N-{3-Brom-2-[2'-carboxy-phenyl]-benzothiophen-5-ylmethyl}-N-propionyl-1-aminomethyl-cyclopentan-1-carbonsäure;
N-{3-Brom-2-[2'-carboxy-phenyl]-benzothiophen-5-ylmethyl}-N-ethoxycarbonyl-1-aminomethyl-cyclopentan-1-carbonsäure;
N-{3-Brom-2-[2'-carboxy-phenyl]-benzothiophen-5-ylmethyl}-N-propyloxycarbonyl-1-aminomethyl-cyclopentan-1-carbonsäure;
N-{3-Brom-2-[2'-carboxy-phenyl]-benzothiophen-5-ylmethyl}-N-cyclopropyloxycarbonyl-1-aminomethyl-cyclopentan-1-carbonsäure;
N-{3-Brom-2-[2'-carboxy-phenyl]-5-ylmethyl-benzothiophen}-N-cyclopentylcarbonyl-1-aminomethyl-cyclopentan-1-carbonsäure ;
N-{3-Brom-2-[2'-carboxy-phenyl]-5-ylmethyl-benzothiophen}-N-cyclopropylcarbonyl-1-aminomethyl-cyclopentan-1-carbonsäure;
N-{3-Brom-2-[2'-carboxy-phenyl]-5-ylmethyl-benzothiophen}-N-cyclopropylmethylcarbonyl-1-aminomethyl-cyclopentan-1-carbonsäure;
N-{3-Brom-2-[2'-carboxy-phenyl]-5-ylmethyl-indol}-N-butyryl-1-aminomethyl-cyclopentan-1-carbonsäure;
N-{3-Brom-2-[2'-carboxy-phenyl]-5-ylmethyl-indol}-N-caproyl-1-aminomethyl-cyclopentan-1-carbonsäure;
N-{3-Brom-2-[2'-carboxy-phenyl]-5-ylmethyl-indol}-N-propionyl-1-aminomethyl-cyclopentan-1-carbonsäure;
N-{3-Brom-2-[2'carboxy-phenyl]-5-ylmethyl-indol}-N-ethoxycarbonyl-1-aminomethyl-cyclopentan-1-carbonsäure;
N-{3-Brom-2-[2'-carboxy-phenyl]-5-ylmethyl-indol}-N-propyloxycarbonyl-1-aminomethyl-cyclopentan-1-carbonsäure;
N-{3-Brom-2-[2'-carboxy-phenyl]-5-ylmethyl-indol}-N-cyclopropyloxycarbonyl-1-aminomethyl-cyclopentan-1-carbonsäure;
N-{3-Brom-2-[2'-carboxy-phenyl]-5-ylmethyl-indol}-N-cyclopentylcarbonyl-1-aminomethyl-cyclopentan-1-carbonsäure;
N-{3-Brom-2-[2'-carboxy-phenyl]-5-ylmethyl-indol}-N-cyclopropylcarbonyl-1-aminomethyl-cyclopentan-1-carbonsäure;
N-{3-Brom-2-[2'-carboxy-phenyl]-5-ylmethyl-indol}-N-cyclopropylmethylcarbonyl-1-aminomethyl-cyclopentan-1-carbonsäure;
N-{3-Brom-2-[2'-(1H-tetrazol-5-yl)-phenyl]-5-ylmethyl-benzofuran}-N-butyryl-valin;
N-{3-Brom-2 [2'-(1H-tetrazol-5-yl)-phenyl]-5-ylmethyl-benzofuran}-N-caproyl-valin;
N-{3-Brom-2-[2'-(1H-tetrazol-5-yl)-phenyl]-5-ylmethyl-benzofuran}-N-propionyl-valin;
N-{3-Brom-2-[2'-(1H-tetrazol-5-yl)-phenyl]-5-ylmethyl-benzofuran}-N-ethoxycarbonyl-valin;
N-{3-Brom-2-[2'-(1H-tetrazol-5-yl)-phenyl]-5-ylmethyl-benzofuran}-N-propyloxycarbonyl-valin;
N-{3-Brom-2-[2'-(1H-tetrazol-5-yl)-phenyl]-5-ylmethyl-benzofuran}-N-cyclopropyloxycarbonyl-vain;
N-{3-Brom-2-[2'-(1H-tetrazol-5-yl)-phenyl]-5-ylmethyl-benzofuran}-N-cyclopentylcarbonyl-valin;
N-{3-Brom-2-[2'-(1H-tetrazol-5-yl)-phenyl]-5-ylmethyl-benzofuran}-N-cyclopropylcarbonyl-valin;
N-{3-Brom-2-[2'-(1H-tetrazol-5-yl)-phenyl]-5-ylmethyl-benzofuran}-N-cyclopropylmethylcarbonyl-valin;
N-{3-Brom-2-[2'-(1H-tetrazol-5-yl)-phenyl]-5-ylmethyl-benzothiophen}-N-butyryl-valin;
N-{3-Brom-2-[2'-(1H-tetrazol-5-yl)-phenyl]-5-ylmethyl-benzothiophen}-N-caproyl-valin;
N-{3-Brom-2-[2'-(1H-tetrazol-5-yl)-phenyl]-5-ylmethyl-benzothiophen}-N-propionyl-valin;
N-{3-Brom-2-[2'-(1H-tetrazol-5-yl)-phenyl]-5-ylmethyl-benzothiophen}-N-ethoxycarbonyl-valin;
N-{3-Brom-2-[2'-(1H-tetrazol-5-yl)-phenyl]-5-ylmethyl-benzothiophen}-N-propyloxycarbonyl-valin;
N-{3-Brom-2-[2'-(1H-tetrazol-5-yl)-phenyl]-5-ylmethyl-benzothiophen}-N-cyclopropyloxycarbonyl-valin;
N-{3-Brom-2-[2'-(1H-tetrazol-5-yl)-phenyl]-5-ylmethyl-benzothiophen}-N-cyclopentylcarbonyl-valin;
N-{3-Brom-2-[2'-(1H-tetrazol-5-yl)-phenyl]-5-ylmethyl-benzothiophen}-N-cyclopropylcarbonyl-valin;
N-{3-Brom-2-[2'-(1H-tetrazol-5-yl)-phenyl]-5-ylmethyl-benzothiophen}-N-cyclopropylmethylcarbonyl-valin;
N-{3-Brom-2-[2'-(1H-tetrazol-5-yl)-phenyl]-5-ylmethyl-indol}-N-butyryl-valin;
N-{3-Brom-2-[2'-(1H-tetrazol-5-yl)-phenyl]-5-ylmethyl-indol}-N-caproyl-valin;
N-{3-Brom-2-[2'-(1H-tetrazol-5-yl)-phenyl]-5-ylmethyl-indol}-N-propionyl-valin;
N-{3-Brom-2-[2'-(1H-tetrazol-5-yl)-phenyl]-5-ylmethyl-indol}-N-ethoxycarbonyl-valin;
N-{3-Brom-2-[2'-(1H-tetrazol-5-yl)-phenyl]-5-ylmethyl-indol}-N-propyloxycarbonyl-valin;
N-{3-Brom-2-[2'-(1H-tetrazol-5-yl)-phenyl]-5-ylmethyl-indol}-N-cyclopropyloxycarbonyl-valin;
N-{3-Brom-2-[2'-(1H-tetrazol-5-yl)-phenyl]-5-ylmethyl-indol}-N-cyclopentylcarbonyl-valin;
N-{3-Brom-2-[2'-(1H-tetrazol-5-yl)-phenyl]-5-ylmethyl-indol}-N-cyclopropylcarbonyl-valin;
N-{3-Brom-2-[2'-(1H-tetrazol-5-yl)-phenyl]-5-ylmethyl-indol}-N-cyclopropylmethylcarbonyl-valin;
N-{3-Brom-2-[2'-(1H-tetrazol-5-yl)-phenyl]-5-ylmethyl-benzofuran}-N-butyryl-alanin;
N-{3-Brom-2-[2'-(1H-tetrazol-5-yl)-phenyl]-5-ylmethyl-benzofuran}-N-caproyl-alanin;
N-{3-Brom-2-[2'-(1H-tetrazol-5-yl)-phenyl]-5-ylmethyl-benzofuran}-N-propionyl-alanin;
N-{3-Brom-2-[2'-(1H-tetrazol-5-yl)-phenyl]-5-ylmethyl-benzofuran}-N-ethoxycarbonyl-alanin;
N-{3-Brom-2-[2'-(1H-tetrazol-5-yl)-phenyl]-5-ylmethyl-benzofuran}-N-propyloxycarbonyl-alanin;
N-{3-Brom-2-[2'-(1H-tetrazol-5-yl)-phenyl]-5-ylmethyl-benzofuran}-N-cyclopropyloxycarbonyl-vain;
N-{3-Brom-2-[2'-(1H-tetrazol-5-yl)-phenyl]-5-ylmethyl-benzofuran}-N-cyclopentylcarbonyl-alanin;
N-{3-Brom-2-[2'-(1H-tetrazol-5-yl)-phenyl]-5-ylmethyl-benzofuran}-N-cyclopropylcarbonyl-alanin;
N-{3-Brom-2-[2'-(1H-tetrazol-5-yl)-phenyl]-5-ylmethyl-benzofuran}-N-cyclopropylmethylcarbonyl-alanin;
N-{3-Brom-2-[2'-(1H-tetrazol-5-yl)-phenyl]-5-ylmethyl-benzothiophen}-N-butyryl-alanin;
N-{3-Brom-2-[2'-(1H-tetrazol-5-yl)-phenyl]-5-ylmethyl-benzothiophen}-N-caproyl-alanin;
N-{3-Brom-2-[2'-(1H-tetrazol-5-yl)-phenyl]-5-ylmethyl-benzothiophen}-N-propionyl-alanin;
N-{3-Brom-2-[2'-(1H-tetrazol-5-yl)-phenyl]-5-ylmethyl-benzothiophen}-N-ethoxycarbonyl-alanin;
N-{3-Brom-2-[2'-(1H-tetrazol-5-yl)-phenyl]-5-ylmethyl-benzothiophen}-N-propyloxycarbonyl-alanin;
N-{3-Brom-2-[2'-(1H-tetrazol-5-yl)-phenyl]-5-ylmethyl-benzothiophen}-N-cyclopropyloxycarbonyl-alanin;
N-{3-Brom-2-[2'-(1H-tetrazol-5-yl)-phenyl]-5-ylmethyl-benzothiophen}-N-cyclopentylcarbonyl-alanin;
N-{3-Brom-2-[2'-(1H-tetrazol-5-yl)-phenyl]-5-ylmethyl-benzothiophen}-N-cyclopropylcarbonyl-alanin;
N-{3-Brom-2-[2'-(1H-tetrazol-5-yl)-phenyl]-5-ylmethyl-benzothiophen}-N-cyclopropylmethylcarbonyl-alanin;
N-{3-Brom-2-[2'-(1H-tetrazol-5-yl)-phenyl]-5-ylmethyl-indol}-N-butyryl-alanin;
N-{3-Brom-2-[2'-(1H-tetrazol-5-yl)-phenyl]-5-ylmethyl-indol}-N-caproyl-alanin;
N-{3-Brom-2-[2'-(1H-tetrazol-5-yl)-phenyl]-5-ylmethyl-indol}-N-propionyl-alanin;
N-{3-Brom-2-[2'-(1H-tetrazol-5-yl)-phenyl]-5-ylmethyl-indol}-N-ethoxycarbonyl-alanin;
N-{3-Brom-2-[2'-(1H-tetrazol-5-yl)-phenyl]-5-ylmethyl-indol}-N-propyloxycarbonyl-alanin;
N-{3-Brom-2-[2'-(1H-tetrazol-5-yl)-phenyl]-5-ylmethyl-indol}-N-cyclopropyloxycarbonyl-alanin;
N-{3-Brom-2-[2'-(1H-tetrazol-5-yl)-phenyl]-5-ylmethyl-indol}-N-cyclopentylcarbonyl-alanin;
N-{3-Brom-2-[2'-(1H-tetrazol-5-yl)-phenyl]-5-ylmethyl-indol}-N-cyclopropylcarbonyl-alanin;
N-{3-Brom-2-[2'-(1H-tetrazol-5-yl)-phenyl]-5-ylmethyl-indol}-N-cyclopropylmethylcarbonyl-alanin;
N-[2-(2'-(Tetrazol-5-yl)-phenyl)-chinolin-6-yl-methyl]-N-butyryl-1-aminomethyl-cyclohexan-1-carbonsäure; R_{f}= 0,35 (CH₂Cl₂/CH₃OH=4:1);
N-[2-(2'-(Tetrazol-5-yl)-phenyl)-chinolin-6-yl-methyl]-N-valeroyl-1-aminomethyl-cyclohexan-1-carbonsäure;R_{f}= 0,26 (CH₂Cl₂/CH₃OH=4:1);
N-[2-(2'-(Tetrazol-5-yl)-phenyl)-chinolin-6-yl-methyl]-N-butyryl-1-aminomethyl-cyclopentan-1-carbonsäure;
N-[2-(2'-(Tetrazol-5-yl)-phenyl)-chinolin-6-yl-methyl]-N-caproyl-1-aminomethyl-cyclopentan-1-carbonsäure;
N-[2-(2'-(Tetrazol-5-yl)-phenyl)-chinolin-6-yl-methyl]-N-propionyl-1-aminomethylcyclopentan-1-carbonsäure;
N-[2-(2'-(Tetrazol-5-yl)-phenyl)-chinolin-6-yl-methyl]-N-ethoxy-carbonyl-1-aminomethyl-cyclopentan-1-carbonsäure;
N-[2-(2'-(Tetrazol-5-yl)-phenyl)-chinolin-6-yl-methyl]-N-propyloxy-carbonyl-1-aminomethyl-cyclopentan-1-carbonsäure;
N-[2-(2'-(Tetrazol-5-yl)-phenyl)-chinolin-6-yl-methyl]-N-cyclopropyloxy-carbonyl1-aminomethyl-cyclopentan-1-carbonsäure;
N-[2-(2'-(Tetrazol-5-yl)-phenyl)-chinolin-6-yl-methyl]-N-cyclopropyl-carbonyl-1-aminomethyl-cyclopentan-1-carbonsäure;
N-[2-(2'-(Tetrazol-5-yl)-phenyl)-chinolin-6-yl-methyl]-N-cyclopentyl-carbonyl-1-aminomethyl-cyclopentan-1-carbonsäure;
N-[2-(2'-(Tetrazol-5-yl)-phenyl)-chinolin-6-yl-methyl]-N-cyclopropylmethyl-carbonyl-1-aminomethyl-cyclopentan-1-carbonsäure;
N-[2-(2'-Carboxy-phenyl)-chinolin-6-yl-methyl]-N-butyryl-1-aminomethyl-cyclopentan-1-carbonsäure;
N-[2-(2'-Carboxy-phenyl)-chinolin-6-yl-methyl]-N-caproyl-1-aminomethyl-cyclopentan-1-carbonsäure;
N-[2-(2'-Carboxy-phenyl)-chinolin-6-yl-methyl]-N-propionyl-1-aminomethyl-cyclopentan-1-carbonsäure;
N-[2-(2'-Carboxy-phenyl)-chinolin-6-yl-methyl]-N-ethoxy-carbonyl-1-aminomethylcyclopentan-1-carbonsäure;
N-[2-(2'-Carboxy-phenyl)-chinolin-6-yl-methyl]-N-propyloxy-carbonyl-1-aminomethylcyclopentan-1-carbonsäure;
N-[2-(2'-Carboxy-phenyl)-chinolin-6-yl-methyl]-N-cyclopropyloxy-carbonyl-1-aminomethyl-cyclopentan-1-carbonsäure;
N-[2-(2'-Carboxy-phenyl)-chinolin-6-yl-methyl]-N-cyclopropyl-carbonyl-1-aminomethyl-cyclopentan-1-carbonsäure;
N-[2-(2'-Carboxy-phenyl)-chinolin-6-yl-methyl]-N-cyclopentyl-carbonyl-1-aminomethyl-cyclopentan-1-carbonsäure;
N-[2-(2'-Carboxy-phenyl)-chinolin-6-yl-methyl]-N-cyclopropylmethyl-carbonyl-1-aminomethyl-cyclopentan-1-carbonsäure;
N-[2-(2'-(Tetrazol-5-yl)-phenyl)-naphthalin-6-yl-methyl]-N-butyryl-1-aminomethylcyclopentan-1-carbonsäure;
N-[2-(2'-(Tetrazol-5-yl)-phenyl)-naphthalin-6-yl-methyl]-N-caproyl-1-aminomethylcyclopentan-1-carbonsäure;
N-[2-(2'-(Tetrazol-5-yl)-phenyl)-naphthalin-6-yl-methyl]-N-propionyl-1-aminomethylcyclopentan-1-carbonsäure;
N-[2-(2'-(Tetrazol-5-yl)-phenyl)-naphthalin-6-yl-methyl]-N-ethoxy-carbonyl-1-aminomethyl-cyclopentan-1-carbonsäure;
N-[2-(2'-(Tetrazol-5-yl)-phenyl)-naphthalin-6-yl-methyl]-N-propyloxy-carbonyl-1-aminomethyl-cyclopentan-1-carbonsäure;
N-[2-(2'-(Tetrazol-5-yl)-phenyl)-naphthalin-6-yl-methyl]-N-cyclopropyloxy-carbonyl-1-aminomethyl-cyclopentan-1-carbonsäure;
N-[2-(2'-(Tetrazol-5-yl)-phenyl)-naphthalin-6-yl-methyl]-N-cyclopropyl-carbonyl-1-aminomethyl-cyclopentan-1-carbonsäure;
N-[2-(2'-(Tetrazol-5-yl)-phenyl)-naphthalin-6-yl-methyl]-N-cyclopentyl-carbonyl-1-aminomethyl-cyclopentan-1-carbonsäure;
N-[2-(2'-(Tetrazol-5-yl)-phenyl)-naphthalin-6-yl-methyl]-N-cyclopropylmethylcarbonyl-1-aminomethyl-cyclopentan-1-carbonsäure;
N-[2-(2'-Carboxy-phenyl)-naphthalin-6-yl-methyl]-N-butyryl-1-aminomethyl-cyclopentan-1-carbonsäure;
N-[2-(2'-Carboxy-phenyl)-naphthalin-6-yl-methyl]-N-caproyl-1-aminomethyl-cyclopentan-1-carbonsäure;
N-[2-(2'-Carboxy-phenyl)-naphthalin-6-yl-methyl]-N-propionyl-1-aminomethyl-cyclopentan-1-carbonsäure;
N-[2-(2'-Carboxy-phenyl)-naphthalin-6-yl-methyl]-N-ethoxy-carbonyl-1-aminomethylcyclopentan-1-carbonsäure;
N-[2-(2'-Carboxy-phenyl)-naphthalin-6-yl-methyl]-N-propyloxy-carbonyl-1-aminomethyl-cyclopentan-1-carbonsäure;
N-[2-(2'-Carboxy-phenyl)-naphthalin-6-yl-methyl]-N-cyclopropyloxy-carbonyl-1-aminomethyl-cyclopentan-1-carbonsäure;
N-[2-(2'-Carboxy-phenyl)-naphthalin-6-yl-methyl]-N-cyclopropyl-carbonyl-1-aminomethyl-cyclopentan-1-carbonsäure;
N-[2-(2'-Carboxy-phenyl)-naphthalin-6-yl-methyl]-N-cyclopentyl-carbonyl-1-aminomethyl-cyclopentan-1-carbonsäure;
N-[2-(2'-Carboxy-phenyl)-naphthalin-6-yl-methyl]-N-cyclopropylmethyl-carbonyl-1-aminomethyl-cyclopentan-1-carbonsäure;
N-[2-(2'-(Tetrazol-5-yl)-phenyl)-chinolin-6-yl-methyl]-N-butyryl-valin;
N-[2-(2'-(Tetrazol-5-yl)-phenyl)-chinolin-6-yl-methyl]-N-caproyl-valin;
N-[2-(2'-(Tetrazol-5-yl)-phenyl)-chinolin-6-yl-methyl]-N-propionyl-valin;
N-[2-(2'-(Tetrazol-5-yl)-phenyl)-chinolin-6-yl-methyl]-N-ethoxy-carbonyl-valin;
N-[2-(2'-(Tetrazol-5-yl)-phenyl)-chinolin-6-yl-methyl]-N-propyloxy-carbonyl-valin;
N-[2-(2'-(Tetrazol-5-yl)-phenyl)-chinolin-6-yl-methyl]-N-cyclopropyloxy-carbonyl-valin;
N-[2-(2'-(Tetrazol-5-yl)-phenyl)-chinolin-6-yl-methyl]-N-cyclopropyl-carbonyl-valin;
N-[2-(2'-(Tetrazol-5-yl)-phenyl)-chinolin-6-yl-methyl]-N-cyclopentyl-carbonyl-valin;
N-[2-(2'-(Tetrazol-5-yl)-phenyl)-chinolin-6-yl-methyl]-N-cyclopropylmethyl-carbonyl-valin;
N-[2-(2'-Carboxy-phenyl)-chinolin-6-yl-methyl]-N-butyryl-valin;
N-[2-(2'-Carboxy-phenyl)-chinolin-6-yl-methyl]-N-caproyl-valin;
N-[2-(2'-Carboxy-phenyl)-chinolin-6-yl-methyl]-N-propionyl-valin;
N-[2-(2'-Carboxy-phenyl)-chinolin-6-yl-methyl]-N-ethoxy-carbonyl-valin;
N-[2-(2'-Carboxy-phenyl)-chinolin-6-yl-methyl]-N-propyloxy-carbonyl-valin;
N-[2-(2'-Carboxy-phenyl)-chinolin-6-yl-methyl]-N-cyclopropyloxy-carbonyl-valin;
N-[2-(2'-Carboxy-phenyl)-chinolin-6-yl-methyl]-N-cyclopropyl-carbonyl-valin;
N-[2-(2'-Carboxy-phenyl)-chinolin-6-yl-methyl]-N-cyclopentyl-carbonyl-valin;
N-[2-(2'-Carboxy-phenyl)-chinolin-6-yl-methyl]-N-cyclopropylmethyl-carbonyl-valin;
N-[2-(2'-(Tetrazol-5-yl)-phenyl)-naphthalin-6-yl-methyl]-N-butyryl-valin;
N-[2-(2'-(Tetrazol-5-yl)-phenyl)-naphthalin-6-yl-methyl]-N-caproyl-valin;
N-[2-(2'-(Tetrazol-5-yl)-phenyl)-naphthalin-6-yl-methyl]-N-propionyl-valin;
N-[2-(2'-(Tetrazol-5-yl)-phenyl)-naphthalin-6-yl-methyl]-N-ethoxy-carbonyl-valin;
N-[2-(2'-(Tetrazol-5-yl)-phenyl)-naphthalin-6-yl-methyl]-N-propyloxy-carbonyl-valin;
N-[2-(2'-(Tetrazol-5-yl)-phenyl)-naphthalin-6-yl-methyl]-N-cyclopropyloxy-carbonyl-valin;
N-[2-(2'-(Tetrazol-5-yl)-phenyl)-naphthalin-6-yl-methyl]-N-cyclopropyl-carbonyl-valin;
N-[2-(2'-(Tetrazol-5-yl)-phenyl)-naphthalin-6-yl-methyl]-N-cyclopentyl-carbonyl-valin;
N-[2-(2'-(Tetrazol-5-yl)-phenyl)-naphthalin-6-yl-methyl]-N-cyclopropylmethyl-carbonyl-valin;
N-[2-(2'-Carboxy-phenyl)-naphthalin-6-yl-methyl]-N-butyryl-valin;
N-[2-(2'-Carboxy-phenyl)-naphthalin-6-yl-methyl]-N-caproyl-valin;
N-[2-(2'-Carboxy-phenyl)-naphthalin-6-yl-methyl]-N-propionyl-valin;
N-[2-(2'-Carboxy-phenyl)-naphthalin-6-yl-methyl]-N-ethoxy-carbonyl-valin;
N-[2-(2'-Carboxy-phenyl)-naphthalin-6-yl-methyl]-N-propyloxy-carbonyl-valin;
N-[2-(2'-Carboxy-phenyl)-naphthalin-6-yl-methyl]-N-cyclopropyloxy-carbonyl-valin;
N-[2-(2'-Carboxy-phenyl)-naphthalin-6-yl-methyl]-N-cyclopropyl-carbonyl-valin;
N-[2-(2'-Carboxy-phenyl)-naphthalin-6-yl-methyl]-N-cyclopentyl-carbonyl-valin;
N-[2-(2'-Carboxy-phenyl)-naphthalin-6-yl-methyl]-N-cyclopropylmethyl-carbonyl-valin;
N-[2-(2'-(Tetrazol-5-yl)-phenyl)-chinolin-6-yl-methyl]-N-butyryl-alanin;
N-[2-(2'-(Tetrazol-5-yl)-phenyl)-chinolin-6-yl-methyl]-N-caproyl-alanin;
N-[2-(2'-(Tetrazol-5-yl)-phenyl)-chinolin-6-yl-methyl]-N-propionyl-alanin;
N-[2-(2'-(Tetrazol-5-yl)-phenyl)-chinolin-6-yl-methyl]-N-ethoxy-carbonyl-alanin;
N-[2-(2'-(Tetrazol-5-yl)-phenyl)-chinolin-6-yl-methyl]-N-propyloxy-carbonyl-alanin;
N-[2-(2'-(Tetrazol-5-yl)-phenyl)-chinolin-6-yl-methyl]-N-cyclopropyloxy-carbonyl-alanin;
N-[2-(2'-(Tetrazol-5-yl)-phenyl)-chinolin-6-yl-methyl]-N-cyclopropyl-carbonyl-alanin;
N-[2-(2'-(Tetrazol-5-yl)-phenyl)-chinolin-6-yl-methyl]-N-cyclopentyl-carbonyl-alanin;
N-[2-(2'-(Tetrazol-5-yl)-phenyl)-chinolin-6-yl-methyl]-N-cyclopropylmethyl1-carbonyl-alanin;
N-[2-(2'-Carboxy-phenyl)-chinolin-6-yl-methyl]-N-butyryl-alanin;
N-[2-(2'-Carboxy-phenyl)-chinolin-6-yl-methyl]-N-caproyl-alanin;
N-[2-(2'-Carboxy-phenyl)-chinolin-6-yl-methyl]-N-propionyl-alanin;
N-[2-(2'-Carboxy-phenyl)-chinolin-6-yl-methyl]-N-ethoxy-carbonyl-alanin;
N-[2-(2'-Carboxy-phenyl)-chinolin-6-yl-methyl]-N-propyloxy-carbonyl-alanin;
N-[2-(2'-Carboxy-phenyl)-chinolin-6-yl-methyl]-N-cyclopropyloxy-carbonyl-alanin;
N-[2-(2'-Carboxy-phenyl)-chinolin-6-yl-methyl]-N-cyclopropyl-carbonyl-alanin;
N-[2-(2'-Carboxy-phenyl)-chinolin-6-yl-methyl]-N-cyclopentyl-carbonyl-alanin;
N-[2-(2'-Carboxy-phenyl)-chinolin-6-yl-methyl]-N-cyclopropylmethyl-carbonyl-alanin;
N-[2-(2'-(Tetrazol-5-yl)-phenyl)-naphthalin-6-yl-methyl]-N-butyryl-alanin; R_{f} = 0,075(CH₂Cl₂/CH₃OH=4:1);
N-[2-(2'-(Tetrazol-5-yl)-phenyl)-naphthalin-6-yl-methyl]-N-caproyl-alanin;
N-[2-(2'-(Tetrazol-5-yl)-phenyl)-naphthalin-6-yl-methyl]-N-propionyl-alanin;
N-[2-(2'-(Tetrazol-5-yl)-phenyl)-naphthalin-6-yl-methyl]-N-ethoxy-carbonyl-alanin;
N-[2-(2'-(Tetrazol-5-yl)-phenyl)-naphthalin-6-yl-methyl]-N-propyloxy-carbonyl-alanin;
N-[2-(2'-(Tetrazol-5-yl)-phenyl)-naphthalin-6-yl-methyl]-N-cyclopropyloxy-carbonyl-alanin;
N-[2-(2'-(Tetrazol-5-yl)-phenyl)-naphthalin-6-yl-methyl]-N-cyclopropyl-carbonyl-alanin;
N-[2-(2'-(Tetrazol-5-yl)-phenyl)-naphthalin-6-yl-methyl]-N-cyclopentyl-carbonyl-alanin;
N-[2-(2'-(Tetrazol-5-yl)-phenyl)-naphthalin-6-yl-methyl]-N-cyclopropylmethyl-carbonyl-alanin;
N-[2-(2'-Carboxy-phenyl)-naphthalin-6-yl-methyl]-N-butyryl-alanin;
N-[2-(2'-Carboxy-phenyl)-naphthalin-6-yl-methyl]-N-caproyl-alanin;
N-[2-(2'-Carboxy-phenyl)-naphthalin-6-yl-methyl]-N-propionyl-alanin;
N-[2-(2'-Carboxy-phenyl)-naphthalin-6-yl-methyl]-N-ethoxy-carbonyl-alanin;
N-[2-(2'-Carboxy-phenyl)-naphthalin-6-yl-methyl]-N-propyloxy-carbonyl-alanin;
N-[2-(2'-Carboxy-phenyl)-naphthalin-6-yl-methyl]-N-cyclopropyloxy-carbonyl-alanin;
N-[2-(2'-Carboxy-phenyl)-naphthalin-6-yl-methyl]-N-cyclopropyl-carbonyl-alanin;
N-[2-(2'-Carboxy-phenyl)-naphthalin-6-yl-methyl]-N-cyclopentyl-carbonyl-alanin;
N-[2-(2'-Carboxy-phenyl)-naphthalin-6-yl-methyl]-N-cyclopropylmethyl-carbonyl-alanin.

### Beispiel 24:

Tabletten, enthaltend je 50 mg Wirkstoff, z.B. N-[2-(2-(1H-Tetrazol-5-yl)-phenyl)-chinolin-6-yl-methyl]-N-valeroyl-1-aminomethyl-cyclopentan-1-carbonsäure, können wie folgt hergestellt werden:

| Zusammensetzung (für 10000 Tabletten): | |
|---|---|
| Wirkstoff | 500,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

### Beispiel 25:

Lacktabletten, enthaltend je 100 mg Wirkstoff, z.B. N-[2-(2-(1H-Tetrazol-5-yl)-phenyl)-chinolin-6-yl-methyl]-N-valeroyl-1-aminomethyl-cyclopentan-1-carbonsäure, können wie folgt hergestellt werden:

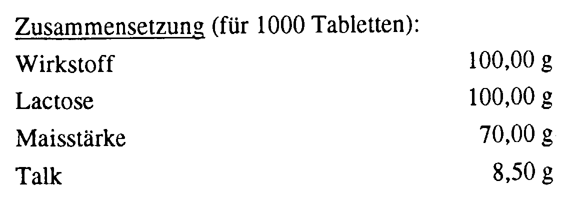

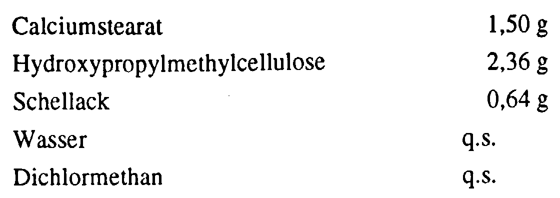

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 280 mg) verpresst und diese mit einer Lösung der Hydroxypropylmethylcellulose und des Schellacks in Dichlormethan lackiert (Endgewicht der Lacktablette: 283 mg).

### Beispiel 26:

In analoger Weise wie in den Beispielen 24 und 25 beschrieben können auch Tabletten und Lacktabletten, enthaltend eine andere Verbindung der Formel I oder ein pharmazeutisch verwendbares Salz einer Verbindung der Formel I, z.B. gemäss einem der Beispiele 1 bis 23, hergestellt werden.

## Patentansprüche (Patentansprüche für folgende(n) Vertragsstaat(en): AT, BE, CH, DE, DK, FR, GB, IE, IT, LI, LU, NL, PT, SE)

1. Eine Verbindung der Formel 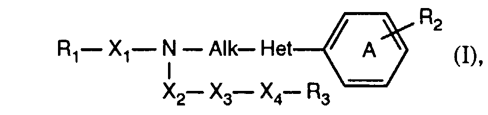 worin
R₁ C₁-C₇-Alkyl bedeutet, welches unsubstituiert oder durch Halogen oder Hydroxy substituiert ist, oder C₃-C₇-Alkenyl, C₂-C₇-Cycloalkyl, C₃-C₇-Cycloalkyloxy,
C₁-C₇-Alkoxy oder C₃-C₇-Cycloalkyl-C₁-C₇-alkoxy bedeutet;
R₂ 1H-Tetrazol-5-yl, Carboxy, C₁-C₇-Alkoxy-carbonyl, SO₃H, PO₂H₂, PO₃H₂ oder Halogen-C₁-C₇-alkansulfonylamino ist;
R₃ 1H-Tetrazol-5-yl, Hydroxymethyl, C₁-C₇-Alkoxy-methyl, Formyl, Carboxy, C₁-C₇-Alkoxy-carbonyl, C₁-C₇-Alkoxy-C₁-C₇-alkoxy-carbonyl, Phenyl-C₁-C₄-alkoxy-carbonyl oder Carbamoyl bedeutet, dessen Aminogruppe unsubstituiert oder durch C₁-C₇-Alkyl, C₃-C₇-Alkenyl oder Phenyl-C₁-C₇-alkyl mono- oder unabhängig voneinander disubstituiert oder durch C₂-C₇-Alkylen oder C₂-C₄-Alkylenoxy-C₂-C₄-alkylen disubstituiert ist;
Alk Methylen, Ethylen oder Ethyliden ist;
Het 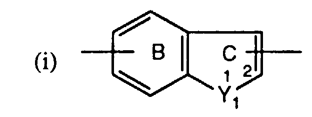 bedeutet, worin Y₁ für O, S oder N(R) steht und R Wasserstoff oder C₁-C₇-Alkyl bedeutet;
oder 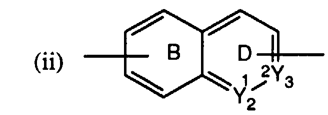 bedeutet, worin eine der Variablen Y₂ und Y₃ für C(R') und die andere für N steht oder beide Variablen jeweils für C(R') stehen; und R' Wasserstoff, Halogen, C₁-C₇-Alkyl, C₁-C₇-Alkoxy, C₂-C₇-Alkenyloxy, Phenoxy, Benzyloxy, Trifluormethyl oder S(O)ₘ-R bedeutet, worin m 0, 1 oder 2 ist; und R Wasserstoff oder C₁-C₇-Alkyl ist;
X₁ für -CO- oder -S(O)ₘ- und der Index m für 0, 1 oder 2 steht;
eine der Variablen X₂ und X₄ für C₁-C₄-Alkylen und die andere der Variablen X₂ und X₄ für eine Bindung steht; oder beide Variablen X₂ und X₄ jeweils für eine Bindung stehen; X₃ C₃-C₇-Cycloalkyliden oder das Strukturelement -C(Xₐ)(X_{b})- bedeutet und Xₐ Wasserstoff oder C₁-C₇-Alkyl ist und X_{b} C₁-C₇-Alkyl ist;
und die Ringe A, B, C und D, bis auf die in der Formel angegebenen Substituenten, sowie aromatische Substituenten unabhängig voneinander unsubstituiert oder ein- oder mehrfach substituiert sind durch Substituenten ausgewählt aus der Gruppe bestehend aus Halogen, C₁-C₇-Alkyl, C₁-C₇-Alkoxy, C₂-C₇-Alkenyloxy, Phenoxy, Benzyloxy, Trifluormethyl und S(O)ₘ-R, worin m 0, 1 oder 2 ist und R Wasserstoff oder C₁-C₇-Alkyl ist;
und ihre Salze.

2. Verbindung gemäss Anspruch 1 der Formel I, worin
R₁ C₁-C₇-Alkyl bedeutet, welches unsubstituiert oder durch Halogen oder Hydroxy substituiert ist, oder C₂-C₇-Alkenyl, C₃-C₇-Cycloalkyl oder C₁-C₇-Alkoxy bedeutet; und ihre Salze.

3. Verbindung gemäss Anspruch 1 der Formel I, worin
R₁ C₂-C₇-Alkyl ist oder C₁-C₇-Alkyl bedeutet, welches durch Halogen oder Hydroxy substituiert ist, oder C₃-C₇-Alkenyl, C₃-C₆-Cycloalkyl oder C₁-C₇-Alkoxy bedeutet;
R₂ 1H-Tetrazol-5-yl, Carboxy, C₁-C₄-Alkoxy-carbonyl oder Halogen-C₁-C₄-alkansulfonylamino ist;
R₃ 1H-Tetrazol-5-yl, Carboxy, C₁-C₄-Alkoxy-carbonyl, C₁-C₄-Alkoxy-C₁-C₄-alkoxy-carbonyl, Phenyl-C₁-C₄-alkoxycarbonyl oder Carbamoyl bedeutet, dessen Aminogruppe durch C₁-C₄-Alkyl mono- oder unabhängig voneinander disubstituiert oder durch C₄-C₆-Alkylen oder Ethylenoxyethylen disubstituiert ist;
Alk Methylen, Ethylen oder Ethyliden ist;
Het 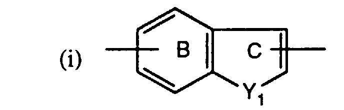 bedeutet, worin Y₁ für O, S oder N(R) steht und R Wasserstoff oder C₁-C₄-Alkyl bedeutet;
oder 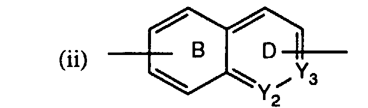 bedeutet, worin eine der Variablen Y₂ und Y₃ für CH und die andere für N steht oder beide Variablen jeweils für CH stehen;
X₁ für -CO- oder -S(O)ₘ- und der Index m für 0, 1 oder 2 steht;
eine der Variablen X₂ und X₄ für C₁-C₄-Alkylen und die andere der Variablen X₂ und X₄ für eine Bindung steht; oder beide Variablen X₂ und X₄ jeweils für eine Bindung stehen;
X₃ C₃-C₆-Cycloalkyliden oder das Strukturelement -C(Xₐ)(X_{b})- bedeutet und Xₐ Wasserstoff oder C₁-C₇-Alkyl und X_{b} C₁-C₇-Alkyl bedeuten;
und die Ringe A, B, C und D, bis auf die in der Formel angegebenen Substituenten, sowie aromatische Substituenten unabhängig voneinander unsubstituiert oder ein- oder mehrfach substituiert sind durch Substituenten ausgewählt aus der Gruppe bestehend aus Halogen, C₁-C₄-Alkyl, C₁-C₄-Alkoxy, C₃-C₅-Alkenyloxy, Phenoxy, Benzyloxy, Trifluormethyl und S(O)ₘ-R, worin m 0, 1 oder 2 ist und R Wasserstoff oder C₁-C₄-Alkyl ist;
und ihre Salze.

4. Verbindung gemäss Anspruch 2 oder 3 der Formel I, worin
R₁ C₂-C₇-Alkyl ist oder C₁-C₄-Alkyl bedeutet, welches durch Halogen oder Hydroxy substituiert ist, oder C₃-C₇-Alkenyl, C₃-C₆-Cycloalkyl oder C₁-C₇-Alkoxy bedeutet;
R₂ 1H-Tetrazol-5-yl, Carboxy oder C₁-C₄-Alkoxy-carbonyl ist;
R₃ 1H-Tetrazol-5-yl, Carboxy, C₁-C₄-Alkoxy-carbonyl, C₁-C₄-Alkoxy-C₁-C₄-alkoxy-carbonyl oder Phenyl-C₁-C₂-alkoxycarbonyl bedeutet;
Alk Methylen, ferner Ethylen oder Ethyliden ist;
Het 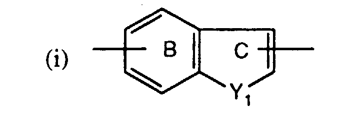 bedeutet, worin Y₁ für O, S oder NH steht; oder 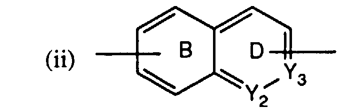 bedeutet, worin eine der Variablen Y₂ und Y₃ für CH und die andere für N steht oder beide Variablen jeweils für CH stehen;
in erster Linie 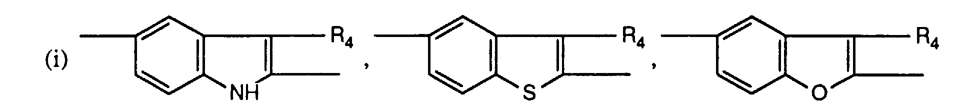 bedeutet und R₄ Wasserstoff, Halogen, wie Brom, ferner C₁-C₇-Alkyl, C₁-C₇-Alkoxy oder Trifluormethyl bedeutet; oder 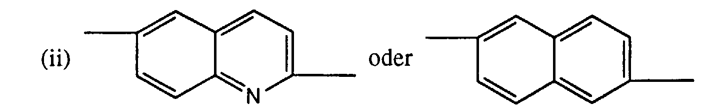 bedeutet,
X₁ für -CO- steht;
eine der Variablen X₂ und X₄ für C₁-C₂-Alkylen und die andere der Variablen X₂ und X₄ für eine Bindung steht; oder beide Variablen X₂ und X₄ jeweils für eine Bindung stehen;
X₃ C₅-C₆-Cycloalkyliden oder das Strukturelement -C(Xₐ)(X_{b})- bedeutet und Xₐ Wasserstoff oder C₁-C₅-Alkyl und X_{b} C₁-C₅-Alkyl bedeuten;
in erster Linie
(i) X₂ C₁-C₂-Alkylen ist; X₄ für eine Bindung steht; X₃ C₅-C₆-Cycloalkyliden bedeutet;
oder
(ii) X₂ und X₄ jeweils für eine Bindung stehen; und X₃ für das Strukturelement -C(Xₐ)(X_{b})- steht und Xₐ Wasserstoff oder C₁-C₅-Alkyl und X_{b} C₁-C₅-Alkyl bedeuten;
und die Ringe A, B, C und D, bis auf die in der Formel angegebenen Substituenten, unabhängig voneinander unsubstituiert oder ein- oder mehrfach substituiert sind durch Substituenten ausgewählt aus der Gruppe bestehend aus Halogen, C₁-C₄-Alkyl, C₁-C₄-Alkoxy und Trifluormethyl;
und ihre Salze.

5. Verbindung gemäss Anspruch 1 der Formel I, worin
eine der Variablen Y₂ und Y₃ für C(R') steht und die andere für N oder C(R') steht, wobei R' Halogen, C₁-C₄-Alkyl, C₁-C₄-Alkoxy oder Trifluormethyl bedeutet, und ihre Salze.

6. Verbindung gemäss Anspruch 1 der Formel I, worin
R₁ C₂-C₇-Alkyl, wie n-Propyl oder n-Butyl, oder C₃-C₆-Cycloalkyl, wie Cyclopropyl, oder C₁-C₄-Alkoxy, wie Methoxy, Ethoxy, Propyloxy oder Butyloxy, bedeutet;
R₂ 1H-Tetrazol-5-yl, Carboxy oder C₁-C₄-Alkoxy-carbonyl, wie Methoxy- oder Ethoxy-carbonyl, ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 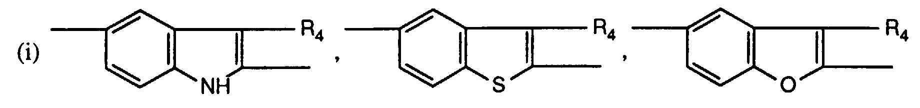 bedeutet und R₄ Wasserstoff, Halogen, wie Brom, ferner C₁-C₄-Alkyl, wie Methyl, C₁-C₄-Alkoxy, wie Methoxy, oder Trifluormethyl bedeutet; oder 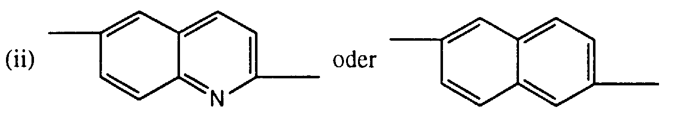 bedeutet;
X₁ für -CO- steht;
(i) X₂ C₁-C₂-Alkylen, insbesondere Methylen, ist; X₄ für eine Bindung steht; X₃ C₅-C₆-Cycloalkyliden bedeutet; oder
(ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie Ethyl oder Isopropyl, bedeuten;
und ihre Salze.

7. Verbindung gemäss einem der Ansprüche 2 bis 5 der Formel I, worin
R₃ Hydroxymethyl, C₁-C₄-Alkoxy-methyl oder Formyl bedeutet; die übrigen Variablen die jeweils vorstehend angegebenen Bedeutungen haben.

8. Verbindung gemäss Anspruch 1 der Formel I, worin
R₁ C₂-C₅-Alkyl, wie n-Propyl oder n-Butyl;
R₂ 1H-Tetrazol-5-yl oder Carboxy ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 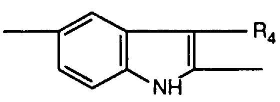 bedeutet und R₄ Wasserstoff, Halogen, wie Brom, oder
C₁-C₄-Alkyl, wie Methyl, ist;
X₁ für -CO- steht;
(i) X₂ C₁-C₂-Alkylen, insbesondere Methylen, ist; X₄ für eine Bindung steht; X₃ C₅-C₆-Cycloalkyliden bedeutet; oder
(ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie Ethyl, Isopropyl oder 3-Butyl, bedeuten;
und ihre Salze.

9. Verbindung gemäss Anspruch 1 der Formel I, worin
R₁ C₂-C₅-Alkyl, wie n-Propyl oder n-Butyl, bedeutet;
R₂ 1H-Tetrazol-5-yl oder Carboxy ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 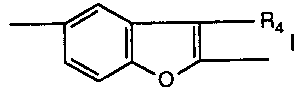 bedeutet und R₄ Wasserstoff, Halogen, wie Brom, oder C₁-C₄-Alkyl, wie Methyl, ist;
X₁ für -CO- steht;
(i) X₂ C₁-C₂-Alkylen, insbesondere Methylen, ist; X₄ für für eine Bindung steht; X₃ C₅-C₆-Cycloalkyliden bedeutet; oder
(ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie Ethyl, Isopropyl oder 3-Butyl, bedeuten;
und ihre Salze.

10. Verbindung gemäss Anspruch 1 der Formel I, worin
R₁ C₂-C₅-Alkyl, wie n-Propyl oder n-Butyl, bedeutet;
R₂ 1H-Tetrazol-5-yl oder Carboxy ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 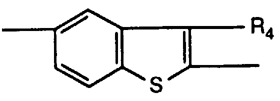 bedeutet, worin R₄ Wasserstoff, Halogen, wie Brom, oder C₁-C₄-Alkyl, wie Methyl, ist; X₁ für -CO- steht;
(i) X₂ C₁-C₂-Alkylen, insbesondere Methylen, ist; X₄ für für eine Bindung steht; X₃ C₅-C₆-Cycloalkyliden, wie Cyclopentyliden oder Cyclohexyliden, bedeutet; oder
(ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie Ethyl, Isopropyl oder 3-Butyl, bedeuten;
und ihre Salze.

11. Verbindung gemäss Anspruch 1 der Formel I, worin
R₁ C₂-C₅-Alkyl, wie n-Propyl oder n-Butyl, bedeutet;
R₂ 1H-Tetrazol-5-yl oder Carboxy ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 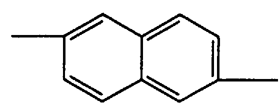 bedeutet;
X₁ für -CO- steht;
(i) X₂ C₁-C₂-Alkylen, insbesondere Methylen, ist; X₄ für für eine Bindung steht; X₃ C₅-C₆-Cycloalkyliden bedeutet; oder
(ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie, Ethyl, Isopropyl oder 3-Butyl, bedeuten;
und ihre Salze.

12. Verbindung gemäss Anspruch 1 der Formel I, worin
R₁ C₂-C₅-Alkyl, wie n-Propyl oder n-Butyl, bedeutet;
R₂ 1H-Tetrazol-5-yl oder Carboxy ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 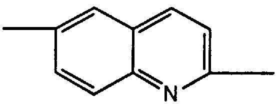 bedeutet;
X₁ für -CO- steht;
(i) X₂ C₁-C₂-Alkylen, insbesondere Methylen, ist; X₄ für für eine Bindung steht; X₃ C₅-C₆-Cycloalkyliden bedeutet; oder
(ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie Ethyl, Isopropyl oder 3-Butyl, bedeuten;
und ihre Salze.

13. Verbindung gemäss Anspruch 1 der Formel I, worin
R₁ C₂-C₅-Alkyl, wie Ethyl, n-Propyl oder n-Butyl, bedeutet;
R₂ 1H-Tetrazol-5-yl oder Carboxy ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 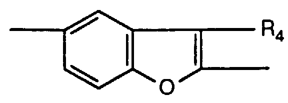 bedeutet; und R₄ Wasserstoff oder Halogen mit Atomnummer bis und mit 35, wie Brom, ist;
X₁ für -CO- steht;
(i) X₂ C₁-C₂-Alkyllen, insbesondere Methylen, ist; X₄ für für eine Bindung steht; X₃ C₅-C₆-Cycloalkyliden bedeutet; oder
(ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie Ethyl, Isopropyl oder 3-Butyl, bedeuten;
und ihre Salze.

14. Verbindung gemäß Anspruch 1, ausgewählt aus
N-[2-(2'-(1H-Tetrazol-5-yl)-phenyl)-chinolin-6-yl-methyl]-N-valeroyl-valin;
N-[2-(2'-(1H-Tetrazol-5-yl)-phenyl)-chinolin-6-yl-methyl]-N-valeroyl-1-aminomethyl-cyclopentan-1-carbonsäure;
N-[2-(2'-(1H-Tetrazol-5-yl)-phenyl)-chinolin-6-yl-methyl]-N-valeroyl-2-aminomethyl-2-ethyl-buttersäure;
N-[2-(2'-(1H-Tetrazol-5-yl)-phenyl)-benzo[b]thiophen-5-yl-methyl]-N-valeroyl-2-aminomethyl-2-ethyl-buttersäure;
N-[3-Brom-2-(2'-carboxy-phenyl)-benzo[b]thiophen-5-yl-methyl]-N-valeroyl-valin;
N-[2-(2'-(1H-Tetrazol-5-yl)-phenyl)-benzofuran-5-yl-methyl]-N-valeroyl-2-aminomethyl-2-ethyl-buttersäure;
N-[2-(2'-(1H-Tetrazol-5-yl)-phenyl)-benzofuran-5-yl-methyl]-N-valeroyl-1-aminomethyl-cyclopentan-1-carbonsäure;
N-[2-(2'-(1H-Tetrazol-5-yl)-phenyl)-benzofuran-5-yl-methyl]-N-valeroyl-valin;
N-[2-(2'-(1H-Tetrazol-5-yl)-phenyl)-5-yl-methyl-benzo[b]thiophen]-N-valeroyl-1-aminomethyl-cyclopentan-1-carbonsäure;
N-[2-(2'-(1H-Tetrazol-5-yl)-phenyl)-benzo[b]thiophen-5-yl-methyl]-N-valeroyl-valin;
N-[3-Brom-2-(2'-carboxyphenyl)-benzo[b]thiophen-5-yl-methyl]-N-valeroyl-1-aminomethyl-cyclopentan-1-carbonsäure; und
N-[2-(2'-(1H-Tetrazol-5-yl)-phenyl)-benzofuran-5-yl-methyl]-N-valeroyl-1-aminomethyl-cyclohexan-1-carbonsäure;
N-[2-(2'-(Tetrazol-5-yl)-phenyl)-naphthalin-6-yl-methyl]-N-valeroyl-valin;
N-[2-(2'-(Tetrazol-5-yl)-phenyl)-naphthalin-6-yl-methyl]-N-valeroyl-1-aminomethyl-cyclopentan-1-carbonsäure;
N-[2-(2'-(Tetrazol-5-yl)-phenyl)-naphthalin-6-yl-methyl]-N-valeroyl-2-aminomethyl-2-ethyl-buttersäure;
N-[2-(2'-(Tetrazol-5-yl)-phenyl)-naphthalin-6-yl-methyl]-N-valeroyl-1-aminomethyl-cyclohexan-1-carbonsäure;
N-[2-(2'-(Tetrazol-5-yl)-phenyl)-chinolin-6-yl-methyl]-N-cyclopropylcarbonyl-valin;
N-[2-(2'-(Tetrazol-5-yl)-phenyl)-chinolin-6-yl-methyl]-N-cyclopropylcarbonyl-1-aminomethyl-cyclopentan-1-carbonsäure;
N-[2-(2'-(Tetrazol-5-yl)-phenyl)-chinolin-6-yl-methyl]-N-cyclopropylcarbonyl-2-anlinomethyl-2-ethyl-buttersäure;
N-[2-(2'-(Tetrazol-5-yl)-phenyl)-chinolin-6-yl-methyl]-N-cyclopropylcarbonyl-valin; oder ein Salz davon.

15. Verbindung gemäß Anspruch 1, ausgewählt aus
N-[2-(2'-(1H-Tetrazol-5-yl)phenyl)-naphthalin-6-ylmethyl]-N-valeroyl-1-amino-methylcyclopentan-1-carbonsäureethylester;
N-[2-(2'-(1H-Tetrazol-5-yl)-phenyl)-naphthalin-6-ylmethyl]-N-valeroyl-1-aminomethyl-cyclopentan-1-carbonsäure;
N-[2-(2'-(1H-Tetrazol-5-yl)-phenyl-naphthalin-6-ylmethyl]-N-valeroyl-3-amino-2,2-dimethyl-propionsäure;
N-[2-(2'-(1H-Tetrazol-5-yl)-phenyl)-naphthalin)-6-yl-methyl]-N-butyryl-valin; und
N-[2-(2'-(1H-Tetrazol-5-yl)-phenyl)-naphthalin)-6-ylmethyl]-N-valeroyl-valin; oder ein Salz davon.

16. Verbindung gemäß Anspruch 1, ausgewählt aus
N-{3-Brom-2-[2'-(1H-tetrazol-5-yl)phenyl]-5-ylmethyl-benzofuran}-N-valeroyl-1-aminomethyl-cyclopentan-1-carbonsäure-ethylester;
N-{3-Brom-2-[2'-(1H-tetrazol-5-yl)phenyl]-5-ylmethyl-benzofuran}-N-butyryl-1-aminomethyl-cyclopentan-1-carbonsäure-ethylester;
N-{3-Brom-2-[2'-(1H-tetrazol-5-yl)phenyl]-5-ylmethyl-benzofuran}-N-caproyl-1-aminomethyl-cyclopentan-1-carbonsäure-ethylester;
N-{3-Brom-2-[2'-(1H-tetrazol-5-yl)phenyl]-5-ylmethyl-benzofuran}-N-valeroyl-valin-ethylester;
N-{3-Brom-2-[2'-(1H-tetrazol-5-yl)phenyl]-5-ylmethyl-benzofuran}-N-butyryl-valin-ethylester;
N-{3-Brom-2-[2'-(1H-tetrazol-5-yl)phenyl]-5-ylmethyl-benzofuran}-N-caproyl-valin-ethylester;
N-{3-Brom-2-[2'-(1H-tetrazol-5-yl)phenyl]-5-ylmethyl-benzofuran}-N-valeroyl-alanin-ethylester;
N-{3-Brom-2-[2'-(1H-tetrazol-5-yl)phenyl]-5-ylmethyl-benzofuran}-N-valeroyl-1-aminomethyl-cyclopentan-1-carbonsäure;
N-{3-Brom-2-[2'-(1H-tetrazol-5-yl)phenyl]-5-ylmethyl-benzofuran}-N-butyryl-1-aminomethyl-cyclopentan-1-carbonsäure;
N-{3-Brom-2-[2'-(1H-tetrazol-5-yl)phenyl]-5-ylmethyl-benzofuran}-N-caproyl-1-aminomethyl-cyclopentan-1-carbonsäure;
N-{3-Brom-2-[2'-(1H-tetrazol-5-yl)phenyl]-5-ylmethyl-benzofuran}-N-valeroyl-valin;
N-{3-Brom-2-[2'-(1H-tetrazol-5-yl)phenyl]-5-ylmethyl-benzofuran}-N-butyryl-valin;
N-{3-Brom-2-[2'-(1H-tetrazol-5-yl)phenyl]-5-ylmethyl-benzofuran}-N-caproyl-valin; und
N-{3-Brom-2-[2'-(1H-tetrazol-5-yl)phenyl]-5-ylmethyl-benzofuran}-N-valeroyl-alanin; oder ein Salz davon.

17. Verbindung gemäß Anspruch 1, ausgewählt aus
N-{3-Brom-2-[2'-(1H-tetrazol-5-yl)-phenyl]-benzofuran-5-ylmethyl}-N-valeroyl-1-aminomethyl-cyclopentan-1-carbonsäure-ethylester,
N-{3-Brom-2-[2'-(1H-tetrazol-5-yl)-phenyl}-benzofuran-5-ylmethyl}-N-butyryl-1-aminomethyl-cyclopentan-1-carbonsäure-ethylester;
N-{3-Brom-2-[2'-(1H-tetrazol-5-yl)-phenyl]-benzofuran-5-ylmethyl}-N-caproyl-1-aminomethyl-cyclopentan-1-carbonsäure-ethylester;
N-{3-Brom-2-[2'-(1H-tetrazol-5-yl)-phenyl]-benzofuran-5-ylmethyl}-N-valeroyl-valin-ethylester;
N-{3-Brom-2-[2'-(1H-tetrazol-5-yl)-phenyl]-benzofuran-5-ylmethyl}-N-butyryl-valin-ethylester;
N-{3-Brom-2-[2'-(1H-tetrazol-5-yl)-phenyl]-benzofuran-5-ylmethyl}-N-caproyl-valin-ethylester;
N-{3-Brom-2-[2'-(1H-tetrazol-5-yl)-phenyl]-benzofuran-5-ylmethyl}-N-valeroyl-alanin-ethylester;
N-{3-Brom-2-[2'-(1H-tetrazol-5-yl)-phenyl]-benzofuran-5-ylmethyl}-N-valeroyl-1-aminomethyl-cyclopentan-1-carbonsäure,
N-{3-Brom-2-[2'-(1H-tetrazol-5-yl)-phenyl]-benzofuran-5-ylmethyl}-N-butyryl-1-aminomethyl-cyclopentan-1-carbonsäure;
N-{3-Brom-2-[2'-(1H-tetrazol-5-yl)-phenyl]-benzofuran-5-ylmethyl}-N-caproyl-1-aminomethyl-cyclopentan-1-carbonsäure;
N-{3-Brom-2-[2'-(1H-tetrazol-5-yl)-phenyl]-benzofuran-5-ylmethyl}-N-valeroyl-valin;
N-{3-Brom-2-[2'-(1H-tetrazol-5-yl)-phenyl]-benzofuran-5-ylmethyl}-N-butyryl-valin;
N-{3-Brom-2-[2'-(1H-tetrazol-5-yl)-phenyl]-benzofuran-5-ylmethyl}-N-caproyl-valin; und
N-{3-Brom-2-[2'-(1H-tetrazol-5-yl)-phenyl]-benzofuran-5-ylmethyl}-N-valeroyl-alanin oder ein Salz davon.

18. Verbindung gemäss einem der Ansprüche 1 bis 17 zur Anwendung in einem Verfahren zur therapeutischen Behandlung des tierischen oder menschlichen Körpers.

19. Verfahren zur Herstellung einer Verbindungen der Formel I und eines Salzes davon gemäss einem der Ansprüche 1 bis 17, dadurch gekennzeichnet ist, dass man
a) in einer Verbindung der Formel 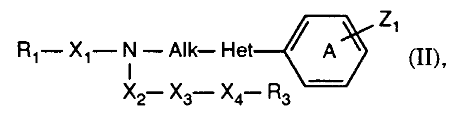 worin Z₁ einen in R₂ überführbaren Rest bedeutet, oder einem Salz davon, Z₁ in R₂ überführt; oder
b) eine Verbindung der Formel 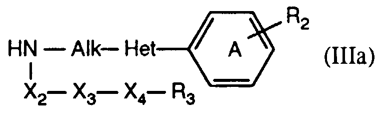 mit einer Verbindung der Formel R₁-X₁-OH (IIIb), einem reaktionsfähigen Derivat davon oder ein Salz davon, umsetzt;
und jeweils, wenn erwünscht, eine verfahrensgemäss oder auf andere Weise erhältliche Verbindung I, in freier Form oder in Salzform, isoliert, eine verfahrensgemäss oder auf andere Weise erhältliche Verbindung I in eine andere Verbindung I überführt, ein verfahrensgemäss erhältliches Gemisch von Isomeren auftrennt und das gewünschte Isomere isoliert und/oder eine verfahrensgemäss erhältliche freie Verbindung I in ein Salz oder ein verfahrensgemäss erhältliches Salz einer Verbindung I in die freie Verbindung I oder in ein anderes Salz überführt.

20. Ein pharmazeutisches Präparat enthaltend als Wirkstoff eine Verbindung gemäss einem der Ansprüche 1-18, in freier Form oder in Form eines pharmazeutisch verwendbaren Salzes, gegebenenfalls neben üblichen Hilfsstoffen.

21. Verwendung einer Verbindung gemäss einem der Ansprüche 1-18, in freier Form oder in Form eines pharmazeutisch verwendbaren Salzes, zur Herstellung eines pharmazeutischen Präparats zur Behandlung von von Bluthochdruck und Herzinsuffizienz sowie von Glaukom.

## Patentansprüche (Patentansprüche für folgende(n) Vertragsstaat(en): ES, GR)

1. Verfahren zur Herstellung einer Verbindungen der Formel 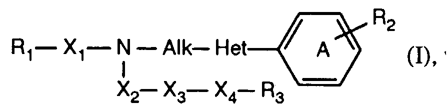 worin
R₁ C₁-C₇-Alkyl bedeutet, welches unsubstituiert oder durch Halogen oder Hydroxy substituiert ist, oder C₂-C₇-Alkenyl, C₃-C₇-Cycloalkyl, C₃-C₇-Cycloalkyloxy, C₁-C₇-Alkoxy oder C₃-C₇-Cycloalkyl-C₁-C₇-alkoxy bedeutet;
R₂ 1H-Tetrazol-5-yl, Carboxy, C₁-C₇-Alkoxy-carbonyl, SO₃H, PO₂H₂, PO₃H₂ oder Halogen-C₁-C₇-alkansulfonylamino ist;
R₃ 1H-Tetrazol-5-yl, Hydroxymethyl, C₁-C₇-Alkoxy-methyl, Formyl, Carboxy, C₁-C₇-Alkoxy-carbonyl, C₁-C₇-Alkoxy-C₁-C₇-alkoxy-carbonyl, Phenyl-C₁-C₄-alkoxy-carbonyl oder Carbamoyl bedeutet, dessen Aminogruppe unsubstituiert oder durch C₁-C₇-Alkyl, C₃-C₇-Alkenyl oder Phenyl-C₁-C₇-alkyl mono- oder unabhängig voneinander disubstituiert oder durch C₂-C₇-Alkylen oder C₂-C₄-Alkylenoxy-C₂-C₄-alkylen disubstituiert ist;
Alk Methylen, Ethylen oder Ethyliden ist;
Het 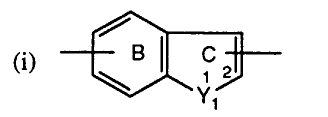
bedeutet, worin Y₁ für O, S oder N(R) steht und R Wasserstoff oder C₁-C₇-Alkyl bedeutet; oder 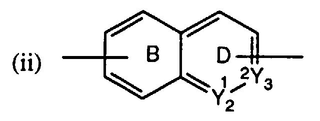 bedeutet, worin eine der Variablen Y₂ und Y₃ für C(R') und die andere für N steht oder beide Variablen jeweils für C(R') stehen; und R' Wasserstoff, Halogen, C₁-C₇-Alkyl, C₁-C₇-Alkoxy, C₂-C₇-Alkenyloxy, Phenoxy, Benzyloxy, Trifluormethyl oder S(O)ₘ-R bedeutet, worin m 0, 1 oder 2 ist; und R Wasserstoff oder C₁-C₇-Alkyl ist;
X₁ für -CO- oder -S(O)ₘ- und der Index m für 0, 1 oder 2 steht;
eine der Variablen X₂ und X₄ für C₁-C₄-Alkylen und die andere der Variablen X₂ und X₄ für eine Bindung steht; oder beide Variablen X₂ und X₄ jeweils für eine Bindung stehen;
X₃ C₃-C₇-Cycloalkyliden oder das Strukturelement -C(Xₐ)(X_{b})- bedeutet und Xₐ
Wasserstoff oder C₁-C₇-Alkyl ist und X_{b} C₁-C₇-Alkyl ist;
und die Ringe A, B, C und D, bis auf die in der Formel angegebenen Substituenten, sowie aromatische Substituenten unabhängig voneinander unsubstituiert oder ein- oder mehrfach substituiert sind durch Substituenten ausgewählt aus der Gruppe bestehend aus Halogen, C₁-C₇-Alkyl, C₁-C₇-Alkoxy, C₂-C₇-Alkenyloxy, Phenoxy, Benzyloxy, Trifluormethyl und
S(O)ₘ-R, worin m 0, 1 oder 2 ist und R Wasserstoff oder C₁-C₇-Alkyl ist;
oder eines Salzes davon;
dadurch gekennzeichnet ist, dass man
a) in einer Verbindung der Formel 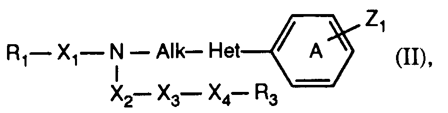 worin Z₁ einen in R₂ überführbaren Rest bedeutet, oder einem Salz davon, Z₁ in R₂ überführt; oder
b) eine Verbindung der Formel 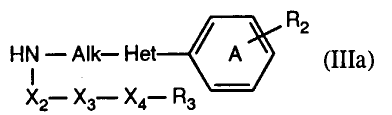 mit einer Verbindung der Formel R₁-X₁-OH (IIIb), einem reaktionsfähigen Derivat davon oder ein Salz davon, umsetzt;
und jeweils, wenn erwünscht, eine verfahrensgemäss oder auf andere Weise erhältliche Verbindung I, in freier Form oder in Salzform, isoliert, eine verfahrensgemäss oder auf andere Weise erhältliche Verbindung I in eine andere Verbindung I überführt, ein verfahrensgemäss erhältliches Gemisch von Isomeren auftrennt und das gewünschte Isomere isoliert und/oder eine verfahrensgemäss erhältliche freie Verbindung I in ein Salz oder ein verfahrensgemäss erhältliches Salz einer Verbindung I in die freie Verbindung I oder in ein anderes Salz überführt.

2. Verfahren nach Anspruch I zur Herstellung einer Verbindung der Formel I, worin R₁ C₁-C₇-Alkyl bedeutet, welches unsubstituiert oder durch Halogen oder Hydroxy substituiert ist, oder C₂-C₇-Alkenyl, C₃-C₇-Cycloalkyl oder C₁-C₇-Alkoxy bedeutet; oder eines Salzes davon.

3. Verfahren nach Anspruch 1 zur Herstellung einer Verbindung der Formel 1, worin
R₁ C₂-C₇-Alkyl ist oder C₁-C₇-Alkyl bedeutet, welches durch Halogen oder Hydroxy substituiert, oder C₃-C₇-Alkenyl, C₃-C₆-Cycloalkyl oder C₁-C₇-Alkoxy bedeudet;
R₂ 1H-Tetrazol-5-yl, Carboxy, C₁-C₄-Alkoxy-carbonyl oder Halogen-C₁-C₄-alkansulfonylamino ist;
R₃ 1H-Tetrazol-5-yl, Carboxy, C₁-C₄-Alkoxy-carbonyl, C₁-C₄-Alkoxy-C₁-C₄-alkoxy-carbonyl, Phenyl-C₁-C₄-alkoxycarbonyl oder Carbamoyl bedeutet, dessen Aminogruppe durch C₁-C₄-Alkyl mono- oder unabhängig voneinander disubstituiert oder durch C₄-C₆-Alkylen oder Ethylenoxyethylen disubstituiert ist;
Alk Methylen, Ethylen oder Ethyliden ist;
Het 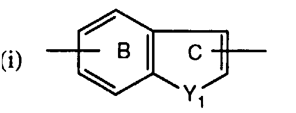 bedeutet, worin Y₁ für O, S oder N(R) steht und R Wasserstoff oder C₁-C₄-Alkyl bedeutet;
oder 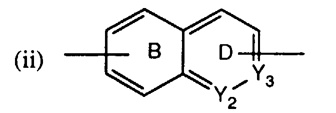 bedeutet, worin eine der Variablen Y₂ und Y₃ für CH und die andere für N steht oder beide Variablen jeweils für CH stehen;
X₁ für -CO- oder -S(O)ₘ- und der Index m für 0, 1 oder 2 steht;
eine der Variablen X₂ und X₄ für C₁-C₄-Alkylen und die andere der Variablen X₂ und X₄ für eine Bindung steht; oder beide Variablen X₂ und X₄ jeweils für eine Bindung stehen;
X₃ C₃-C₆-Cycloalkyliden oder das Strukturelement -C(Xₐ)(X_{b})- bedeutet und Xₐ Wasserstoff oder C₁-C₇-Alkyl und X_{b} C₁-C₇-Alkyl bedeuten;
und die Ringe A, B, C und D, bis auf die in der Formel angegebenen Substituenten, sowie aromatische Substituenten unabhängig voneinander unsubstituiert oder ein- oder mehrfach substituiert sind durch Substituenten ausgewählt aus der Gruppe bestehend aus Halogen, C₁-C₄-Alkyl, C₁-C₄-Alkoxy, C₃-C₅-Alkenyloxy, Phenoxy, Benzyloxy, Trifluormethyl und
S(O)ₘ-R, worin m 0, 1 oder 2 ist und R Wasserstoff oder C₁-C₄-Alkyl ist;
oder eines Salzes davon.

4. Verfahren nach Anspruch 1 zur Herstellung einer Verbindung der Formel I, worin
R₁ C₂-C₇-Alkyl ist oder C₁-C₄-Alkyl bedeutet, welches durch Halogen oder Hydroxy substituiert ist, oder C₃-C₇-Alkenyl, C₃-C₆-Cycloalkyl oder C₁-C₇-Alkoxy bedeutet;
R₂ 1H-Tetrazol-5-yl, Carboxy oder C₁-C₄-Alkoxy-carbonyl ist;
R₃ 1H-Tetrazol-5-yl, Carboxy, C₁-C₄-Alkoxy-carbonyl, C₁-C₄-Alkoxy-C₁-C₄-Alkoxy-carbonyl oder Phenyl-C₁-C₂-alkoxycarbonyl bedeutet;
Alk Methylen, ferner Ethylen oder Ethyliden ist;
Het 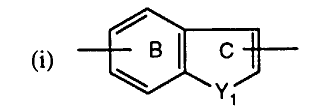 bedeutet, worin Y₁ für O, S oder NH steht; oder 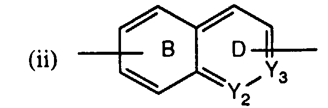 bedeutet, worin eine der Variablen Y₂ und Y₃ für CH und die andere für N steht oder beide Variablen jeweils für CH stehen; in erster Linie 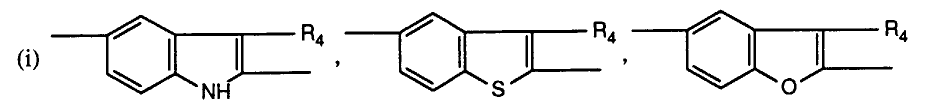 bedeutet und R₄ Wasserstoff, Halogen, wie Brom, ferner C₁-C₇-Alkyl, C₁-C₇-Alkoxy oder Trifluormethyl bedeutet; oder 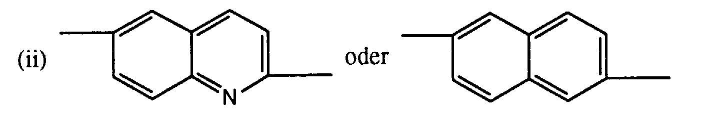 bedeutet, X₁ für -CO- steht; eine der Variablen X₂ und X₄ für C₁-C₂-Alkylen und die andere der Variablen X₂ und X₄ für eine Bindung steht; oder beide Variablen X₂ und X₄ jeweils für eine Bindung stehen; X₃ C₅-C₆-Cycloalkyliden oder das Strukturelement -C(Xₐ)(X_{b})- bedeutet und Xₐ Wasserstoff oder C₁-C₅-Alkyl und X_{b} C₁-C₅-Alkyl bedeuten; in erster Linie (i) X₂ C₁-C₂-Alkylen ist; X₄ für eine Bindung steht; X₃ C₅-C₆-Cycloalkyliden bedeutet; oder (ii) X₂ und X₄ jeweils für eine Bindung stehen; und X₃ für das Strukturelement -C(Xₐ)(X_{b})- steht und Xₐ Wasserstoff oder C₁-C₅-Alkyl und X_{b} C₁-C₅-Alkyl bedeuten; und die Ringe A, B, C und D, bis auf die in der Formel angegebenen Substituenten, unabhängig voneinander unsubstituiert oder ein- oder mehrfach substituiert sind durch Substituenten ausgewählt aus der Gruppe bestehend aus Halogen, C₁-C₄-Alkyl, C₁-C₄-Alkoxy und Trifluormethyl;eine der Variablen Y₂ und Y₃ für C(R') steht und die andere für N oder C(R') steht, wobei R' Halogen, C₁-C₄-Alkyl, C₁-C₄-Alkoxy oder Trifluormethyl bedeutet; oder eines Salzes davon.

5. Verfahren nach Anspruch 1 zur Herstellung einer Verbindung der Formel I, worin eine der Variablen Y₂ und Y₃ für C(R') steht und die andere für N oder C(R') steht, wobei R' Halogen, C₁-C₄-Alkyl, C₁-C₄-Alkoxy oder Trifluormethyl bedeutet, oder eines Salzes davon.

6. Verfahren nach Anspruch 1 zur Herstellung einer Verbindung der Formel I, worin
R₁ C₂-C₇-Alkyl, wie n-Propyl oder n-Butyl, oder C₃-C₆-Cycloalkyl, wie Cyclopropyl, oder C₁-C₄-Alkoxy, wie Methoxy, Ethoxy, Propyloxy oder Butyloxy, bedeutet;
R₂ 1H-Tetrazol-5-yl, Carboxy oder C₁-C₄-Alkoxy-carbonyl, wie Methoxy- oder Ethoxy-carbonyl, ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 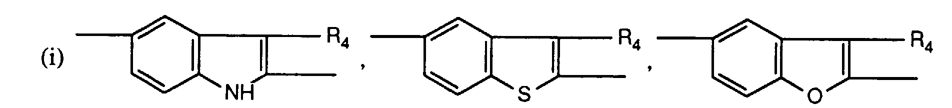 bedeutet und R₄ Wasserstoff, Halogen, wie Brom, ferner C₁-C₄-Alkyl, wie Methyl, C₁-C₄-Alkoxy, wie Methoxy, oder Trifluormethyl bedeutet; oder 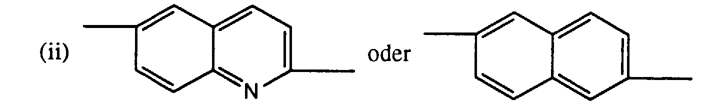 bedeutet; X₁ für -CO- steht; (i) X₂ C₁-C₂-Alkylen, insbesondere Methylen, ist; X₄ für eine Bindung steht; X₃ C₅-C₆-Cycloalkyliden bedeutet; oder (ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie Ethyl oder Isopropyl, bedeuten; oder eines Salzes davon.

7. Verfahren nach Anspruch 1 zur Herstellung einer Verbindung der Formel I, worin R₃ Hydroxymethyl, C₁-C₄-Alkoxy-methyl oder Formyl bedeutet; die übrigen Variablen die jeweils vorstehend angegebenen Bedeutungen haben; oder eines Salzes davon.

8. Verfahren nach Anspruch 1 zur Herstellung einer Verbindung der Formel I, worin
R₁ C₂-C₅-Alkyl, wie n-Propyl oder n-Butyl;
R₂ 1H-Tetrazol-5-yl oder Carboxy ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 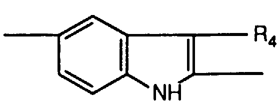 bedeutet und R₄ Wasserstoff, Halogen, wie Brom, oder C₁-C₄-Alkyl, wie Methyl, ist;
X₁ für -CO- steht;
(i) X₂ C₁-C₂-Alkylen, insbesondere Methylen, ist; X₄ für eine Bindung steht; X₃ C₅-C₆-Cycloalkyliden bedeutet; oder
(ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie Ethyl, Isopropyl oder 3-Butyl, bedeuten;
oder eines Salzes davon.

9. Verfahren nach Anspruch 1 zur Herstellung einer Verbindung der Formel I, worin
R₁ C₂-C₅-Alkyl, wie n-Propyl oder n-Butyl, bedeutet;
R₂ 1H-Tetrazol-5-yl oder Carboxy ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 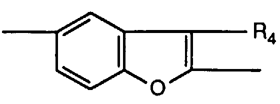 bedeutet und R₄ Wasserstoff, Halogen, wie Brom, oder C₁-C₄-Alkyl, wie Methyl, ist;
X₁ für -CO- steht;
(i) X₂ C₁-C₂-Alkylen, insbesondere Methylen, ist; X₄ für für eine Bindung steht; X₃ C₅-C₆-Cycloalkyliden bedeutet; oder
(ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie Ethyl, Isopropyl oder 3-Butyl, bedeuten;
oder eines Salzes davon.

10. Verfahren nach Anspruch 1 zur Herstellung einer Verbindung der Formel I, worin
R₁ C₂-C₅-Alkyl, wie n-Propyl oder n-Butyl, bedeutet;
R₂ 1H-Tetrazol-5-yl oder Carboxy ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 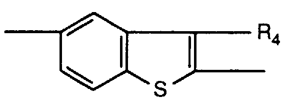 bedeutet, worin R₄ Wasserstoff, Halogen, wie Brom, oder C₁-C₄-Alkyl, wie Methyl, ist;
X₁ für -CO- steht;
(i) X₂ C₁-C₂-Alkylen, insbesondere Methylen, ist; X₄ für für eine Bindung steht; X₃ C₅-C₆-Cycloalkyliden, wie Cyclopentyliden oder Cyclohexyliden, bedeutet; oder
(ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie Ethyl, Isopropyl oder 3-Butyl, bedeuten;
oder eines Salzes davon.

11. Verfahren nach Anspruch 1 zur Herstellung einer Verbindung der Formel I, worin
R₁ C₂-C₅-Alkyl, wie n-Propyl oder n-Butyl, bedeutet;
R₂ 1H-Tetrazol-5-yl oder Carboxy ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 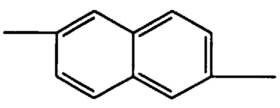 bedeutet;
X₁ für -CO- steht;
(i) X₂ C₁-C₂-Alkylen, insbesondere Methylen, ist; X₄ für für eine Bindung steht; X₃ C₅-C₆-Cycloalkyliden bedeutet; oder
(ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie, Ethyl, Isopropyl oder 3-Butyl, bedeuten; oder eines Salzes davon.

12. Verfahren nach Anspruch 1 zur Herstellung einer Verbindung der Formel I, worin
R₁ C₂-C₅-Alkyl, wie n-Propyl oder n-Butyl, bedeutet;
R₂ 1H-Tetrazol-5-yl oder Carboxy ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 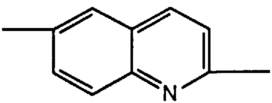 bedeutet;
X₁ für -CO- steht;
(i) X₂ C₁-C₂-Alkylen, insbesondere Methylen, ist; X₄ für für eine Bindung steht; X₃ C₅-C₆-Cycloalkyliden bedeutet; oder
(ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie Ethyl, Isopropyl oder 3-Butyl, bedeuten;
oder eines Salzes davon.

13. Verfahren nach Anspruch 1 zur Herstellung einer Verbindung der Formel I, worin
R₁ C₂-C₅-Alkyl, wie Ethyl, n-Propyl oder n-Butyl, bedeutet;
R₂ 1H-Tetrazol-5-yl oder Carboxy ist;
R₃ Carboxy oder C₁-C₄-Alkoxy-carbonyl, wie Methoxy-, Ethoxy- oder tert-Butyloxy-carbonyl, bedeutet;
Alk Methylen ist;
Het 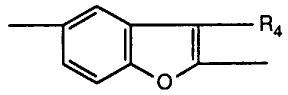 bedeutet; und R₄ Wasserstoff oder Halogen mit Atomnummer bis und mit 35, wie Brom, ist;
X₁ für -CO- steht;
(i) X₂ C₁-C₂-Alkylen, insbesondere Methylen, ist; X₄ für für eine Bindung steht; X₃ C₅-C₆-Cycloalkyliden bedeutet; oder
(ii) X₂ und X₄ jeweils für eine Bindung stehen; und X₃ für das Strukturelement -C(Xₐ)(X_{b})- steht und Xₐ Wasserstoff oder C₁-C₅-Alkyl, wie Ethyl, und X_{b} C₁-C₅-Alkyl, wie Ethyl, Isopropyl oder 3-Butyl, bedeuten;
oder eines Salzes davon.

14. Verfahren nach Anspruch 1 zur Herstellung einer Verbindung der Formel I ausgewählt aus
N-[2-(2'-(1H-Tetrazol-5-yl)-phenyl)-chinolin-6-yl-methyl]-N-valeroyl-valin;
N-[2-(2'-(1H-Tetrazol-5-yl)-phenyl)-chinolin-6-yl-methyl]-N-valeroyl-1-aminomethyl-cyclopentan-1-carbonsäure;
N-[2-(2'-(1H-Tetrazol-5-yl)-phenyl)-chinolin-6-yl-methyl]-N-valeroyl-2-aminomethyl-2-ethyl-buttersäure;
N-[2-(2'-(1H-Tetrazol-5-yl)-phenyl)-benzo[b]thiophen-5-yl-methyl]-N-valeroyl-2-aminomethyl-2-ethyl-buttersäure;
N-[3-Brom-2-(2'-carboxy-phenyl)-benzo[b]thiophen-5-yl-methyl]-N-valeroyl-valin;
N-[2-(2'-(1H-Tetrazol-5-yl)-phenyl)-benzofuran-5-yl-methyl]-N-valeroyl-2-aminomethyl-2-ethyl-buttersäure;
N-[2-(2'-(1H-Tetrazol-5-yl)-phenyl)-benzofuran-5-yl-methyl]-N-valeroyl-1-aminomethyl-cyclopentan-1-carbonsäure;
N-[2-(2'-(1H-Tetrazol-5-yl)-phenyl)-benzofuran-5-yl-methyl]-N-valeroyl-valin;
N-[2-(2'-(1H-Tetrazol-5-yl)-phenyl)-5-yl-methyl-benzo[b]thiophen]-N-valeroyl-1-aminomethyl-cyclopentan-1-carbonsäure;
N-[2-(2'-(1H-Tetrazol-5-yl)-phenyl)-benzo[b]thiophen-5-yl-methyl]-N-valeroyl-valin;
N-[3-Brom-2-(2'-carboxyphenyl)-benzo[b]thiophen-5-yl-methyl]-N-valeroyl-1-aminomethyl-cyclopentan-1-carbonsäure;
N-[2-(2'-(1H-Tetrazol-5-yl)-phenyl)-benzofuran-5-yl-methyl]-N-valeroyl-1-aminomethyl-cyclohexan-1-carbonsäure;
N-[2-(2'-(Tetrazol-5-yl)-phenyl)-naphthalin-6-yl-methyl]-N-valeroyl-valin;
N-[2-(2'-(Tetrazol-5-yl)-phenyl)-naphthalin-6-yl-methyl]-N-valeroyl-1-aminomethyl-cyclopentan-1-carbonsäure;
N-[2-(2'-(Tetrazol-5-yl)-phenyl)-naphthalin-6-yl-methyl]-N-valeroyl-2-aminomethyl-2-ethyl-buttersäure;
N-[2-(2'-(Tetrazol-5-yl)-phenyl)-naphthalin-6-yl-methyl]-N-valeroyl-1-aminomethyl-cyclohexan-1-carbonsäure;
N-[2-(2'-(Tetrazol-5-yl)-phenyl)-chinolin-6-yl-methyl]-N-cyclopropylcarbonyl-valin;
N-[2-(2'-(Tetrazol-5-yl)-phenyl)-chinolin-6-yl-methyl]-N-cyclopropylcarbonyl-1-aminomethyl-cyclopentan-1-carbonsäure;
N-[2-(2'-(Tetrazol-5-yl)-phenyl)-chinolin-6-yl-methyl]-N-cyclopropylcarbonyl-2-aminomethyl-2-ethyl-buttersäure; und
N-[2-(2'-(Tetrazol-5-yl)-phenyl)-chinolin-6-yl-methyl]-N-cyclopropylcarbonyl-valin; oder eines Salz davon.

15. Verfahren nach Anspruch 1 zur Herstellung einer Verbindung der Formel I ausgewählt aus
N-[2-(2'-(1H-Tetrazol-5-yl)phenyl)-naphthalin-6-ylmethyl]-N-valeroyl-1-amino-methyl-cyclopentan-1-carbonsäureethylester;
N-[2-(2'-(1H-Tetrazol-5-yl)-phenyl)-naphthalin-6-ylmethyl]-N-valeroyl-1-aminomethyl-cyclopentan-1-carbonsäure;
N-[2-(2'-(1H-Tetrazol-5-yl)-phenyl-naphthalin-6-ylmethyl]-N-valeroyl-3-amino-2,2-dimethyl-propionsäure;
N-[2-(2'-(1H-Tetrazol-5-yl)-phenyl)-naphthalin)-6-yl-methyl]-N-butyryl-valin; und
N-[2-(2'-(1H-Tetrazol-5-yl)-phenyl)-naphthalin)-6-ylmethyl]-N-valeroyl-valin; oder eines Salz davon

16. Verfahren nach Anspruch 1 zur Herstellung einer Verbindung der Formel I ausgewählt aus
N-{3-Brom-2-[2'-(1H-tetrazol-5-yl)phenyl]-5-ylmethyl-benzofuran}-N-valeroyl-1-aminomethyl-cyclopentan-1-carbonsäure-ethylester;
N-{3-Brom-2-[2'-(1H-tetrazol-5-yl)phenyl]-5-ylmethyl-benzofuran}-N-butyryl-1-aminomethyl-cyclopentan-1-carbonsäure-ethylester;
N-{3-Brom-2-[2'-(1H-tetrazol-5-yl)phenyl]-5-ylmethyl-benzofuran}-N-caproyl-1-aminomethyl-cyclopentan-1-carbonsäure-ethylester;
N-{3-Brom-2-[2'-(1H-tetrazol-5-yl)phenyl]-5-ylmethyl-benzofuran}-N-valeroyl-valin-ethylester;
N-{3-Brom-2-[2'-(1H-tetrazol-5-yl)phenyl]-5-ylmethyl-benzofuran}-N-butyryl-valin-ethylester;
N-{3-Brom-2-[2'-(1H-tetrazol-5-yl)phenyl]-5-ylmethyl-benzofuran}-N-caproyl-valin-ethylester;
N-{3-Brom-2-[2'-(1H-tetrazol-5-yl)phenyl]-5-ylmethyl-benzofuran}-N-valeroyl-alaninethylester;
N-{3-Brom-2-[2'-(1H-tetrazol-5-yl)phenyl]-5-ylmethyl-benzofuran}-N-valeroyl-1-aminomethyl-cyclopentan-1-carbonsäure;
N-{3-Brom-2-[2'-(1H-tetrazol-5-yl)phenyl]-5-ylmethyl-benzofuran}-N-butyryl-1-aminomethyl-cyclopentan-1-carbonsäure;
N-{3-Brom-2-[2'-(1H-tetrazol-5-yl)phenyl]-5-ylmethyl-benzofuran}-N-caproyl-1-aminomethyl-cyclopentan-1-carbonsäure;
N-{3-Brom-2-[2'-(1H-tetrazol-5-yl)phenyl]-5-ylmethyl-benzofuran}-N-valeroyl-valin;
N-{3-Brom-2-[2'-(1H-tetrazol-5-yl)phenyl]-5-ylmethyl-benzofuran}-N-butyryl-valin;
N-{3-Brom-2-[2'-(1H-tetrazol-5-yl)phenyl]-5-ylmethyl-benzofuran}-N-caproyl-valin; und
N-{3-Brom-2-[2'-(1H-tetrazol-5-yl)phenyl]-5-ylmethyl-benzofuran}-N-valeroyl-alanin; oder eines Salz davon.

17. Verfahren nach Anspruch 1 zur Herstellung einer Verbindung der Formel I ausgewählt aus
N-{3-Brom-2-[2'-(1H-tetrazol-5-yl)-phenyl]-benzofuran-5-ylmethyl}-N-valeroyl-1-aminomethyl-cyclopentan-1-carbonsäure-ethylester, N-{3-Brom-2-[2'-(1H-tetrazol-5-yl)-phenyl]-benzofuran-5-ylmethyl}-N-butyryl-1-aminomethyl-cyclopentan-1-carbonsäure-ethylester;
N-{3-Brom-2-[2'-(1H-tetrazol-5-yl)-phenyl]-benzofuran-5-ylmethyl}-N-caproyl-1-aminomethyl-cyclopentan-1-carbonsäure-ethylester;
N-{3-Brom-2-[2'-(1H-tetrazol-5-yl)-phenyl]-benzofuran-5-ylmethyl}-N-valeroyl-valin-ethylester;
N-{3-Brom-2-[2'-(1H-tetrazol-5-yl)-phenyl]-benzofuran-5-ylmethyl}-N-butyryl-valin-ethylester;
N-{3-Brom-2-[2'-(1H-tetrazol-5-yl)-phenyl]-benzofuran-5-ylmethyl}-N-caproyl-valin-ethylester;
N-{3-Brom-2-[2'-(1H-tetrazol-5-yl)-phenyl]-benzofuran-5-ylmethyl}-N-valeroyl-alaninethylester;
N-{3-Brom-2-[2'-(1H-tetrazol-5-yl)-phenyl]-benzofuran-5-ylmethyl}-N-valeroyl-1-aminomethyl-cyclopentan-1-carbonsäure,
N-{3-Brom-2-[2'-(1H-tetrazol-5-yl)-phenyl]-benzofuran-5-ylmethyl}-N-butyryl-1-aminomethyl-cyclopentan-1-carbonsäure;
N-{3-Brom-2-[2'-(1H-tetrazol-5-yl)-phenyl]-benzofuran-5-ylmethyl}-N-caproyl-1-aminomethyl-cyclopentan-1-carbonsäure;
N-{3-Brom-2-[2'-(1H-tetrazol-5-yl)-phenyl]-benzofuran-5-ylmethyl}-N-valeroyl-valin;
N-{3-Brom-2-[2'-(1H-tetrazol-5-yl)-phenyl]-benzofuran-5-ylmethyl}-N-butyryl-valin;
N-{3-Brom-2-[2'-(1H-tetrazol-5-yl)-phenyl]-benzofuran-5-ylmethyl}-N-caproyl-valin; und
N-{3-Brom-2-[2'-(1H-tetrazol-5-yl)-phenyl]-benzofuran-5-ylmethyl}-N-valeroyl-alanin oder eines Salz davon.

18. Verfahren zur Herstellung eines pharmazeutischen Präparats, dadurch gekennzeichnet, dass man eine Verbindung gemäss einem der Ansprüche 1-18 oder ein pharmazeutisch verwendbares Salz davon gegebenenfalls mit pharmazeutisch verwendbaren Hilfsund/oder Trägerstoffen vermischt.

19. Verwendung einer Verbindung gemäss einem der Ansprüche 1-17 oder eines pharmazeutisch verwendbaren Salzes davon, zur Herstellung eines pharmazeutischen Präparats zur Behandlung von Bluthochdruck und Herzinsuffizienz sowie von Glaukom.

## Claims (Claims for the following Contracting State(s): AT, BE, CH, DE, DK, FR, GB, IE, IT, LI, LU, NL, PT, SE)

1. A compound of the formula 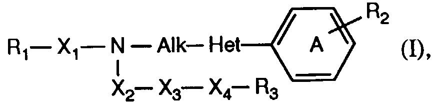 wherein
R₁ is C₁-C₇alkyl that is unsubstituted or substituted by halogen or by hydroxy, or is C₂-C₇alkenyl, C₃-C₇cycloalkyl, C₃-C₇cycloalkoxy, C₁-C₇alkoxy or C₃-C₇cycloalkyl-C₁-C₇alkoxy;
R₂ is 1H-tetrazol-5-yl, carboxy, C₁-C₇alkoxycarbonyl, SO₃H, PO₂H₂, PO₃H₂ or halo- C₁-C₇alkanesulfonylanlino;
R₃ is 1H-tetrazol-5-yl, hydroxymethyl, C₁-C₇alkoxymethyl, formyl, carboxy, C₁-C₇alkoxycarbonyl, C₁-C₇alkoxy-C₁-C₇alkoxycarbonyl, phenyl-C₁-C₄alkoxycarbonyl or carbamoyl, the amino group of which is unsubstituted or mono-substituted by C₁-C₇alkyl, C₃-C₇alkenyl or by phenyl-C₁-C₇alkyl or di-substituted by C₁-C₇alkyl, C₃-C₇alkenyl or by phenyl-C₁-C₇alkyl independently of one another, or is di-substituted by C₂-C₇alkylene or by C₂-C₄alkyleneoxy-C₂-C₄alkylene;
Alk is methylene, ethylene or ethylidene;
Het is 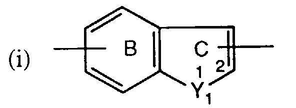 wherein Y₁ is O, S or N(R) and R is hydrogen or C₁-C₇alkyl; or 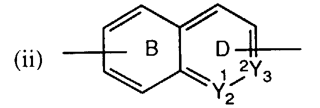 wherein one of the variables Y₂ and Y₃ is C(R') and the other is N or each of the variables is C(R'); and R' is hydrogen, halogen, C₁-C₇alkyl, C₁-C₇alkoxy, C₂-C₇alkenyloxy, phenoxy, benzyloxy, trifluoromethyl or S(O)ₘ-R, wherein m is 0, 1 or 2; and R is hydrogen or C₁-C₇alkyl;
X₁ is -CO- or -S(O)ₘ- and the index m is 0, 1 or 2;
one of the variables X₂ and X₄ is C₁-C₄alkylene and the other of the variables X₂ and X₄ is a bond; or each of the variables X₂ and X₄ is a bond;
X₃ is C₃-C₇cycloalkylidene or the structural element -C(Xₐ)(X_{b})- and Xₐ is hydrogen or C₁-C₇alkyl and X_{b} is C₁-C₇alkyl;
and the rings A, B, C and D, with the exception of the substituents indicated in the formula, and also aromatic substituents are, independently of one another, unsubstituted or mono- or poly-substituted by substituents selected from the group consisting of halogen, C₁-C₇alkyl, C₁-C₇alkoxy, C₂-C₇alkenyloxy, phenoxy, benzyloxy, trifluoromethyl and S(O)ₘ-R, wherein m is 0, 1 or 2 and R is hydrogen or C₁-C₇alkyl;
or a salt thereof.

2. A compound according to claim 1 of formula I, wherein
R₁ is C₁-C₇alkyl that is unsubstituted or substituted by halogen or by hydroxy, or is C₂-C₇alkenyl, C₃-C₇cycloalkyl or C₁-C₇alkoxy; or a salt thereof.

3. A compound according to claim 1 of formula I, wherein
R₁ is C₂-C₇alkyl or is C₁-C₇alkyl that is substituted by halogen or by hydroxy, or is C₃-C₇alkenyl, C₃-C₆cycloalkyl or C₁-C₇alkoxy;
R₂ is 1H-tetrazol-5-yl, carboxy, C₁-C₄alkoxycarbonyl or halo-C₁-C₄alkanesulfonylamino;
R₃ is 1H-tetrazol-5-yl, carboxy, C₁-C₄alkoxycarbonyl, C₁-C₄alkoxy-C₁-C₄alkoxycarbonyl, phenyl-C₁-C₄alkoxycarbonyl or carbamoyl, the amino group of which is mono-substituted by C₁-C₄alkyl or di-substituted by C₁-C₄alkyl groups which may be the same or different, or is di-substituted by C₄-C₆alkylene or by ethyleneoxyethylene; Alk is methylene, ethylene or ethylidene;
Het is 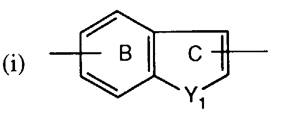 wherein Y₁ is O, S or N(R) and R is hydrogen or C₁-C₄alkyl; or 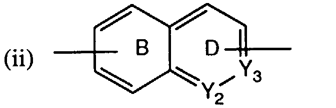 wherein one of the variables Y₂ and Y₃ is CH and the other is N or each of the variables is CH;
X₁ is -CO- or -S(O)ₘ- and the index m is 0, 1 or 2;
one of the variables X₂ and X₄ is C₁-C₄alkylene and the other of the variables X₂ and X₄ is a bond; or each of the variables X₂ and X₄ is a bond;
X₃ is C₃-C₆cycloalkylidene or the structural element -C(Xₐ)(X_{b})- and Xₐ is hydrogen or C₁-C₇alkyl and X_{b} is C₁-C₇alkyl;
and the rings A, B, C and D, with the exception of the substituents indicated in the formula, and also aromatic substituents are, independently of one another, unsubstituted or mono- or poly-substituted by substituents selected from the group consisting of halogen, C₁-C₄alkyl, C₁-C₄alkoxy, C₃-C₅alkenyloxy, phenoxy, benzyloxy, trifluoromethyl and S(O)ₘ-R, wherein m is 0, 1 or 2 and R is hydrogen or C₁-C₄alkyl;
or a salt thereof.

4. A compound according to claim 2 or claim 3 of formula I, wherein
R₁ is C₂-C₇alkyl or is C₁-C₄alkyl that is substituted by halogen or by hydroxy, or is C₃-C₇alkenyl, C₃-C₆cycloalkyl or C₁-C₇alkoxy;
R₂ is 1H-tetrazol-5-yl, carboxy or C₁-C₄alkoxycarbonyl;
R₃ is 1H-tetrazol-5-yl, carboxy, C₁-C₄alkoxycarbonyl, C₁-C₄alkoxy-C₁-C₄alkoxycarbonyl or phenyl-C₁-C₂alkoxycarbonyl;
Alk is methylene, also ethylene or ethylidene;
Het is 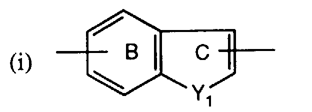 wherein Y₁ is O, S or NH; or 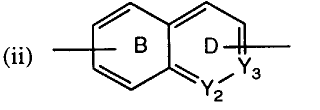 wherein one of the variables Y₂ and Y₃ is CH and the other is N or each of the variables is CH;
especially 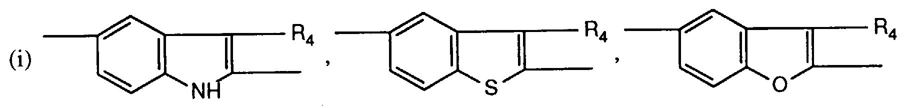 and R₄ is hydrogen, halogen, such as bromine, also C₁-C₇alkyl, C₁-C₇alkoxy or trifluoromethyl; or 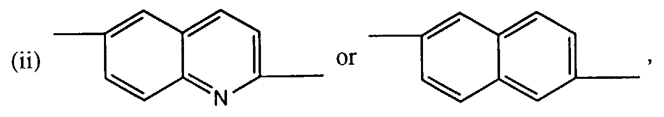 X₁ is -CO-;
one of the variables X₂ and X₄ is C₁-C₂alkylene and the other of the variables X₂ and X₄ is a bond; or each of the variables X₂ and X₄ is a bond;
X₃ is C₅-C₆cycloalkylidene or the structural element -C(Xₐ)(X_{b})- and Xₐ is hydrogen or C₁-C₅alkyl and X_{b} is C₁-C₅alkyl;
especially
(i) X₂ is C₁-C₂alkylene; X₄ is a bond; X₃ is C₅-C₆cycloalkylidene; or
(ii) each of X₂ and X₄ is a bond; and X₃ is the structural element -C(Xₐ)(X_{b})- and Xₐ is hydrogen or C₁-C₅alkyl and X_{b} is C₁-C₅alkyl;
and the rings A, B, C and D, with the exception of the substituents indicated in the formula, are, independently of one another, unsubstituted or mono- or poly-substituted by substituents selected from the group consisting of halogen, C₁-C₄alkyl, C₁-C₄alkoxy and trifluoromethyl;
or a salt thereof.

5. A compound according to claim 1 of formula I, wherein
one of the variables Y₂ and Y₃ is C(R') and the other is N or C(R'), wherein R' is halogen, C₁-C₄alkyl, C₁-C₄alkoxy or trifluoromethyl, or a salt thereof.

6. A compound according to claim 1 of formula I, wherein
R₁ is C₂-C₇alkyl, such as n-propyl or n-butyl, or C₃-C₆cycloalkyl, such as cyclopropyl, or C₁-C₄alkoxy, such as methoxy, ethoxy, propoxy or butoxy;
R₂ is 1H-tetrazol-5-yl, carboxy or C₁-C₄alkoxycarbonyl, such as methoxy- or ethoxycarbonyl;
R₃ is carboxy or C₁-C₄alkoxycarbonyl, such as methoxy-, ethoxy- or tert-butoxy-carbonyl; Alk is methylene;
Het is 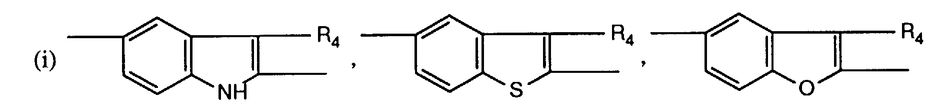 and R₄ is hydrogen, halogen, such as bromine, also C₁-C₄alkyl, such as methyl, C₁-C₄alkoxy, such as methoxy, or trifluoromethyl; or 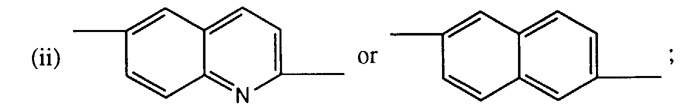 X₁ is -CO-;
(i) X₂ is C₁-C₂alkylene, especially methylene; X₄ is a bond; X₃ is C₅-C₆cycloalkylidene; or
(ii) each of X₂ and X₄ is a bond; and X₃ is the structural element -C(Xₐ)(X_{b})- and Xₐ is hydrogen or C₁-C₅alkyl, such as ethyl, and X_{b} is C₁-C₅alkyl, such as ethyl or isopropyl; or a salt thereof.

7. A compound according to any one of claims 2 to 5 of formula I, wherein
R₃ is hydroxymethyl, C₁-C₄alkoxymethyl or formyl; the other variables are as defined above in each case.

8. A compound according to claim 1 of formula I, wherein
R₁ is C₂-C₅alkyl, such as n-propyl or n-butyl;
R₂ is 1H-tetrazol-5-yl or carboxy;
R₃ is carboxy or C₁-C₄alkoxycarbonyl, such as methoxy-, ethoxy- or tert-butoxy-carbonyl; Alk is methylene;
Het is 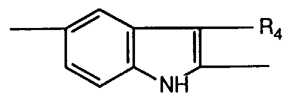 and R₄ is hydrogen, halogen, such as bromine, or C₁-C₄alkyl, such as methyl;
X₁ is -CO-;
(i) X₂ is C₁-C₂alkylene, especially methylene; X₄ is a bond; X₃ is C₅-C₆cycloalkylidene; or
(ii) each of X₂ and X₄ is a bond; and X₃ is the structural element -C(Xₐ)(X_{b})- and Xₐ is hydrogen or C₁-C₅alkyl, such as ethyl, and X_{b} is C₁-C₅alkyl, such as ethyl, isopropyl or 3-butyl;
or a salt thereof.

9. A compound according to claim 1 of formula I, wherein
R₁ is C₂-C₅alkyl, such as n-propyl or n-butyl;
R₂ is 1H-tetrazol-5-yl or carboxy;
R₃ is carboxy or C₁-C₄alkoxycarbonyl, such as methoxy-, ethoxy- or tert-butoxy-carbonyl; Alk is methylene;
Het is 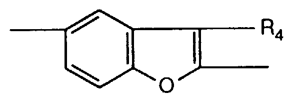 and R₄ is hydrogen, halogen, such as bromine, or C₁-C₄alkyl, such as methyl;
X₁ is -CO-;
(i) X₂ is C₁-C₂alkylene, especially methylene; X₄ is a bond; X₃ is C₅-C₆cycloalkylidene; or
(ii) each of X₂ and X₄ is a bond; and X₃ is the structural element -C(Xₐ)(X_{b})- and Xₐ is hydrogen or C₁-C₅alkyl, such as ethyl, and X_{b} is C₁-C₅alkyl, such as ethyl, isopropyl or 3-butyl;
or a salt thereof.

10. A compound according to claim 1 of formula I, wherein
R₁ is C₂-C₅alkyl, such as n-propyl or n-butyl;
R₂ is 1H-tetrazol-5-yl or carboxy;
R₃ is carboxy or C₁-C₄alkoxycarbonyl, such as methoxy-, ethoxy- or tert-butoxy-carbonyl; Alk is methylene;
Het is 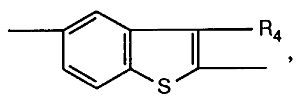 wherein R₄ is hydrogen, halogen, such as bromine, or C₁-C₄alkyl, such as methyl;
X₁ is -CO-;
(i) X₂ is C₁-C₂alkylene, especially methylene; X₄ is a bond; X₃ is C₅-C₆cycloalkylidene, such as cyclopentylidene or cyclohexylidene; or
(ii) each of X₂ and X₄ is a bond; and X₃ is the structural element -C(Xₐ)(X_{b})- and Xₐ is hydrogen or C₁-C₅alkyl, such as ethyl, and X_{b} is C₁-C₅alkyl, such as ethyl, isopropyl or 3-butyl;
or a salt thereof.

11. A compound according to claim 1 of formula I, wherein
R₁ is C₂-C₅alkyl, such as n-propyl or n-butyl;
R₂ is 1H-tetrazol-5-yl or carboxy;
R₃ is carboxy or C₁-C₄alkoxycarbonyl, such as methoxy-, ethoxy- or tert-butoxy-carbonyl; Alk is methylene; 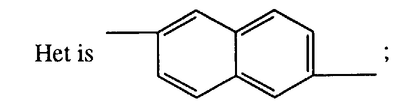 X₁ is -CO-;
(i) X₂ is C₁-C₂alkylene, especially methylene; X₄ is a bond; X₃ is C₅-C₆cycloalkylidene; or
(ii) each of X₂ and X₄ is a bond; and X₃ is the structural element -C(Xₐ)(X_{b})- and Xₐ is hydrogen or C₁-C₅alkyl, such as ethyl, and X_{b} is C₁-C₅alkyl, such as ethyl, isopropyl or 3-butyl;
or a salt thereof.

12. A compound according to claim 1 of formula I, wherein
R₁ is C₂-C₅alkyl, such as n-propyl or n-butyl;
R₂ is 1H-tetrazol-5-yl or carboxy;
R₃ is carboxy or C₁-C₄alkoxycarbonyl, such as methoxy-, ethoxy- or tert-butoxy-carbonyl; Alk is methylene;
Het is 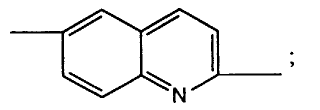 X₁ is -CO-;
(i) X₂ is C₁-C₂alkylene, especially methylene; X₄ is a bond; X₃ is C₅-C₆cycloalkylidene; or
(ii) each of X₂ and X₄ is a bond; and X₃ is the structural element -C(Xₐ)(X_{b})- and Xₐ is hydrogen or C₁-C₅alkyl, such as ethyl, and X_{b} is C₁-C₅alkyl, such as ethyl, isopropyl or 3-butyl;
or a salt thereof.

13. A compound according to claim 1 of formula I, wherein
R₁ is C₂-C₅alkyl, such as ethyl, n-propyl or n-butyl;
R₂ is 1H-tetrazol-5-yl or carboxy;
R₃ is carboxy or C₁-C₄alkoxycarbonyl, such as methoxy-, ethoxy- or tert-butoxy-carbonyl; Alk is methylene;
Het is 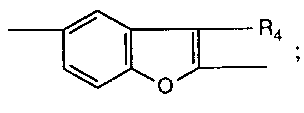 and R₄ is hydrogen or halogen having an atomic number of up to and including 35, such as bromine;
X₁ is -CO-;
(i) X₂ is C₁-C₂alkylene, especially methylene; X₄ is a bond; X₃ is C₅-C₆cycloalkylidene; or
(ii) each of X₂ and X₄ is a bond; and X₃ is the structural element -C(Xₐ)(X_{b})- and Xₐ is hydrogen or C₁-C₅alkyl, such as ethyl, and X_{b} is C₁-C₅alkyl, such as ethyl, isopropyl or 3-butyl;
or a salt thereof.

14. A compound according to claim 1 selected from
N-[2-(2'-(1H-tetrazol-5-yl)-phenyl)-quinolin-6-ylmethyl]-N-valeroylvaline;
N-[2-(2'-(1H-tetrazol-5-yl)-phenyl)-quinolin-6-ylmethyl]-N-valeroyl-1-aminomethylcyclopentane-1-carboxylic acid;
N-[2-(2'-(1H-tetrazol-5-yl)-phenyl)-quinolin-6-ylmethyl]-N-valeroyl-2-aminomethyl-2-ethylbutyric acid;
N-[2-(2'-(1H-tetrazol-5-yl)-phenyl)-benzo[b]thiophen-5-ylmethyl]-N-valeroyl-2-aminomethyl-2-ethylbutyric acid;
N-[3-bromo-2-(2'-carboxyphenyl)-benzo[b]thiophen-5-ylmethyl]-N-valeroylvaline;
N-[2-(2'-(1H-tetrazol-5-yl)-phenyl)-benzofuran-5-ylmethyl]-N-valeroyl-2-aminomethyl-2-ethylbutyric acid;
N-[2-(2'-(1H-tetrazol-5-yl)-phenyl)-benzofuran-5-ylmethyl]-N-valeroyl-1-aminomethylcyclopentane-1-carboxylic acid;
N-[2-(2'-(1H-tetrazol-5-yl)-phenyl)-benzofuran-5-ylmethyl]-N-valeroylvaline;
N-[2-(2'-(1H-tetrazol-5-yl)-phenyl)-5-ylmethylbenzo[b]thiophene]-N-valeroyl-1-aminomethylcyclopentane-1-carboxylic acid;
N-[2-(2'-(1H-tetrazol-5-yl)-phenyl)-benzo[b]thiophen-5-ylmethyl]-N-valeroylvaline;
N-[3-bromo-2-(2'-carboxyphenyl)-benzo[b]thiophen-5-ylmethyl]-N-valeroyl-1-aminomethylcyclopentane-1-carboxylic acid; and
N-[2-(2'-(1H-tetrazol-5-yl)-phenyl)-benzofuran-5-ylmethyl]-N-valeroyl-1-aminomethylcyclohexane-1-carboxylic acid;
N-[2-(2'-(tetrazol-5-yl)-phenyl)-naphthalen-6-ylmethyl]-N-valeroylvaline;
N-[2-(2'-(tetrazol-5-yl)-phenyl)-naphthalen-6-ylmethyl]-N-valeroyl-1-aminomethylcyclopentane-1-carboxylic acid;
N-[2-(2'-(tetrazol-5-yl)-phenyl)-naphthalen-6-ylmethyl]-N-valeroyl-2-aminomethyl-2-ethylbutyric acid;
N-[2-(2'-(tetrazol-5-yl)-phenyl)-naphthalen-6-ylmethyl]-N-valeroyl-1-aminomethylcyclohexane-1-carboxylic acid;
N-[2-(2'-(tetrazol-5-yl)-phenyl)-quinolin-6-ylmethyl]-N-cyclopropylcarbonylvaline;
N-[2-(2'-(tetrazol-5-yl)-phenyl)-quinolin-6-ylmethyl]-N-cyclopropylcarbonyl-1-aminomethylcyclopentane-1-carboxylic acid;
N-[2-(2'-(tetrazol-5-yl)-phenyl)-quinolin-6-ylmethyl]-N-cyclopropylcarbonyl-2-aminomethyl-2-ethylbutyric acid;
N-[2-(2'-(tetrazol-5-yl)-phenyl)-quinolin-6-ylmethyl]-N-cyclopropylcarbonylvaline; or a salt thereof.

15. A compound according to claim 1 selected from
N-[2-(2'-(1H-tetrazol-5-yl)-phenyl)-naphthalen-6-ylmethyl]-N-valeroyl-1-aminomethylcyclopentane-1-carboxylic acid ethyl ester;
N-[2-(2'-(1H-tetrazol-5-yl)-phenyl)-naphthalen-6-ylmethyl]-N-valeroyl-1-aminomethylcyclopentane-1-carboxylic acid;
N-[2-(2'-(1H-tetrazol-5-yl)-phenyl)-naphthalen-6-ylmethyl]-N-valeroyl-3-amino-2,2-dimethylpropionic acid;
N-[2-(2'-(1H-tetrazol-5-yl)-phenyl)-naphthalen-6-ylmethyl]-N-butyrylvaline; and
N-[2-(2'-(1H-tetrazol-5-yl)-phenyl)-naphthalen-6-ylmethyl]-N-valeroylvaline; or a salt thereof.

16. A compound according to claim 1 selected from
N-{3-bromo-2-[2'-(1H-tetrazol-5-yl)-phenyl]-5-ylmethylbenzofuran}-N-valeroyl-1-aminomethylcyclopentane-1-carboxylic acid ethyl ester;
N-{3-bromo-2-[2'-(1H-tetrazol-5-yl)-phenyl]-5-ylmethylbenzofuran}-N-butyryl-1-aminomethylcyclopentane-1-carboxylic acid ethyl ester;
N-{3-bromo-2-[2'-(1H-tetrazol-5-yl)-phenyl]-5-ylmethylbenzofuran}-N-caproyl-1-aminomethylcyclopentane-1-carboxylic acid ethyl ester;
N-{3-bromo-2-[2'-(1H-tetrazol-5-yl)-phenyl]-5-ylmethylbenzofuran}-N-valeroylvaline ethyl ester;
N-{3-bromo-2-[2'-(1H-tetrazol-5-yl)-phenyl]-5-ylmethylbenzofuran}-N-butyrylvaline ethyl ester;
N-{3-bromo-2-[2'-(1H-tetrazol-5-yl)-phenyl]-5-ylmethylbenzofuran}-N-caproylvaline ethyl ester;
N-{3-bromo-2-[2'-(1H-tetrazol-5-yl)-phenyl]-5-ylmethylbenzofuran}-N-valeroylalanine ethyl ester;
N-{3-bromo-2-[2'-(1H-tetrazol-5-yl)-phenyl]-5-ylmethylbenzofuran}-N-valeroyl-1-aminomethylcyclopentane-1-carboxylic acid;
N-{3-bromo-2-[2'-(1H-tetrazol-5-yl)-phenyl]-5-ylmethylbenzofuran}-N-butyryl-1-aminomethylcyclopentane-1-carboxylic acid;
N-{3-bromo-2-[2'-(1H-tetrazol-5-yl)-phenyl]-5-ylmethylbenzofuran}-N-caproyl-1-aminomethylcyclopentane-1-carboxylic acid;
N-{3-bromo-2-[2'-(1H-tetrazol-5-yl)-phenyl]-5-ylmethylbenzofuran}-N-valeroylvaline;
N-{3-bromo-2-[2'-(1H-tetrazol-5-yl)-phenyl]-5-ylmethylbenzofuran}-N-butyrylvaline;
N-{3-bromo-2-[2'-(1H-tetrazol-5-yl)-phenyl]-5-ylmethylbenzofuran}-N-caproylvaline; and
N-{3-bromo-2-[2'-(1H-tetrazol-5-yl)-phenyl]-5-ylmethylbenzofuran}-N-valeroylalanine; or a salt thereof.

17. A compound according to claim 1 selected from
N-{3-bromo-2-[2'-(1H-tetrazol-5-yl)-phenyl]-benzofuran-5-ylmethyl}-N-valeroyl-1-aminomethylcyclopentane-1-carboxylic acid ethyl ester,
N-{3-bromo-2-[2'-(1H-tetrazol-5-yl)-phenyl]-benzofuran-5-ylmethyl}-N-butyryl-1-aminomethylcyclopentane-1-carboxylic acid ethyl ester;
N-{3-bromo-2-[2'-(1H-tetrazol-5-yl)-phenyl]-benzofuran-5-ylmethyl}-N-caproyl-1-aminomethylcyclopentane-1-carboxylic acid ethyl ester;
N-{3-bromo-2-[2'-(1H-tetrazol-5-yl)-phenyl]-benzofuran-5-ylmethyl}-N-valeroylvaline ethyl ester;
N-{3-bromo-2-[2'-(1H-tetrazol-5-yl)-phenyl]-benzofuran-5-ylmethyl}-N-butyrylvaline ethyl ester;
N-{3-bromo-2-[2'-(1H-tetrazol-5-yl)-phenyl]-benzofuran-5-ylmethyl}-N-caproylvaline ethyl ester;
N-{3-bromo-2-[2'-(1H-tetrazol-5-yl)-phenyl]-benzofuran-5-ylmethyl}-N-valeroylalanine ethyl ester;
N-{3-bromo-2-[2'-(1H-tetrazol-5-yl)-phenyl]-benzofuran-5-ylmethyl}-N-valeroyl-1-aminomethylcyclopentane-1-carboxylic acid,
N-{3-bromo-2-[2'-(1H-tetrazol-5-yl)-phenyl]-benzofuran-5-ylmethyl}-N-butyryl-1-aminomethylcyclopentane-1-carboxylic acid;
N-{3-bromo-2-[2'-(1H-tetrazol-5-yl)-phenyl]-benzofuran-5-ylmethyl}-N-caproyl-1-aminomethylcyclopentane-1-carboxylic acid;
N-{3-bromo-2-[2'-(1H-tetrazol-5-yl)-phenyl]-benzofuran-5-ylmethyl}-N-valeroylvaline;
N-{3-bromo-2-[2'-(1H-tetrazol-5-yl)-phenyl]-benzofuran-5-ylmethyl}-N-butyrylvaline;
N-{3-bromo-2-[2'-(1H-tetrazol-5-yl)-phenyl]-benzofuran-5-ylmethyl}-N-caproylvaline and
N-{3-bromo-2-[2'-(1H-tetrazol-5-yl)-phenyl]-benzofuran-5-ylmethyl}-N-valeroylalanine; or a salt thereof.

18. A compound according to any one of claims 1 to 17 for use in a method for the therapeutic treatment of the animal or human body.

19. A process for the preparation of a compound of formula I or a salt thereof according to any one of claims 1 to 17, wherein
a) in a compound of the formula 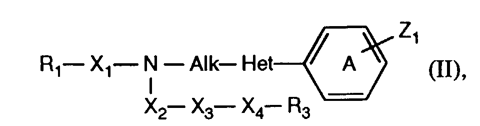 wherein Z₁ is a radical that can be converted into R₂, or in a salt thereof, Z₁ is converted into R₂; or
b) a compound of the formula 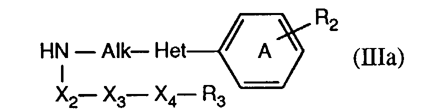 is reacted with a compound of the formula R₁-X₁-OH (IIIb), a reactive derivative thereof or a salt thereof;
and, in each case, if desired, a compound I obtainable according to the process or by another method, in free form or in salt form, is isolated, a compound I obtainable according to the process or by another method is converted into a different compound I, a mixture of isomers obtainable according to the process is separated and the desired isomer is isolated and/or a free compound I obtainable according to the process is converted into a salt, or a salt, obtainable according to the process, of a compound I is converted into the free compound I or into a different salt.

20. A pharmaceutical composition comprising as active ingredient a compound according to any one of claims 1 to 18, in free form or in the form of a pharmaceutically acceptable salt, if desired together with customary excipients.

21. The use of a compound according to any one of claims 1 to 18, in free form or in the form of a pharmaceutically acceptable salt, for the preparation of a pharmaceutical composition for the treatment of high blood pressure and cardiac insufficiency and also glaucoma.

## Claims (Claims for the following Contracting State(s): ES, GR)

1. A process for the preparation of a compound of the formula 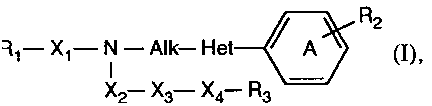 wherein
R₁ is C₁-C₇alkyl that is unsubstituted or substituted by halogen or by hydroxy, or is C₂-C₇alkenyl, C₃-C₇cycloalkyl, C₃-C₇cycloalkoxy, C₁-C₇alkoxy or C₃-C₇cycloalkyl-C₁-C₇alkoxy;
R₂ is 1H-tetrazol-5-yl, carboxy, C₁-C₇alkoxycarbonyl, SO₃H, PO₂H₂, PO₃H₂ or halo- C₁-C₇alkanesulfonylamino;
R₃ is 1H-tetrazol-5-yl, hydroxymethyl, C₁-C₇alkoxymethyl, formyl, carboxy, C₁-C₇alkoxycarbonyl, C₁-C₇alkoxy-C₁-C₇alkoxycarbonyl, phenyl-C₁-C₄alkoxycarbonyl or carbamoyl, the amino group of which is unsubstituted or mono-substituted by C₁-C₇alkyl, C₃-C₇alkenyl or by phenyl-C₁-C₇alkyl or di-substituted by C₁-C₇alkyl, C₃-C₇alkenyl or by phenyl-C₁-C₇alkyl independently of one another, or is di-substituted by C₂-C₇alkylene or by C₂-C₄alkyleneoxy-C₂-C₄alkylene;
Alk is methylene, ethylene or ethylidene;
Het is 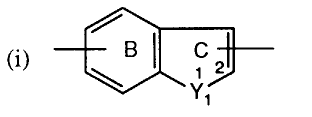 wherein Y₁ is O, S or N(R) and R is hydrogen or C₁-C₇alkyl; or 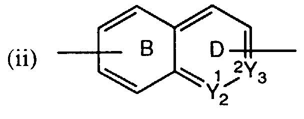 wherein one of the variables Y₂ and Y₃ is C(R') and the other is N or each of the variables is C(R'); and R' is hydrogen, halogen, C₁-C₇alkyl, C₁-C₇alkoxy, C₂-C₇alkenyloxy, phenoxy, benzyloxy, trifluoromethyl or S(O)ₘ-R, wherein m is 0, 1 or 2; and R is hydrogen or C₁-C₇alkyl;
X₁ is -CO- or -S(O)ₘ- and the index m is 0, 1 or 2;
one of the variables X₂ and X₄ is C₁-C₄alkylene and the other of the variables X₂ and X₄ is a bond; or each of the variables X₂ and X₄ is a bond;
X₃ is C₃-C₇cycloalkylidene or the structural element -C(Xₐ)(X_{b})- and Xₐ is hydrogen or C₁-C₇alkyl and X_{b} is C₁-C₇alkyl;
and the rings A, B, C and D, with the exception of the substituents indicated in the formula, and also aromatic substituents are, independently of one another, unsubstituted or mono- or poly-substituted by substituents selected from the group consisting of halogen, C₁-C₇alkyl, C₁-C₇alkoxy, C₂-C₇alkenyloxy, phenoxy, benzyloxy, trifluoromethyl and S(O)ₘ-R, wherein m is 0, 1 or 2 and R is hydrogen or C₁-C₇alkyl;
or of a salt thereof; wherein
a) in a compound of the formula 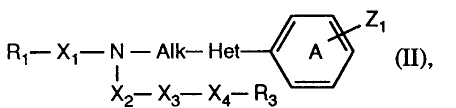 wherein Z₁ is a radical that can be converted into R₂, or in a salt thereof, Z₁ is converted into R₂; or
b) a compound of the formula 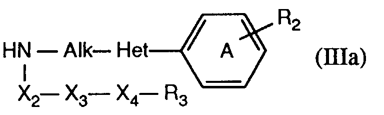 is reacted with a compound of the formula R₁-X₁-OH (IIIb), a reactive derivative thereof or a salt thereof;
and, in each case, if desired, a compound I obtainable according to the process or by another method, in free form or in salt form, is isolated, a compound I obtainable according to the process or by another method is converted into a different compound I, a mixture of isomers obtainable according to the process is separated and the desired isomer is isolated and/or a free compound I obtainable according to the process is converted into a salt, or a salt, obtainable according to the process, of a compound I is converted into the free compound I or into a different salt.

2. A process according to claim 1 for the preparation of a compound of formula I, wherein R₁ is C₁-C₇alkyl that is unsubstituted or substituted by halogen or by hydroxy, or is C₂-C₇alkenyl, C₃-C₇cycloalkyl or C₁-C₇alkoxy; or of a salt thereof.

3. A process according to claim 1 for the preparation of a compound of formula I, wherein
R₁ is C₂-C₇alkyl or is C₁-C₇alkyl that is substituted by halogen or by hydroxy, or is C₃-C₇alkenyl, C₃-C₆cycloalkyl or C₁-C₇alkoxy;
R₂ is 1H-tetrazol-5-yl, carboxy, C₁-C₄alkoxycarbonyl or halo-C₁-C₄alkanesulfonylamino; R₃ is 1H-tetrazol-5-yl, carboxy, C₁-C₄alkoxycarbonyl, C₁-C₄alkoxy-C₁-C₄alkoxycarbonyl, phenyl-C₁-C₄alkoxycarbonyl or carbamoyl, the amino group of which is mono-substituted by C₁-C₄alkyl or di-substituted by C₁-C₄alkyl groups which may be the same or different, or is di-substituted by C₄-C₆alkylene or by ethyleneoxyethylene;
Alk is methylene, ethylene or ethylidene;
Het is 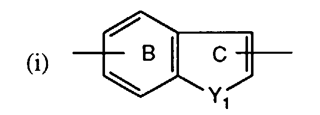 wherein Y₁ is O, S or N(R) and R is hydrogen or C₁-C₄alkyl; or 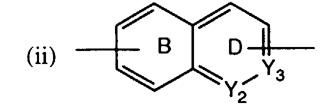 wherein one of the variables Y₂ and Y₃ is CH and the other is N or each of the variables is CH;
X₁ is -CO- or -S(O)ₘ- and the index m is 0, 1 or 2;
one of the variables X₂ and X₄ is C₁-C₄alkylene and the other of the variables X₂ and X₄ is a bond; or each of the variables X₂ and X₄ is a bond;
X₃ is C₃-C₆cycloalkylidene or the structural element -C(Xₐ)(X_{b})- and Xₐ is hydrogen or C₁-C₇alkyl and X_{b} is C₁-C₇alkyl;
and the rings A, B, C and D, with the exception of the substituents indicated in the formula, and also aromatic substituents are, independently of one another, unsubstituted or mono- or poly-substituted by substituents selected from the group consisting of halogen, C₁-C₄alkyl, C₁-C₄alkoxy, C₃-C₅alkenyloxy, phenoxy, benzyloxy, trifluoromethyl and S(O)ₘ-R, wherein m is 0, 1 or 2 and R is hydrogen or C₁-C₄alkyl;
or of a salt thereof.

4. A process according to claim 1 for the preparation of a compound of formula I, wherein R₁ is C₂-C₇alkyl or is C₁-C₄alkyl that is substituted by halogen or by hydroxy, or is C₃-C₇alkenyl, C₃-C₆cycloalkyl or C₁-C₇alkoxy;
R₂ is 1H-tetrazol-5-yl, carboxy or C₁-C₄alkoxycarbonyl;
R₃ is 1H-tetrazol-5-yl, carboxy, C₁-C₄alkoxycarbonyl, C₁-C₄alkoxy-C₁-C₄alkoxycarbonyl or phenyl-C₁-C₂alkoxycarbonyl;
Alk is methylene, also ethylene or ethylidene;
Het is 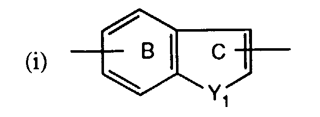 wherein Y₁ is O, S or NH; or 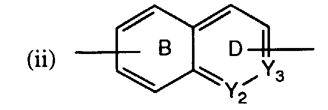 wherein one of the variables Y₂ and Y₃ is CH and the other is N or each of the variables is CH;
especially 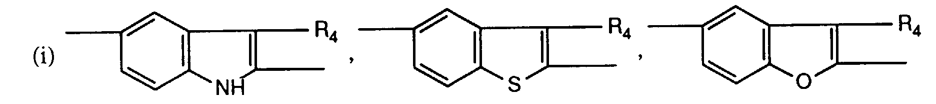 and R₄ is hydrogen, halogen, such as bromine, also C₁-C₇alkyl, C₁-C₇alkoxy or trifluoromethyl; or 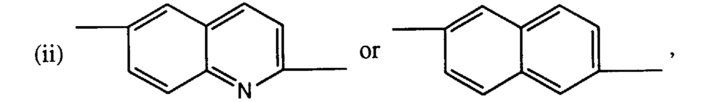 X₁ is -CO-;
one of the variables X₂ and X₄ is C₁-C₂akylene and the other of the variables X₂ and X₄ is a bond; or each of the variables X₂ and X₄ is a bond;
X₃ is C₅-C₆cycloalkylidene or the structural element -C(Xₐ)(X_{b})- and Xₐ is hydrogen or C₁-C₅alkyl and X_{b} is C₁-C₅alkyl;
especially
(i) X₂ is C₁-C₂alkylene; X₄ is a bond; X₃ is C₅-C₆cycloalkylidene; or
(ii) each of X₂ and X₄ is a bond; and X₃ is the structural element -C(Xₐ)(X_{b})- and Xₐ is hydrogen or C₁-C₅alkyl and X_{b} is C₁-C₅alkyl;
and the rings A, B, C and D, with the exception of the substituents indicated in the formula, are, independently of one another, unsubstituted or mono- or poly-substituted by substituents selected from the group consisting of halogen, C₁-C₄alkyl, C₁-C₄alkoxy and trifluoromethyl; one of the variables Y₂ and Y₃ is C(R') and the other is N or C(R'), wherein R' is halogen, C₁-C₄alkyl, C₁-C₄alkoxy or trifluoromethyl;
or of a salt thereof.

5. A process according to claim 1 for the preparation of a compound of formula I, wherein one of the variables Y₂ and Y₃ is C(R') and the other is N or C(R'), wherein R' is halogen, C₁-C₄alkyl, C₁-C₄alkoxy or trifluoromethyl, or of a salt thereof.

6. A process according to claim I for the preparation of a compound of formula I, wherein
R₁ is C₂-C₇alkyl, such as n-propyl or n-butyl, or C₃-C₆cycloalkyl, such as cyclopropyl, or C₁-C₄alkoxy, such as methoxy, ethoxy, propoxy or butoxy;
R₂ is 1H-tetrazol-5-yl, carboxy or C₁-C₄alkoxycarbonyl, such as methoxy- or ethoxycarbonyl;
R₃ is carboxy or C₁-C₄alkoxycarbonyl, such as methoxy-, ethoxy- or tert-butoxy-carbonyl; Alk is methylene;
Het is 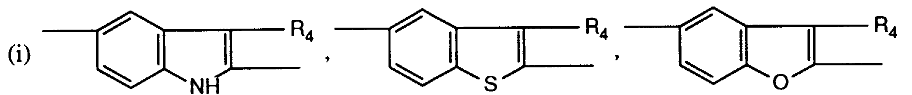 and R₄ is hydrogen, halogen, such as bromine, also C₁-C₄alkyl, such as methyl, C₁-C₄alkoxy, such as methoxy, or trifluoromethyl; or 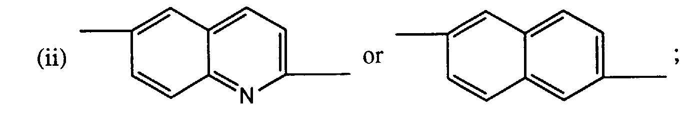 X₁ is -CO-;
(i) X₂ is C₁-C₂alkylene, especially methylene; X₄ is a bond; X₃ is C₅-C₆cycloalkylidene; or
(ii) each of X₂ and X₄ is a bond; and X₃ is the structural element -C(Xₐ)(X_{b})- and Xₐ is hydrogen or C₁-C₅alkyl, such as ethyl, and X_{b} is C₁-C₅alkyl, such as ethyl or isopropyl;
or of a salt thereof.

7. A process according to claim 1 for the preparation of a compound of formula I, wherein R₃ is hydroxymethyl, C₁-C₄alkoxymethyl or formyl; the other variables are as defined above in each case; or of a salt thereof.

8. A process according to claim 1 for the preparation of a compound of formula I, wherein
R₁ is C₂-C₅alkyl, such as n-propyl or n-butyl;
R₂ is 1H-tetrazol-5-yl or carboxy;
R₃ is carboxy or C₁-C₄alkoxycarbonyl, such as methoxy-, ethoxy- or tert-butoxy-carbonyl;
Alk is methylene;
Het is 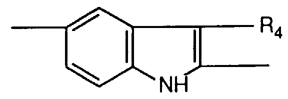 and R₄ is hydrogen, halogen, such as bromine, or C₁-C₄alkyl, such as methyl;
X₁ is -CO-;
(i) X₂ is C₁-C₂alkylene, especially methylene; X₄ is a bond; X₃ is C₅-C₆cycloalkylidene; or
(ii) each of X₂ and X₄ is a bond; and X₃ is the structural element -C(Xₐ)(X_{b})- and Xₐ is hydrogen or C₁-C₅alkyl, such as ethyl, and X_{b} is C₁-C₅alkyl, such as ethyl, isopropyl or 3-butyl;
or of a salt thereof.

9. A process according to claim 1 for the preparation of a compound of formula I, wherein
R₁ is C₂-C₅alkyl, such as n-propyl or n-butyl;
R₂ is 1H-tetrazol-5-yl or carboxy;
R₃ is carboxy or C₁-C₄alkoxycarbonyl, such as methoxy-, ethoxy- or tert-butoxy-carbonyl; Alk is methylene;
Het is 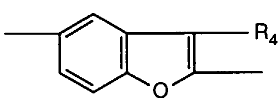 and R₄ is hydrogen, halogen, such as bromine, or C₁-C₄alkyl, such as methyl;
X₁ is -CO-;
(i) X₂ is C₁-C₂alkylene, especially methylene; X₄ is a bond; X₃ is C₅-C₆cycloalkylidene; or
(ii) each of X₂ and X₄ is a bond; and X₃ is the structural element -C(Xₐ)(X_{b})- and Xₐ is hydrogen or C₁-C₅alkyl, such as ethyl, and X_{b} is C₁-C₅alkyl, such as ethyl, isopropyl or 3-butyl;
or of a salt thereof.

10. A process according to claim 1 for the preparation of a compound of formula I, wherein
R₁ is C₂-C₅alkyl, such as n-propyl or n-butyl;
R₂ is 1H-tetrazol-5-yl or carboxy;
R₃ is carboxy or C₁-C₄alkoxycarbonyl, such as methoxy-, ethoxy- or tert-butoxy-carbonyl; Alk is methylene;
Het is 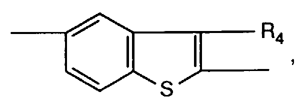 wherein R₄ is hydrogen, halogen, such as bromine, or C₁-C₄alkyl, such as methyl;
X₁ is -CO- ;
(i) X₂ is C₁-C₂alkylene, especially methylene; X₄ is a bond; X₃ is C₅-C₆cycloalkylidene, such as cyclopentylidene or cyclohexylidene; or
(ii) each of X₂ and X₄ is a bond; and X₃ is the structural element -C(Xₐ)(X_{b})- and Xₐ is hydrogen or C₁-C₅alkyl, such as ethyl, and X_{b} is C₁-C₅alkyl, such as ethyl, isopropyl or 3-butyl;
or of a salt thereof.

11. A process according to claim 1 for the preparation of a compound of formula I, wherein
R₁ is C₂-C₅alkyl, such as n-propyl or n-butyl;
R₂ is 1H-tetrazol-5-yl or carboxy;
R₃ is carboxy or C₁-C₄alkoxycarbonyl, such as methoxy-, ethoxy- or tert-butoxy-carbonyl;
Alk is methylene;
Het is 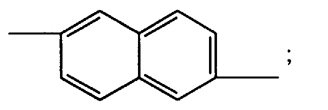 X₁ is -CO-;
(i) X₂ is C₁-C₂alkylene, especially methylene; X₄ is a bond; X₃ is C₅-C₆cycloalkylidene; or
(ii) each of X₂ and X₄ is a bond; and X₃ is the structural element -C(Xₐ)(X_{b})- and Xₐ is hydrogen or C₁-C₅alkyl, such as ethyl, and X_{b} is C₁-C₅alkyl, such as ethyl, isopropyl or 3-butyl;
or of a salt thereof.

12. A process according to claim 1 for the preparation of a compound of formula I, wherein
R₁ is C₂-C₅alkyl, such as n-propyl or n-butyl;
R₂ is 1H-tetrazol-5-yl or carboxy;
R₃ is carboxy or C₁-C₄alkoxycarbonyl, such as methoxy-, ethoxy- or tert-butoxy-carbonyl; Alk is methylene;
Het is 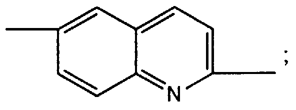 X₁ is -CO-;
(i) X₂ is C₁-C₂alkylene, especially methylene; X₄ is a bond; X₃ is C₅-C₆cycloalkylidene;
or
(ii) each of X₂ and X₄ is a bond; and X₃ is the structural element -C(Xₐ)(X_{b})- and Xₐ is hydrogen or C₁-C₅alkyl, such as ethyl, and X_{b} is C₁-C₅alkyl, such as ethyl, isopropyl or 3-butyl;
or of a salt thereof.

13. A process according to claim 1 for the preparation of a compound of formula I, wherein
R₁ is C₂-C₅alkyl, such as ethyl, n-propyl or n-butyl;
R₂ is 1H-tetrazol-5-yl or carboxy;
R₃ is carboxy or C₁-C₄alkoxycarbonyl, such as methoxy-, ethoxy- or tert-butoxy-carbonyl; Alk is methylene;
Het is 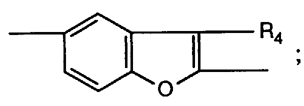 and R₄ is hydrogen or halogen having an atomic number of up to and including 35, such as bromine;
X₁ is -CO-;
(i) X₂ is C₁-C₂alkylene, especially methylene; X₄ is a bond; X₃ is C₅-C₆cycloalkylidene; or
(ii) each of X₂ and X₄ is a bond; and X₃ is the structural element -C(Xₐ)(X_{b})- and Xₐ is hydrogen or C₁-C₅alkyl, such as ethyl, and X_{b} is C₁-C₅alkyl, such as ethyl, isopropyl or 3-butyl;
or of a salt thereof.

14. A process according to claim 1 for the preparation of a compound of formula I selected from
N-[2-(2'-(1H-tetrazol-5-yl)-phenyl)-quinolin-6-ylmethyl]-N-valeroylvaline;
N-[2-(2'-(1H-tetrazol-5-yl)-phenyl)-quinolin-6-ylmethyl]-N-valeroyl-1-aminomethylcyclopentane-1-carboxylic acid;
N-[2-(2'-(1H-tetrazol-5-yl)-phenyl)-quinolin-6-ylmethyl]-N-valeroyl-2-aminomethyl-2-ethylbutyric acid;
N-[2-(2'-(1H-tetrazol-5-yl)-phenyl)-benzo[b]thiophen-5-ylmethyl]-N-valeroyl-2-aminomethyl-2-ethylbutyric acid;
N-[3-bromo-2-(2'-carboxyphenyl)-benzo[b]thiophen-5-ylmethyl]-N-valeroylvaline;
N-[2-(2'-(1H-tetrazol-5-yl)-phenyl)-benzofuran-5-ylmethyl]-N-valeroyl-2-aminomethyl-2-ethylbutyric acid;
N-[2-(2'-(1H-tetrazol-5-yl)-phenyl)-benzofuran-5-ylmethyl]-N-valeroyl-1-aminomethylcyclopentane-1-carboxylic acid;
N-[2-(2'-(1H-tetrazol-5-yl)-phenyl)-benzofuran-5-ylmethyl]-N-valeroylvaline;
N-[2-(2'-(1H-tetrazol-5-yl)-phenyl)-5-ylmethylbenzo[b]thiophene]-N-valeroyl-1-aminomethylcyclopentane-1-carboxylic acid;
N-[2-(2'-(1H-tetrazol-5-yl)-phenyl)-benzo[b]thiophen-5-ylmethyl]-N-valeroylvaline;
N-[3-bromo-2-(2'-carboxyphenyl)-benzo[b]thiophen-5-ylmethyl]-N-valeroyl-1-aminomethylcyclopentane-1-carboxylic acid;
N-[2-(2'-(1H-tetrazol-5-yl)-phenyl)-benzofuran-5-ylmethyl]-N-valeroyl-1-aminomethylcyclohexane-1-carboxylic acid;
N-[2-(2'-(tetrazol-5-yl)-phenyl)-naphthalen-6-ylmethyl]-N-valeroylvaline;
N-[2-(2'-(tetrazol-5-yl)-phenyl)-naphthalen-6-ylmethyl]-N-valeroyl-1-aminomethylcyclopentane-1-carboxylic acid;
N-[2-(2'-(tetrazol-5-yl)-phenyl)-naphthalen-6-ylmethyl]-N-valeroyl-2-aminomethyl-2-ethylbutyric acid;
N-[2-(2'-(tetrazol-5-yl)-phenyl)-naphthalen-6-ylmethyl]-N-valeroyl-1-aminomethylcyclohexane-1-carboxylic acid;
N-[2-(2'-(tetrazol-5-yl)-phenyl)-quinolin-6-ylmethyl]-N-cyclopropylcarbonylvaline;
N-[2-(2'-(tetrazol-5-yl)-phenyl)-quinolin-6-ylmethyl]-N-cyclopropylcarbonyl-1-aminomethylcyclopentane-1-carboxylic acid;
N-[2-(2'-(tetrazol-5-yl)-phenyl)-quinolin-6-ylmethyl]-N-cyclopropylcarbonyl-2-aminomethyl-2-ethylbutyric acid; and
N-[2-(2'-(tetrazol-5-yl)-phenyl)-quinolin-6-ylmethyl]-N-cyclopropylcarbonylvaline; or of a salt thereof.

15. A process according to claim 1 for the preparation of a compound of formula I selected from
N-[2-(2'-(1H-tetrazol-5-yl)-phenyl)-naphthalen-6-ylmethyl]-N-valeroyl-1-aminomethylcyclopentane-1-carboxylic acid ethyl ester;
N-[2-(2'-(1H-tetrazol-5-yl)-phenyl)-naphthalen-6-ylmethyl]-N-valeroyl-1-aminomethylcyclopentane-1-carboxylic acid;
N-[2-(2'-(1H-tetrazol-5-yl)-phenyl)-naphthalen-6-ylmethyl]-N-valeroyl-3-amino-2,2-dimethylpropionic acid;
N-[2-(2'-(1H-tetrazol-5-yl)-phenyl)-naphthalen-6-ylmethyl]-N-butyrylvaline; and
N-[2-(2'-(1H-tetrazol-5-yl)-phenyl)-naphthalen-6-ylmethyl]-N-valeroylvaline; or of a salt thereof.

16. A process according to claim 1 for the preparation of a compound of formula I selected from
N-{3-bromo-2-[2'-(1H-tetrazol-5-yl)-phenyl]-5-ylmethylbenzofuran}-N-valeroyl-1-aminomethylcyclopentane-1-carboxylic acid ethyl ester;
N-{3-bromo-2-[2'-(1H-tetrazol-5-yl)-phenyl]-5-ylmethylbenzofuran}-N-butyryl-1-aminomethylcyclopentane-1-carboxylic acid ethyl ester;
N-{3-bromo-2-[2'-(1H-tetrazol-5-yl)-phenyl]-5-ylmethylbenzofuran}-N-caproyl-1-aminomethylcyclopentane-1-carboxylic acid ethyl ester;
N-{3-bromo-2-[2'-(1H-tetrazol-5-yl)-phenyl]-5-ylmethylbenzofuran}-N-valeroylvaline ethyl ester;
N-{3-bromo-2-[2'-(1H-tetrazol-5-yl)-phenyl]-5-ylmethylbenzofuran}-N-butyrylvaline ethyl ester;
N-{3-bromo-2-[2'-(1H-tetrazol-5-yl)-phenyl]-5-ylmethylbenzofuran}-N-caproylvaline ethyl ester;
N-{3-bromo-2-[2'-(1H-tetrazol-5-yl)-phenyl]-5-ylmethylbenzofuran}-N-valeroylalanine ethyl ester;
N-{3-bromo-2-[2'-(1H-tetrazol-5-yl)-phenyl]-5-ylmethylbenzofuran}-N-valeroyl-1-aminomethylcyclopentane-1-carboxylic acid;
N-{3-bromo-2-[2'-(1H-tetrazol-5-yl)-phenyl]-5-ylmethylbenzofuran}-N-butyryl-1-aminomethylcyclopentane-1-carboxylic acid;
N-{3-bromo-2-[2'-(1H-tetrazol-5-yl)-phenyl]-5-ylmethylbenzofuran}-N-caproyl-1-aminomethylcyclopentane-1-carboxylic acid;
N-{3-bromo-2-[2'-(1H-tetrazol-5-yl)-phenyl]-5-ylmethylbenzofuran}-N-valeroylvaline;
N-{3-bromo-2-[2'-(1H-tetrazol-5-yl)-phenyl]-5-ylmethylbenzofuran}-N-butyrylvaline;
N-{3-bromo-2-[2'-(1H-tetrazol-5-yl)-phenyl]-5-ylmethylbenzofuran}-N-caproylvaline; and
N-{3-bromo-2-[2'-(1H-tetrazol-5-yl)-phenyl]-5-ylmethylbenzofuran}-N-valeroylalanine; or of a salt thereof.

17. A process according to claim 1 for the preparation of a compound of formula I selected from
N-{3-bromo-2-[2'-(1H-tetrazol-5-yl)-phenyl]-benzofuran-5-ylmethyl}-N-valeroyl-1-aminomethylcyclopentane-1-carboxylic acid ethyl ester,
N-{3-bromo-2-[2'-(1H-tetrazol-5-yl)-phenyl]-benzofuran-5-ylmethyl}-N-butyryl-1-aminomethylcyclopentane-1-carboxylic acid ethyl ester;
N-{3-bromo-2-[2'-(1H-tetrazol-5-yl)-phenyl]-benzofuran-5-ylmethyl}-N-caproyl-1-aminomethylcyclopentane-1-carboxylic acid ethyl ester;
N-{3-bromo-2-[2'-(1H-tetrazol-5-yl)-phenyl]-benzofuran-5-ylmethyl}-N-valeroylvaline ethyl ester;
N-{3-bromo-2-[2'-(1H-tetrazol-5-yl)-phenyl]-benzofuran-5-ylmethyl}-N-butyrylvaline ethyl ester;
N-{3-bromo-2-[2'-(1H-tetrazol-5-yl)-phenyl]-benzofuran-5-ylmethyl}-N-caproylvaline ethyl ester;
N-{3-bromo-2-[2'-(1H-tetrazol-5-yl)-phenyl]-benzofuran-5-ylmethyl}-N-valeroylalanine ethyl ester;
N-{3-bromo-2-[2'-(1H-tetrazol-5-yl)-phenyl]-benzofuran-5-ylmethyl}-N-valeroyl-1-aminomethylcyclopentane-1-carboxylic acid,
N-{3-bromo-2-[2'-(1H-tetrazol-5-yl)-phenyl]-benzofuran-5-ylmethyl}-N-butyryl-1-aminomethylcyclopentane-1-carboxylic acid;
N-{3-bromo-2-[2'-(1H-tetrazol-5-yl)-phenyl]-benzofuran-5-ylmethyl}-N-caproyl-1-aminomethylcyclopentane-1-carboxylic acid;
N-{3-bromo-2-[2'-(1H-tetrazol-5-yl)-phenyl]-benzofuran-5-ylmethyl}-N-valeroylvaline;
N-{3-bromo-2-[2'-(1H-tetrazol-5-yl)-phenyl]-benzofuran-5-ylmethyl}-N-butyrylvaline;
N-{3-bromo-2-[2'-(1H-tetrazol-5-yl)-phenyl]-benzofuran-5-ylmethyl}-N-caproylvaline and
N-{3-bromo-2-[2'-(1H-tetrazol-5-yl)-phenyl]-benzofuran-5-ylmethyl}-N-valeroylalanine; or of a salt thereof.

18. A process for the preparation of a pharmaceutical composition wherein a compound according to any one of claims 1 to 18 or a pharmaceutically acceptable salt thereof is mixed, if desired, with pharmaceutically acceptable excipients and/or carriers.

19. The use of a compound according to any one of claims 1 to 17 or of a pharmaceutically acceptable salt thereof for the preparation of a pharmaceutical composition for the treatment of high blood pressure and cardiac insufficiency and also glaucoma.

## Revendications (Revendications pour l'(les) Etat(s) contractant(s) suivant(s): AT, BE, CH, DE, DK, FR, GB, IE, IT, LI, LU, NL, PT, SE)

1. Composé de formule 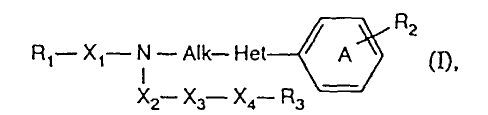 dans laquelle
R₁ représente un alkyle en C₁ à C₇, qui est non substitué ou substitué par un halogène ou un hydroxy, ou représente un alcényle en C₂ à C₇, un cycloalkyle en C₃ à C₇, un cycloalkyloxy en C₃ à C₇, un alcoxy en C₁ à C₇ ou un cycloalkyle en C₃ à C₇ - alcoxy en C₁ à C₇ ;
R₂ représente un 1-tétrazol-5-yle, un carboxy, un alcoxy en C₁ à C₇-carbonyle, SO₃H, PO₂H₂, PO₃H₂ ou un halogèn-alcane en C₁ à C₇-sulfonylamino ;
R₃ représente un 1H-tétrazol-5-yle, un hydroxyméthyle, un alcoxy en C₁ à C₇-méthyle, un formyle, un carboxy, un alcoxy en C₁ à C₇-carbonyle, un alcoxy en C₁ à C₇ - alcoxy en C₁ à C₇-carbonyle, un phényl-alcoxy en C₁ à C₇ - carbonyle ou un carbamoyle, dont le groupe amino est non substitué ou est mono- ou de façon indépendante disubstitué par un alkyle en C₁ à C₇, un alcényle en C₃ à C₇ ou un phényl-alkyle en C₁ à C₇, ou est disubstitué par un alkylène en C₂ à C₇ ou un alcényloxy en C₂ à C₄ - alkylène en C₂ à C₄ ;
Alk représente un méthylène, un éthylène ou un éthylidène ;
Het représente 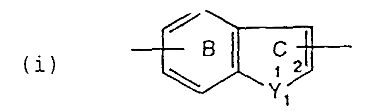 dans laquelle Y₁ représente O, S ou N(R) et R représente un hydrogène ou un alkyle en C₁ à C₇ ; ou 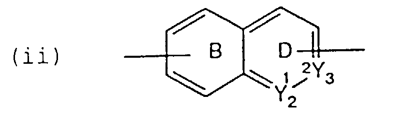 dans laquelle l'une des variables Y₂ et Y₃ représente C(R') et l'autre représente N, ou les deux variables représentent l'une et l'autre C(R') ; et R' représente un hydrogène, un halogène, un alkyle en C₁ à C₇, un alcoxy en C₁ à C₇, un alcényloxy en C₂ à C₇, un phénoxy, un benzyloxy, un trifluorométhyle ou S(O)ₘ-R, où M vaut 0, 1 ou 2 ; et R représente un hydrogène ou un alkyle en C₁ à C₇ ;
X₁ représente -CO- ou -S(O)ₘ- et l'indice m vaut 0, 1 ou 2 ;
l'une des variables X₂ et X₄ représente un alkylène en C₁ à C₄ et l'autre des variables X₂ et X₄ représente une liaison ; ou les deux variables X₂ et X₄ représentent l'une et l'autre une liaison ;
X₃ représente un cycloalkylidène en C₃ à C₇ ou l'élément de structure -C(Xₐ)(X_{b})- et Xₐ représente un hydrogène ou un alkyle en C₁ à C₇ et X_{b} est un alkyle en C₁ à C₇ ;
et les noyaux A, B, C et D jusqu'aux substituants indiqués dans la formule, ainsi que les substituants aromatiques, sont indépendamment l'un de l'autre non substitués ou mono- ou polysubstitués par des substituants choisis dans le groupe constitué par halogène, alkyle en C₁ à C₇, alcoxy en C₁ à C₇, alcényloxy en C₂ à C₇, phénoxy, benzyloxy, trifluorométhyle et S(O)ₘ-R, où m vaut 0, 1 ou 2, et R représente un hydrogène ou un alkyle en C₁ à C₇ ;
et leurs sels.

2. Composé selon la revendication 1 de formule I, dans laquelle
R₁ représente un alkyle en C₁ à C₇ qui est non substitué ou substitué par un halogène ou un hydroxy, ou un alcényle en C₂ à C₇, cycloalkyle en C₃ à C₇ ou alcoxy en C₁ à C₇ ; et ses sels.

3. Composé selon la revendication 1 de formule I dans laquelle
R₁ représente un alkyle en C₂ à C₇, ou un alkyle en C₁ à C₇ qui est substitué par un halogène ou un hydroxy, ou un alcényle en C₃ à C₇, un cycloalkyle en C₃ à C₆, ou un alcoxy en C₁ à C₇ ;
R₂ représente un 1H-tétrazol-5-yle, un carboxy, un alcoxy en C₁ à C₄ - carbonyle ou un halogén-alcane en C₁ à C₄ - sulfonylamino ;
R₃ représente un 1H-tétrazol-5-yle, un carboxy, un alcoxy en C₁ à C₄ - carbonyle, un alcoxy en C₁ à C₄ - alcoxy en C₁ à C₄ - carbonyle, un phényl-alcoxy en C₁ à C₄ -carbonyle ou un carbamoyle, dont le groupe amino est mono- ou indépendamment disubstitué par un alkyle en C₁ à C₄, ou disubstitué par un alkylène en C₄ à C₆ ou un éthylènoxyéthylène ;
Alk représente un méthylène, éthylène ou éthylidène ;
Het représente 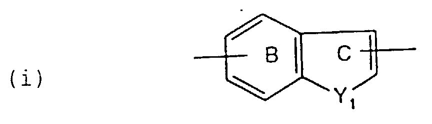
où Y₁ représente O, S ou N(R) et R représente un hydrogène ou un alkyle en C₁ à C₄ ; ou 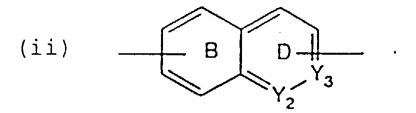 dans laquelle l'une des variables Y₂ et Y₃ représente CH et l'autre représente N, ou les deux variables représentent l'une et l'autre CH ;
X₁ représente -CO- ou -S(O)ₘ- et l'indice m vaut 0, 1 ou 2 ;
l'une des variables X₂ et X₄ représente un alkylène en C₁ à C₄ et l'autre des variables X₂ et X₄ représente une liaison ; ou les deux variables X₂ et X₄ représentent chacune une liaison ;
X₃ représente un cycloalkylidène en C₃ à C₆ ou l'élément de structure -C(X_{A})(X_{b})- et Xₐ représente un hydrogène ou un alkyle en C₁ à C₇ et X_{b} représente un alkyle en C₁ à C₇ ;
et les noyaux A, B, C et D, jusqu'aux substituants indiqués dans la formule, ainsi que les substituants aromatiques, sont indépendamment l'un de l'autre non substitués ou mono- ou polysubstitués par des substituants choisis dans le groupe constitué par halogène, alkyle en C₁ à C₄, alcoxy en C₁ à C₄, alcényloxy en C₃ à C₅, phénoxy, benzyloxy, trifluorométhyle et S(O)ₘ-R, où m vaut 0, 1 ou 2, et R représente un hydrogène ou un alkyle en C₁ à C₄ ; et ses sels.

4. Composé selon la revendication 2 ou 3, de formule I, dans laquelle :
R₁ représente un alkyle en C₂ à C₇ ou un alkyle en C₁ à C₄, qui est substitué par un halogène ou un hydroxy, ou un alcényle en C₃ à C₇, un cycloalkyle en C₃ à C₆ ou un alcoxy en C₁ à C₇ ;
R₂ représente un 1H-tétrazol-5-yle, un carboxy ou un alcoxy en C₁ à C₄ - carbonyle ;
R₃ représente un 1H-tétrazol-5-yle, un carboxy, un alcoxy en C₁ à C₄ - carbonyle, un alcoxy en C₁ à C₄ - alcoxy en C₁ à C₄ - carbonyle, ou un phényl-alcoxy en C₁ à C₂ - carbonyle ;
Alk représente un méthylène, ou encore un éthylène ou un éthylidène ;
Het représente 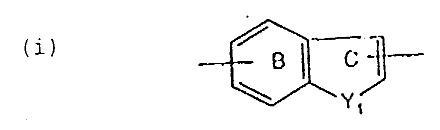
dans laquelle Y₁ représente O, S ou NH ; ou 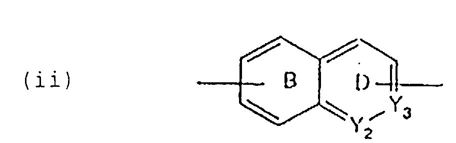 dans laquelle l'une des variables Y₂ et Y₃ représente CH et l'autre représente N, ou les deux variables représentent l'une et l'autre CH ;
en premier lieu 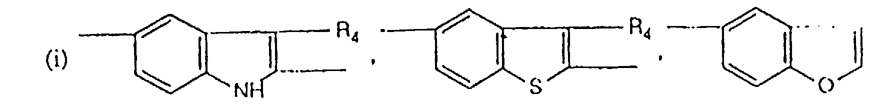 et R₄ représente un hydrogène, un halogène, comme le brome, ou encore un alkyle en C₁ à C₇, un alcoxy en C₁ à C₇ ou un trifluorométhyle ; ou 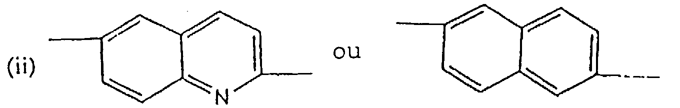 X₁ représente -CO- ;
l'une des variables X₂ et X₄ représente un alkylène en C₁ à C₂, et l'autre des variables X₂ et X₄ représente une liaison ; ou les deux variables X₂ et X₄ représentent l'une et l'autre une liaison ;
X₃ représente un cycloalkylidène en C₅ à C₆ ou l'élément de structure -C(Xₐ)(X_{b})-, et Xₐ représente un hydrogène ou un alkyle en C₁ à C₅, et X_{b} représente un alkyle en C₁ à C₅ ;
en premier lieu
(i) X₂ représente un alkylène en C₁ à C₂ ; X₄ est une liaison ; X₃ représente un cycloalkylidène en C₅ à C₆ ; ou
(ii) X₂ et X₄ représentent chacun une liaison ; et X₃ représente l'élément de structure -C(Xₐ)(X_{b})-, et Xₐ représente un hydrogène ou un alkyle en C₁ à C₅ et X_{b} représente un alkyle en C₁ à C₅ ;
et les noyaux A, B, C et D, jusqu'aux substituants indiqués dans la formule, sont indépendamment l'un de l'autre non substitués ou mono- ou polysubstitués par des substituants choisis dans le groupe constitué par halogène, alkyle en C₁ à C₄, alcoxy en C₁ à C₄ et trifluorométhyle ;
et ses sels.

5. Composé selon la revendication 1, de formule I dans laquelle l'une des variables Y₂ et Y₃ représente C(R') et l'autre représente N ou C(R'), où R' représente un halogène, un alkyle en C₁ à C₄, un alcoxy en C₁ à C₄ ou un trifluorométhyle, et ses sels.

6. Composé selon la revendication 1 de formule I, dans laquelle
R₁ représente un alkyle en C₂ à C₇, comme n-propyle ou n-butyle, ou un cycloalkyle en C₃ à C₆, comme cyclopropyle, ou un alcoxy en C₁ à C₄, comme méthoxy, éthoxy, propyloxy ou butyloxy ;
R₂ représente un 1H-tétrazol-5-yle, un carboxy ou un alcoxy en C₁ à C₄ - carbonyle, comme méthoxy- ou éthoxy-carbonyle ;
R₃ représente un carboxy ou un alcoxy en C₁ à C₄ - carbonyle, comme méthoxy-, éthoxy-, ou tert.-butyloxy-carbonyle ;
Alk est un méthylène ;
Het représente 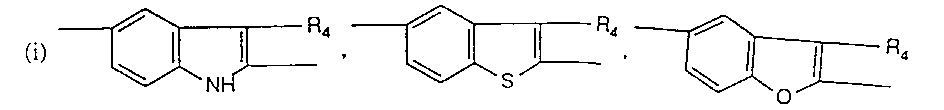 et R₄ représente un hydrogène, un halogène, comme le brome, ou encore un alkyle en C₁ à C₄, comme méthyle, un alcoxy en C₁ à C₄, comme méthoxy, ou trifluorométhyle ; ou 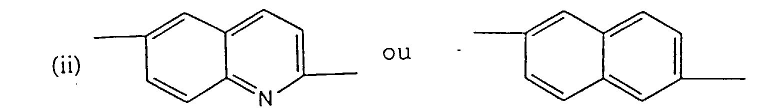 X₁ représente -CO- ;
(i) X₂ représente un alkylène en C₁ à C₂, en particulier un méthylène ; X₄ représente une liaison ; X₃ représente un cycloalkylidène en C₅ à C₆ ; ou
(ii) X₂ et X₄ représentent chacun une liaison ; et X₃ représente l'élément de structure -C(Xₐ) (X_{b})- et Xₐ représente un hydrogène ou un alkyle en C₁ à C₅, comme éthyle, et X_{b} représente un alkyle en C₁ à C₅, comme éthyle ou isopropyle ;
et ses sels.

7. Composé selon l'une des revendications 2 à 5, de formule I, dans laquelle
R₃ représente un hydroxyméthyle, un alcoxy en C₁ à C₄ -méthyl- ou un formyle ; les autres variables ont les significations indiquées respectivement ci-dessus.

8. Composé selon la revendication 1, de formule I, dans laquelle
R₁ représente un alkyle en C₂ à C₅, comme n-propyle ou n-butyle ;
R₂ représente un 1H-tétrazol-5-yle ou un carboxy ;
R₃ représente un carboxy ou un alcoxy en C₁ à C₄ - carbonyle, comme méthoxy-, éthoxy-, ou tert-butyloxy-carbonyle ;
Alk est un méthylène ;
Het est un 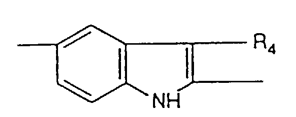 et R₄ représente un hydrogène, un halogène, comme le brome, ou un alkyle en C₁ à C₄, comme le méthyle ;
X₁ représente -CO- ;
(i) X₂ représente un alkylène en C₁ à C₂, en particulier le méthylène ; X₄ représente une liaison ; X₃ représente un cycloalkylidène en C₅ à C₆ ; ou
(ii) X₂ et X₄ représentent chacun une liaison ; et X₃ représente l'élément de structure -C (Xₐ) (X_{b})- et Xₐ représente un hydrogène ou un alkyle en C₁ à C₅, comme éthyle, et X_{b} représente un alkyle en C₁ à C₅, comme éthyle, isopropyle ou 3-butyle ;
et ses sels.

9. Composé selon la revendication 1 de formule I, dans laquelle
R₁ représente un alkyle en C₂ à C₅, comme n-propyle ou n-butyle ;
R₂ représente un 1H-tétrazol-5-yle ou un carboxy ;
R₃ représente un carboxy ou un alcoxy en C₁ à C₄ - carbonyle, comme méthoxy-, éthoxy-, ou tert-butyloxy-carbonyle ;
Alk est un méthylène ;
Het est un 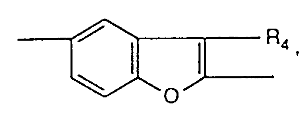 et R₄ représente un hydrogène, un halogène, comme le brome, ou un alkyle en C₁ à C₄, comme le méthyle ;
X₁ représente -CO- ;
(i) X₂ représente un alkylène en C₁ à C₂, en particulier le méthylène ; X₄ représente une liaison ; X₃ représente un cycloalkylidène en C₅ à C₆ ; ou
(ii) X₂ et X₄ représentent chacun une liaison ; et X₃ représente l'élément de structure -C(Xₐ)(X_{b})- et Xₐ représente un hydrogène ou un alkyle en C₁ à C₅, comme éthyle, et X_{b} représente un alkyle en C₁ à C₅, comme éthyle, isopropyle ou 3-butyle ;
et ses sels.

10. Composé selon la revendication 1 de formule I, dans laquelle :
R₁ représente un alkyle en C₂ à C₅, comme n-propyle ou n-butyle ;
R₂ représente un 1H-tétrazol-5-yle ou un carboxy ;
R₃ représente un carboxy ou un alcoxy en C₁ à C₄ - carbonyle, comme méthoxy-, éthoxy-, ou tert-butyloxy-carbonyle ;
Alk est un méthylène ;
Het est un 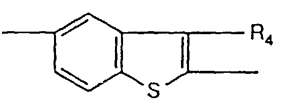 où R₄ représente un hydrogène, un halogène, comme le brome, ou un alkyle en C₁ à C₄, comme le méthyle ;
X₁ représente -CO- ;
(i) X₂ représente un alkylène en C₁ à C₂, en particulier le méthylène ; X₄ représente une liaison ; X₃ représente un cycloalkylidène en C₅ à C₆, comme cyclopentylidène, ou cyclohexylidène ; ou
(ii) X₂ et X₄ représentent chacun une liaison ; et X₃ représente l'élément de structure -C(Xₐ)(X_{b})- et Xₐ représente un hydrogène ou un alkyle en C₁ à C₅, comme éthyle, et X_{b} représente un alkyle en C₁ à C₅, comme éthyle, isopropyle ou 3-butyle ;
et ses sels.

11. Composé selon la revendication 1 de formule I, dans laquelle
R₁ représente un alkyle en C₂ à C₅, comme n-propyle ou n-butyle ;
R₂ représente un 1H-tétrazol-5-yle ou un carboxy ;
R₃ représente un carboxy ou un alcoxy en C₁ à C₄ - carbonyle, comme méthoxy-, éthoxy-, ou tert-butyloxy-carbonyle ;
Alk est un méthylène ;
Het est un 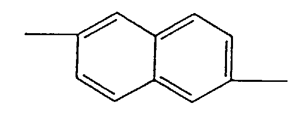
X₁ représente -CO- ;
(i) X₂ représente un alkylène en C₁ à C₂, en particulier le méthylène ; X₄ représente une liaison ; X₃ représente un cycloalkylidène en C₅ à C₆ ; ou
(ii) X₂ et X₄ représentent chacun une liaison ; et X₃ représente l'élément de structure -C(Xₐ) (X_{b})- et Xₐ représente un hydrogène ou un alkyle en C₁ à C₅, comme éthyle, et X_{b} représente un alkyle en C₁ à C₅, comme éthyle, isopropyle ou 3-butyle ;
et ses sels.

12. Composé selon la revendication 1, de formule I, dans laquelle
R₁ représente un alkyle en C₂ à C₅, comme n-propyle ou n-butyle ;
R₂ représente un 1H-tétrazol-5-yle ou un carboxy ;
R₃ représente un carboxy ou un alcoxy en C₁ à C₄ - carbonyle, comme méthoxy-, éthoxy-, ou tert-butyloxy-carbonyle ;
Alk est un méthylène ;
Het est un 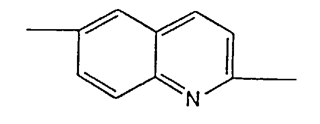 X₁ représente -CO- ;
(i) X₂ représente un alkylène en C₁ à C₂, en particulier le méthylène ; X₄ représente une liaison ; X₃ représente un cycloalkylidène en C₅ à C₆ ; ou
(ii) X₂ et X₄ représentent chacun une liaison ; et X₃ représente l'élément de structure -C(Xₐ) (X_{b})- et Xₐ représente un hydrogène ou un alkyle en C₁ à C₅, comme éthyle, et X_{b} représente un alkyle en C₁ à C₅, comme éthyle, isopropyle ou 3-butyle ;
et ses sels.

13. Composé selon la revendication de formule I, dans laquelle
R₁ représente un alkyle en C₂ à C₅, comme n-propyle ou n-butyle ;
R₂ représente un 1H-tétrazol-5-yle ou un carboxy ;
R₃ représente un carboxy ou un alcoxy en C₁ à C₄ - carbonyle, comme méthoxy-, éthoxy-, ou tert-butyloxy-carbonyle ;
Alk est un méthylène ;
Het est un 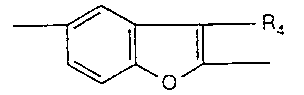 et R₄ représente un hydrogène ou un halogène dont le nombre d'atomes va jusqu'à 35 compris, comme le brome ;
X₁ représente -CO- ;
(i) X₂ représente un alkylène en C₁ à C₂, en particulier le méthylène ; X₄ représente une liaison ; X₃ représente un cycloalkylidène en C₅ à C₆ ; ou
(ii) X₂ et X₄ représentent chacun une liaison ; et X₃ représente l'élément de structure -C(Xₐ) (X_{b})- et Xₐ représente un hydrogène ou un alkyle en C₁ à C₅, comme éthyle, et X_{b} représente un alkyle en C₁ à C₅, comme éthyle, isopropyle ou 3-butyle ;
et ses sels.

14. Composé selon la revendication 1 choisi parmi les composés suivants :
N-[2-(2'-(1H-tétrazol-5-yl)-phényl)-quinoléin-6-yl-méthyl]-N-valéroyl-valine ;
Acide N-[2-(2'-(1H-tétrazol-5-yl-phényl)-quinoléin-6-yl-méthyl]-N-valéroyl-1-aminométhyl-cyclopentane-1-carboxylique ;
Acide N-[2-(2'-(1H-tétrazol-5-yl)-phényl)-quinoléin-6-yl-méthyl]-N-valéroyl-2-aminométhyl-2-éthyl-butyrique ;
Acide N-[2-(2'-(1H-tétrazol-5-yl)-phényl)benzo[b]-thiophén-5-yl-méthyl]-N-valéroyl-2-aminométhyl-2-éthyl-butyrique ;
N-[3-bromo-2-(2'-carboxy-phényl)-benzo[b]-thiophén-5-yl-méthyl]-N-valéroyl-valine ;
Acide N-[2-(2'-(1H-tétrazol-5-yl)-phényl)-benzofuran-5-yl-méthyl]-N-valéroyl-2-aminométhyl-2-éthyl-butyrique ;
Acide N-[2-(2'-(1H-tétrazol-5-yl)-phényl-benzofuran-5-yl-méthyl]-N-valéroyl-1-aminométhyl-cyclopentane-1-carboxylique ;
N-[2-(2'-(1H-tétrazol-5-yl)-phényl)-benzofuran-5-yl-méthyl]-N-valéroyl-valine;
Acide N-[2-(2'-(1H-tétrazol-5-yl)-phényl)-5-yl-méthyl-benzo[b]thiophén]-N-valéroyl-1-aminométhylcyclopentan-1-carboxylique ;
N-[2-(2'-(1H-tétrazol-5-yl)-phényl-benzo[b]-thiophén-5-yl-méthyl]-N-valéroyl-valine ;
Acide N-[3-bromo-2-(2'-carboxyphényl)-benzo[b]-thiophén-5-yl-méthyl]-N-valéroyl-1-aminométhyl-cyclopentane-1-carboxylique; et
Acide N-[2-(2'-(1H-tétrazol-5-yl)-phényl)-benzofuran-5-yl-méthyl]-N-valéroyl-1-aminométhyl-cyclohexane-1-carboxylique;
N-[2-(2'-(tétrazol-5-yl)-phényl)-naphtalène-6-yl-méthyl]-N-valéroyl-valine;
Acide N-[2-(2'-(tétrazol-5-yl)-phényl)-naphtalène-6-yl-méthyl]-N-valéroyl-1-aminométhyl-cyclopentane-1-carboxylique ;
Acide N-[2-(2'-(tétrazol-5-yl)-phényl)-naphtalène-6-yl-méthyl]-N-valéroyl-2-aminométhyl-2-éthylbutyrique ;
Acide N-[2-(2'-(tétrazol-5-yl)-phényl)-naphtalène-6-yl-méthyl]-N-valéroyl-1-aminométhyl-cyclohexane-1-carboxylique ;
N-[2-(2'-(tétrazol-5-yl)-phényl)-quinoléin-6-yl-méthyl]-N-cyclopropylcarbonyl-valine;
Acide N-[2-(2'-(tétrazol-5-yl)-phényl)-quinoléin-6-yl-méthyl]-N-cyclopropylcarbonyl-1-aminométhyl-cyclopentane-1-carboxylique ;
Acide N-[2-(2'-(tétrazol-5-yl)-phényl)-quinoléin-6-yl-méthyl]-N-cyclopropylcarbonyl-2-aminométhyl-2-éthylbutyrique ;
N-[2-(2'-(tétrazol-5-yl)-phényl)-quinoléin-6-yl-méthyl]-N-cyclopropylcarbonyl-valine;
ou un de leurs sels.

15. Composé selon la revendication 1, choisi parmi les suivants :
Ester éthylique de l'acide N-[2-(2'-(1H-tétrazol-5-yl)-phényl)-naphtalène-6-ylméthyl]-N-valéroyl-1-aminométhyl-cyclopentane-1-carboxylique ;
Acide N-[2-(2'-(1H-tétrazol-5-yl)-phényl)-naphtalène-6-ylméthyl]-N-valéroyl-1-aminométhyl-cyclopentane-1-carboxylique ;
Acide N-[2-(2'-(1H-tétrazol-5-yl)-phényl-naphtalène-6-ylméthyl]-N-valéroyl-3-amino-2,2-diméthylpropionique ;
N-[2-(2'-(1H-tétrazol-5-yl)-phényl)-naphtalène)-6-yl-méthyl]-N-butyryl-valine ; et
N-[2-(2'-(1H-tétrazol-5-yl)-phényl)-naphtalène)-6-ylméthyl]-N-valéroyl-valine ;
ou un de leurs sels.

16. Composé selon la revendication 1, choisi parmi les suivants :
Ester éthylique de l'acide N-{3-bromo-2-[2'-(1H-tétrazol-5-yl)-phényl]-5-ylméthyl-benzofuran}-N-valéroyl-1-aminométhyl-cyclopentane-1-carboxylique ;
Ester éthylique de l'acide N-{3-bromo-2-[2'-(1H-tétrazol-5-yl)-phényl]-5-ylméthyl-benzofuran}-N-butyryl-1-aminométhyl-cyclopentane-1-carboxylique ;
Ester éthylique de l'acide N-{3-bromo-2-[2'-(1H-tétrazol-5-yl)-phényl]-5-ylméthyl-benzofuran}-N-caproyl-1-aminométhyl-cyclopentane-1-carboxylique ;
Ester éthylique de N-{3-bromo-2-[2'-(1H-tétrazol-5-yl)-phényl]-5-ylméthyl-benzofuran}-N-valéroyl-valine ;
Ester éthylique de N-{3-bromo-2-[2'-(1H-tétrazol-5-yl)phényl]-5-ylméthyl-benzofuran}-N-butyryl-valine ;
Ester éthylique de N-{3-bromo-2-[2'-(1H-tétrazol-5-yl)phényl]-5-ylméthyl-benzofuran}-N-caproyl-valine ;
Ester éthylique de N-{3-bromo-2-[2'-(1H-tétrazol-5-yl)phényl]-5-ylméthyl-benzofuran}-N-valéroyl-alanine ;
Acide N-{3-bromo-2-[2'-(1H-tétrazol-5-yl)phényl]5-ylméthyl-benzofuran}-N-valéroyl-1-aminométhyl-cyclopentane-1-carboxylique ;
Acide N-{3-bromo-2-[2'-(1H-tétrazol-5-yl)phényl]-5-ylméthyl-benzofuran}-N-butyryl-1-aminométhyl-cyclopentan-1-carboxylique ;
Acide N-{3-bromo-2-[2'-(1H-tétrazol-5-yl)phényl]-5-ylméthyl-benzofuran}-N-caproyl-1-aminométhyl-cyclopentane-1-carboxylique ;
N-{3-bromo-2-[2'-(1H-tétrazol-5-yl)phényl]-5-ylméthyl-benzofuran}-N-valéroyl-valine ;
N-{3-bromo-2-[2'-(1H-tétrazol-5-yl)phényl]-5-ylméthyl-benzofuran}-N-butyryl-valine;
N-{3-bromo-2-[2'-(1H-tétrazol-5-yl)phényl]-5-ylméthyl-benzofuran}-N-caproyl-valine; et
N-{3-bromo-2-[2'-(1H-tétrazol-5-yl)phényl]-5-ylméthyl-benzofuran}-N-valéroyl-alanine;
ou un de leurs sels.

17. Composé selon la revendication 1, choisi parmi les suivants :
Ester éthylique de l'acide N-{3-bromo-2-[2'-(1H-tétrazol-5-yl)phényl]-benzofuran-5-ylméthyl}-N-valéroyl-1-aminométhyl-cyclopentane-1-carboxylique ;
Ester éthylique de l'acide N-{3-bromo-2-[2'-(1H-tétrazol-5-yl)-phényl]-benzofuran-5-ylméthyl}-N-butyryl-1-amino-méthylcyclopentane-1-carboxylique ;
Ester éthylique de l'acide N-{3-bromo-2-[2'-(1H-tétrazol-5-yl)-phényl]-benzofuran-5-ylméthyl}-N-caproyl-1-aminométhyl-cyclopentane-1-carboxylique ;
Ester éthylique de N-{3-bromo-2-[2'-(1H-tétrazol-5-yl)-phényl]-benzofuran-5-ylméthyl}-N-valéroyl-valine ;
Ester éthylique de N-{3-bromo-2-[2'-(1H-tétrazol-5-yl)-phényl]-benzofuran-5-ylméthyl}-N-butyryl-valine ;
Ester éthylique de N-{3-bromo-2-[2'-(1H-tétrazol-5-yl)-phényl]-benzofuran-5-ylméthyl}-N-caproyl-valine ;
Ester éthylique de N-{3-bromo-2-[2'-(1H-tétrazol-5-yl)-phényl]-benzofuran-5-ylméthyl}-N-valéroyl-alanine ;
Acide N-{3-bromo-2-[2'-(1H-tétrazol-5-yl)-phényl]-benzofuran-5-ylméthyl}-N-valéroyl-1-aminométhyl-cyclopentane-1-carboxylique ;
Acide N-{3-bromo-2-[2'-(1H-tétrazol-5-yl)-phényl]-benzofuran-5-ylméthyl}-N-butyryl-1-aminométhyl-cyclopentane-1-carboxylique ,
Acide N-{3-bromo-2-[2'-(1H-tétrazol-5-yl)-phényl]-benzofuran-5-ylméthyl}-N-caproyl-1-aminométhyl-cyclopentane-1-carboxylique ;
N-{3-bromo-2-[2'-(1H-tétrazol-5-yl)-phényl]-benzofuran-5-ylméthyl}-N-valéroyl-valine;
N-{3-bromo-2-[2'-(1H-tétrazol-5-yl)-phényl]-benzofuran-5-ylméthyl}-N-butyryl-valine ;
N-{3-bromo-2-[2'-(1H-tétrazol-5-yl)-phényl]-benzofuran-5-ylméthyl}-N-caproyl-valine; et
N-{3-bromo-2-[2'-(1H-tétrazol-5-yl)-phényl]-benzofuran-5-ylméthyl}-N-valéroyl-alanine,
ou un de leurs sels.

18. Composé selon l'une des revendications 1 à 17, aux fins d'application dans un procédé de traitement thérapeutique du corps animal ou humain.

19. Procédé de préparation d'un composé de formule I et de l'un de ses sels selon l'une des revendications 1 à 17, caractérisé en ce que
a) dans un composé de formule 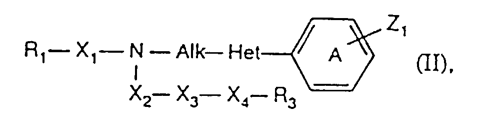 dans laquelle Z₁ représente un radical transformable en R₂ ou l'un de ses sels, on transforme Z₁ en R₂ ; ou
b) on fait réagir un composé de formule 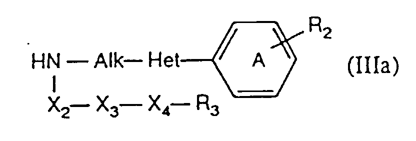 avec un composé de formule R₁-X₁-OH (IIIb), un de ses dérivés réactifs ou un de ses sels ;
et à chaque fois, si on le désire, on isole un composé I obtenu selon le procédé ou d'une autre manière, sous forme libre ou sous forme de sel, et on transforme un composé I obtenu selon le procédé ou d'une autre manière en un autre composé I, on sépare un mélange d'isomères obtenu selon le procédé et on isole l'isomère désiré, et/ou on transforme un composé libre I obtenu selon le procédé en un sel, ou un sel d'un composé I obtenu selon le procédé en le composé I libre ou en un autre sel.

20. Préparation pharmaceutique contenant comme substance active un composé selon l'une des revendications 1 - 18, sous forme libre ou sous la forme d'un sel pharmaceutiquement acceptable, avec éventuellement des additifs habituels.

21. Application d'un composé selon l'un des revendications 1 - 18, sous forme libre ou sous la forme d'un sel pharmaceutiquement acceptable, à la préparation d'une préparation pharmaceutique pour le traitement de l'hypertension artérielle et de l'insuffisance cardiaque ainsi que du glaucome.

## Revendications (Revendications pour l'(les) Etat(s) contractant(s) suivant(s): ES, GR)

1. Procédé de préparation d'un composé de formule 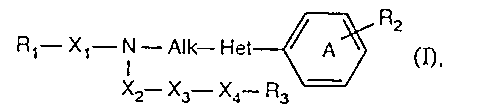 dans laquelle
R₁ représente un alkyle en C₁ à C₇, qui est non substitué ou substitué par un halogène ou un hydroxy, ou représente un alcényle en C₂ à C₇, un cycloalkyle en C₃ à C₇, un cycloalkyloxy en C₃ à C₇, un alcoxy en C₁ à C₇ ou un cycloalkyle en C₃ à C₇ - alcoxy en C₁ à C₇ ;
R₂ représente un 1-tétrazol-5-yle, un carboxy, un alcoxy en C₁ à C₇-carbonyle, SO₃H, PO₂H₂, PO₃H₂ ou un halogèn-alcane en C₁ à C₇-sulfonylamino ;
R₃ représente un 1H-tétrazol-5-yle, un hydroxyméthyle, un alcoxy en C₁ à C₇-méthyle, un formyle, un carboxy, un alcoxy en C₁ à C₇-carbonyle, un alcoxy en C₁ à C₇ - alcoxy en C₁ à C₇ -carbonyle, un phényl-alcoxy en C₁ à C₇ - carbonyle ou un carbamoyle, dont le groupe amino est non substitué ou est mono- ou de façon indépendante disubstitué par un alkyle en C₁ à C₇, un alcényle en C₃ à C₇ ou un phényl-alkyle en C₁ à C₇, ou est disubstitué par un alkylène en C₂ à C₇ ou un alcényloxy en C₂ à C₄ - alkylène en C₂ à C₄ ;
Alk représente un méthylène, un éthylène ou un éthylidène ;
Het représente 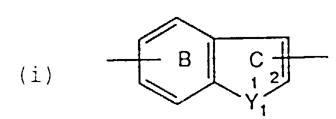 danslaquelle Y₁ représente O, S ou N(R) et R représente un hydrogène ou un alkyle en C₁ à C₇ ; ou 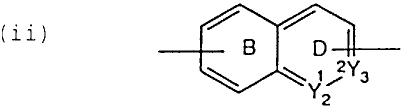 dans laquelle l'une des variables Y₂ et Y₃ représente C(R') et l'autre représente N, ou les deux variables représentent l'une et l'autre C(R') ; et R' représente un hydrogène, un halogène, un alkyle en C₁ à C₇, un alcoxy en C₁ à C₇, un alcényloxy en C₂ à C₇, un phénoxy, un benzyloxy, un trifluorométhyle ou S(O)ₘ-R, où M vaut 0, 1 ou 2 ; et R représente un hydrogène ou un alkyle en C₁ à C₇ ;
X₁ représente -CO- ou -S(O)ₘ- et l'indice m vaut 0, 1 ou 2 ;
l'une des variables X₂ et X₄ représente un alkylène en C₁ à C₄ et l'autre des variables X₂ et X₄ représente une liaison ; ou les deux variables X₂ et X₄ représentent l'une et l'autre une liaison ;
X₃ représente un cycloalkylidène en C₃ à C₇ ou l'élément de structure -C(Xₐ)(X_{b})- et Xₐ représente un hydrogène ou un alkyle en C₁ à C₇ et X_{b} est un alkyle en C₁ à C₇ ;
et les noyaux A, B, C et D jusqu'aux substituants indiqués dans la formule, ainsi que les substituants aromatiques, sont indépendamment l'un de l'autre non substitués ou mono- ou polysubstitués par des substituants choisis dans le groupe constitué par halogène, alkyle en C₁ à C₇, alcoxy en C₁ à C₇, alcényloxy en C₂ à C₇, phénoxy, benzyloxy, trifluorométhyle et S(O)ₘ-R, où m vaut 0, 1 ou 2, et R représente un hydrogène ou un alkyle en C₁ à C₇ ;
ou d'un de ses sels.
caractérisé en ce que
a) dans un composé de formule 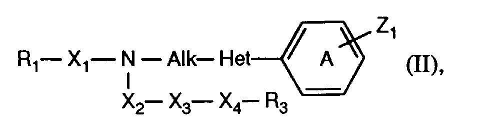 dans laquelle Z₁ représente un radical transformable en R₂ ou l'un de ses sels, on transforme Z₁ en R₂ ; ou
b) on fait réagir un composé de formule 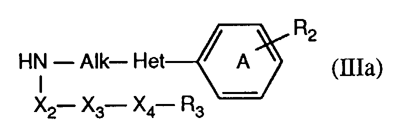 avec un composé de formule R₁-X₁-OH (IIIb), un de ses dérivés réactifs ou un de ses sels ;
et à chaque fois, si on le désire, on isole un composé I obtenu selon le procédé ou d'une autre manière, sous forme libre ou sous forme de sel, et on transforme un composé I obtenu selon le procédé ou d'une autre manière en un autre composé I, on sépare un mélange d'isomères obtenu selon le procédé et on isole l'isomère désiré, et/ou on transforme un composé libre I obtenu selon le procédé en un sel, ou un sel d'un composé I obtenu selon le procédé en le composé I libre ou en un autre sel.

2. Procédé selon la revendication 1 de préparation d'un composé de formule I dans laquelle
R₁ représente un alkyle en C₁ à C₇ qui est non substitué ou substitué par un halogène ou un hydroxy, ou un alcényle en C₂ à C₇, cycloalkyle en C₃ à C₇ ou alcoxy en C₁ à C₇ ; et ses sels.

3. Procédé selon la revendication 1 de préparation d'un composé de formule I dans laquelle
R₁ représente un alkyle en C₂ à C₇, ou un alkyle en C₁ à C₇ qui est substitué par un halogène ou un hydroxy, ou un alcényle en C₃ à C₇, un cycloalkyle en C₃ à C₆, ou un alcoxy en C₁ à C₇ ;
R₂ représente un 1H-tétrazol-5-yle, un carboxy, un alcoxy en C₁ à C₄ - carbonyle ou un halogèn-alcane en C₁ à C₄ - sulfonylamino ;
R₃ représente un 1H-tétrazol-5-yle, un carboxy, un alcoxy en C₁ à C₄ - carbonyle, un alcoxy en C₁ à C₄ - alcoxy en C₁ à C₄ - carbonyle, un phényl-alcoxy en C₁ à C₄ -carbonyle ou un carbamoyle, dont le groupe amino est mono- ou indépendamment disubstitué par un alkyle en C₁ à C₄, ou disubstitué par un alkylène en C₄ à C₆ ou un éthylènoxyéthylène ;
Alk représente un méthylène, éthylène ou éthylidène ;
Het représente 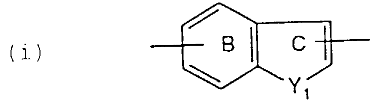 où Y₁ représente O, S ou N(R) et R représente un hydrogène ou un alkyle en C₁ à C₄ ; ou 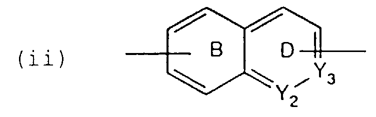
dans laquelle l'une des variables Y₂ et Y₃ représente CH et l'autre représente N, ou les deux variables représentent l'une et l'autre CH ;
X₁ représente -CO- ou -S(O)ₘ- et l'indice m vaut 0, 1 ou 2 ;
l'une des variables X₂ et X₄ représente un alkylène en C₁ à C₄ et l'autre des variables X₂ et X₄ représente une liaison ; ou les deux variables X₂ et X₄ représentent chacune une liaison ;
X₃ représente un cycloalkylidène en C₃ à C₆ ou l'élément de structure -C(X_{A})(X_{b})- et Xₐ représente un hydrogène ou un alkyle en C₁ à C₇ et X_{b} représente un alkyle en C₁ à C₇ ;
et les noyaux A, B, C et D, jusqu'aux substituants indiqués dans la formule, ainsi que les substituants aromatiques, sont indépendamment l'un de l'autre non substitués ou mono- ou polysubstitués par des substituants choisis dans le groupe constitué par halogène, alkyle en C₁ à C₄, alcoxy en C₁ à C₄, alcényloxy en C₃ à C₅, phénoxy, benzyloxy, trifluorométhyle et S(O)ₘ-R, où m vaut 0, 1 ou 2, et R représente un hydrogène ou un alkyle en C₁ à C₄ ;
et ses sels.

4. Procédé selon la revendication 1 de préparation d'un composé de formule I, dans laquelle :
R₁ représente un alkyle en C₂ à C₇ ou un alkyle en C₁ à C₄, qui est substitué par un halogène ou un hydroxy, ou un alcényle en C₃ à C₇, un cycloalkyle en C₃ à C₆ ou un alcoxy en C₁ à C₇ ;
R₂ représente un 1H-tétrazol-5-yle, un carboxy ou un alcoxy en C₁ à C₄ - carbonyle ;
R₃ représente un 1H-tétrazol-5-yle, un carboxy, un alcoxy en C₁ à C₄ - carbonyle, un alcoxy en C₁ à C₄ - alcoxy en C₁ à C₄ - carbonyle, ou un phényl-alcoxy en C₁ à C₂ - carbonyle ;
Alk représente un méthylène, ou encore un éthylène ou un éthylidène ;
Het représente 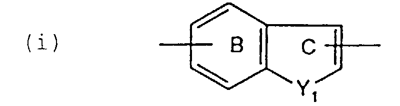 dans laquelle Y₁ représente O, S ou NH ; ou 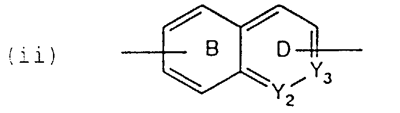 dans laquelle l'une des variables Y₂ et Y₃ représente CH et l'autre représente N, ou les deux variables représentent l'une et l'autre CH ;
en premier lieu 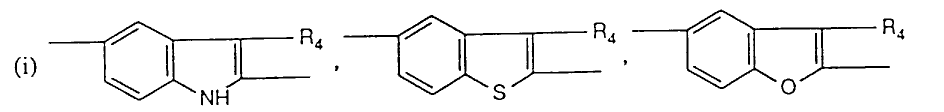 et R₄ représente un hydrogène, un halogène, comme le brome, ou encore un alkyle en C₁ à C₇, un alcoxy en C₁ à C₇ ou un trifluorométhyle ; ou 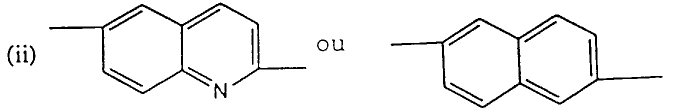
X₁ représente -CO- ;
l'une des variables X₂ et X₄ représente un alkylène en C₁ à C₂, et l'autre des variables X₂ et X₄ représente une liaison ; ou les deux variables X₂ et X₄ représentent l'une et l'autre une liaison ;
X₃ représente un cycloalkylidène en C₅ à C₆ ou l'élément de structure -C(Xₐ) (X_{b})-, et Xₐ représente un hydrogène ou un alkyle en C₁ à C₅, et X_{b} représente un alkyle en C₁ à C₅,
en premier lieu
(i) X₂ représente un alkylène en C₁ à C₂ ; X₄ est une liaison ; X₃ représente un cycloalkylidène en C₅ à C₆ ; ou
(ii) X₂ et X₄ représentent chacun une liaison ; et X₃ représente l'élément de structure -C(Xₐ) (X_{b})-, et X₃ représente un hydrogène ou un alkyle en C₁ à C₅ et X_{b} représente un alkyle en C₁ à C₅ ;
et les noyaux A, B, C et D, jusqu'aux substituants indiqués dans la formule, sont indépendamment l'un de l'autre non substitués ou mono- ou polysubstitués par des substituants choisis dans le groupe constitué par halogène, alkyle en C₁ à C₄, alcoxy en C₁ à C₄ et trifluorométhyle ;
et ses sels.

5. Procédé selon la revendication 1, de préparation d'un composé de formule I dans laquelle l'une des variables Y₂ et Y₃ représente C(R') et l'autre représente N ou C(R'), où R' représente un halogène, un alkyle en C₁ à C₄, un alcoxy en C₁ à C₄ ou un trifluorométhyle, ou un de ses sels.

6. Procédé selon la revendication 1, de préparation d'un composé de formule I, dans laquelle
R₁ représente un alkyle en C₂ à C₇, comme n-propyle ou n-butyle, ou un cycloalkyle en C₃ à C₆, comme cyclopropyle, ou un alcoxy en C₁ à C₄, comme méthoxy, éthoxy, propyloxy ou butyloxy ;
R₂ représente un 1H-tétrazol-5-yle, un carboxy ou un alcoxy en C₁ à C₄ - carbonyle, comme méthoxy- ou éthoxy-carbonyle ;
R₃ représente un carboxy ou un alcoxy en C₁ à C₄ - carbonyle, comme méthoxy-, éthoxy-, ou tert.-butyloxy-carbonyle ;
Alk est un méthylène ;
Het représente 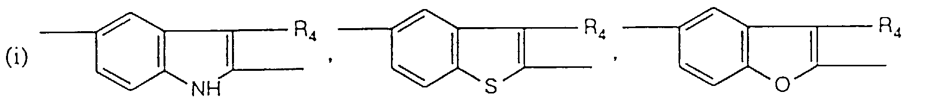 et R₄ représente un hydrogène, un halogène, comme le brome, ou encore un alkyle en C₁ à C₄, comme méthyle, un alcoxy en C₁ à C₄, comme méthoxy, ou trifluorométhyle ; ou 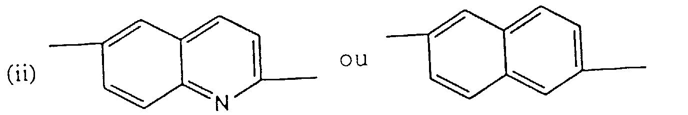 X₁ représente -CO- ;
(i) X₂ représente un alkylène en C₁ à C₂, en particulier un méthylène ; X₄ représente une liaison ; X₃ représente un cycloalkylidène en C₅ à C₆ ; ou
(ii) X₂ et X₄ représentent chacun une liaison ; et X₃ représente l'élément de structure -C (Xₐ) (X_{b})- et Xₐ représente un hydrogène ou un alkyle en C₁ à C₅, comme éthyle, et X_{b} représente un alkyle en C₁ à C₅, comme éthyle ou isopropyle ;
ou d'un de ses sels.

7. Procédé selon la revendication 1, de préparation d'un composé de formule I, dans laquelle
R₃ représente un hydroxyméthyle, un alcoxy en C₁ à C₄ -méthyl- ou un formyle ; les autres variables ont les significations indiquées respectivement ci-dessus ; ou d'un de ses sels.

8. Procédé selon la revendication 1, de préparation d'un composé de formule I, dans laquelle
R₁ représente un alkyle en C₂ à C₅, comme n-propyle ou n-butyle ;
R₂ représente un 1H-tétrazol-5-yle ou un carboxy ;
R₃ représente un carboxy ou un alcoxy en C₁ à C₄ - carbonyle, comme méthoxy-, éthoxy-, ou tert-butyloxy-carbonyle ;
Alk est un méthylène ;
Het est un 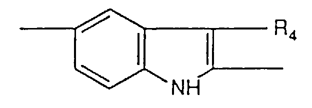 et R₄ représente un hydrogène, un halogène, comme le brome, ou un alkyle en C₁ à C₄, comme le méthyle ;
X₁ représente -CO- ;
(i) X₂ représente un alkylène en C₁ à C₂, en particulier le méthylène ; X₄ représente une liaison ; X₃ représente un cycloalkylidène en C₅ à C₆ ; ou
(ii) X₂ et X₄ représentent chacun une liaison ; et X₃ représente l'élément de structure -C(Xₐ) (X_{b})- et Xₐ représente un hydrogène ou un alkyle en C₁ à C₅, comme éthyle, et X_{b} représente un alkyle en C₁ à C₅, comme éthyle, isopropyle ou 3-butyle ;
ou d'un de ses sels.

9. Procédé selon la revendication 1, de préparation d'un composé de formule I, dans laquelle
R₁ représente un alkyle en C₂ à C₅, comme n-propyle ou n-butyle ;
R₂ représente un 1H-tétrazol-5-yle ou un carboxy ;
R₃ représente un carboxy ou un alcoxy en C₁ à C₄ - carbonyle, comme méthoxy-, éthoxy-, ou tert-butyloxy-carbonyle ;
Alk est un méthylène ;
Het est un 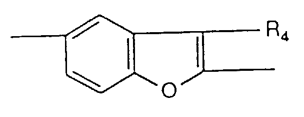 et R₄ représente un hydrogène, un halogène, comme le brome, ou un alkyle en C₁ à C₄, comme le méthyle ;
X₁ représente -CO- ;
(i) X₂ représente un alkylène en C₁ à C₂, en particulier le méthylène ; X₄ représente une liaison ; X₃ représente un cycloalkylidène en C₅ à C₆ ; ou
(ii) X₂ et X₄ représentent chacun une liaison ; et X₃ représente l'élément de structure -C(Xₐ) (X_{b})- et Xₐ représente un hydrogène ou un alkyle en C₁ à C₅, comme éthyle, et X_{b} représente un alkyle en C₁ à C₅, comme éthyle, isopropyle ou 3-butyle ;
ou d'un de ses sels.

10. Procédé selon la revendication 1, de préparation d'un composé de formule I, dans laquelle :
R₁ représente un alkyle en C₂ à C₅, comme n-propyle ou n-butyle ;
R₂ représente un 1H-tétrazol-5-yle ou un carboxy ;
R₃ représente un carboxy ou un alcoxy en C₁ à C₄ - carbonyle, comme méthoxy-, éthoxy-, ou tert-butyloxy-carbonyle ;
Alk est un méthylène ;
Het est un 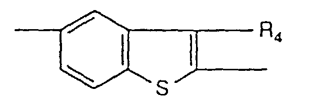 où R₄ représente un hydrogène, un halogène, comme le brome, ou un alkyle en C₁ à C₄, comme le méthyle ;
X₁ représente -CO- ;
(i) X₂ représente un alkylène en C₁ à C₂, en particulier le méthylène ; X₄ représente une liaison ; X₃ représente un cycloalkylidène en C₅ à C₆, comme cyclopentylidène, ou cyclohexylidène ; ou
(ii) X₂ et X₄ représentent chacun une liaison ; et X₃ représente l'élément de structure -C(Xₐ) (X_{b})- et Xₐ représente un hydrogène ou un alkyle en C₁ à C₅, comme éthyle, et X_{b} représente un alkyle en C₁ à C₅, comme éthyle, isopropyle ou 3-butyle ;
ou d'un de ses sels.

11. Procédé selon la revendication 1, de préparation d'un composé de formule I, dans laquelle
R₁ représente un alkyle en C₂ à C₅, comme n-propyle ou n-butyle ;
R₂ représente un 1H-tétrazol-5-yle ou un carboxy ;
R₃ représente un carboxy ou un alcoxy en C₁ à C₄ - carbonyle, comme méthoxy-, éthoxy-, ou tert-butyloxy-carbonyle ;
Alk est un méthylène ;
Het est un 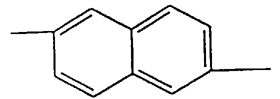
X₁ représente -CO- ;
(i) X₂ représente un alkylène en C₁ à C₂, en particulier le méthylène ; X₄ représente une liaison ; X₃ représente un cycloalkylidène en C₅ à C₆ ; ou
(ii) X₂ et X₄ représentent chacun une liaison ; et X₃ représente l'élément de structure -C(Xₐ) (X_{b})- et Xₐ représente un hydrogène ou un alkyle en C₁ à C₅, comme éthyle, et X_{b} représente un alkyle en C₁ à C₅, comme éthyle, isopropyle ou 3-butyle ;
ou d'un de ses sels.

12. Procédé selon la revendication 1, de préparation d'un composé de formule I, dans laquelle
R₁ représente un alkyle en C₂ à C₅, comme n-propyle ou n-butyle ;
R₂ représente un 1H-tétrazol-5-yle ou un carboxy ;
R₃ représente un carboxy ou un alcoxy en C₁ à C₄ - carbonyle, comme méthoxy-, éthoxy-, ou tert-butyloxy-carbonyle ;
Alk est un méthylène ;
Het est un 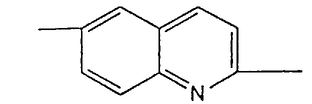
X₁ représente -CO- ;
(i) X₂ représente un alkylène en C₁ à C₂, en particulier le méthylène ; X₄ représente une liaison ; X₃ représente un cycloalkylidène en C₅ à C₆ ; ou
(ii) X₂ et X₄ représentent chacun une liaison ; et X₃ représente l'élément de structure -C(Xₐ)(X_{b})- et Xₐ représente un hydrogène ou un alkyle en C₁ à C₅, comme éthyle, et X_{b} représente un alkyle en C₁ à C₅, comme éthyle, isopropyle ou 3-butyle ;
ou d'un de ses sels.

13. Procédé selon la revendication 1, de préparation d'un composé de formule I, dans laquelle
R₁ représente un alkyle en C₂ à C₅, comme n-propyle ou n-butyle ;
R₂ représente un 1H-tétrazol-5-yle ou un carboxy ;
R₃ représente un carboxy ou un alcoxy en C₁ à C₄ - carbonyle, comme méthoxy-, éthoxy-, ou tert-butyloxy-carbonyle ;
Alk est un méthylène ;
Het est un 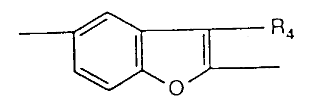 et R₄ représente un hydrogène ou un halogène dont le nombre d'atomes va jusqu'à 35 compris, comme le brome ;
X₁ représente -CO- ;
(i) X₂ représente un alkylène en C₁ à C₂, en particulier le méthylène ; X₄ représente une liaison ; X₃ représente un cycloalkylidène en C₅ à C₆ ; ou
(ii) X₂ et X₄ représentent chacun une liaison ; et X₃ représente l'élément de structure -C(Xₐ) (X_{b})- et Xₐ représente un hydrogène ou un alkyle en C₁ à C₅, comme éthyle, et X_{b} représente un alkyle en C₁ à C₅, comme éthyle, isopropyle ou 3-butyle ;
ou d'un de ses sels.

14. Procédé selon la revendication 1, de préparation d'un composé de formule I choisi parmi les composés suivants :
N-[2-(2'-(1H-tétrazol-5-yl)-phényl)-quinoléin-6-yl-méthyl]-N-valéroyl-valine ;
Acide N-[2-(2'-(1H-tétrazol-5-yl-phényl)-quinoléin-6-yl-méthyl]-N-valéroyl-1-aminométhyl-cyclopentane-1-carboxylique ;
Acide N-[2-(2'-(1H-tétrazol-5-yl)-phényl)-quinoléin-6-yl-méthyl]-N-valéroyl-2-aminométhyl-2-éthyl-butyrique ;
Acide N-[2-(2'-(1H-tétrazol-5-yl)-phényl)-benzo[b]-thiophén-5-yl-méthyl]-N-valéroyl-2-aminométhyl-2-éthyl-butyrique ;
N-[3-bromo-2-(2'-carboxy-phényl)-benzo[b]-thiophén-5-yl-méthyl]-N-valéroyl-valine ;
Acide N-[2-(2'-(1H-tétrazol-5-yl)-phényl)-benzofuran-5-yl-méthyl]-N-valéroyl-2-aminométhyl-2-éthyl-butyrique ;
Acide N-[2-(2'-(1H-tétrazol-5-yl)-phényl-benzofuran-5-yl-méthyl]-N-valéroyl-1-aminométhyl-cyclopentane-1-carboxylique ;
N-[2-(2'-(1H-tétrazol-5-yl)-phényl)-benzofuran-5-yl-méthyl]-N-valéroyl-valine ;
Acide N-[2-(2'-(1H-tétrazol-5-yl)-phényl)-5-yl-méthyl-benzo[b]thiophén]-N-valéroyl-1-aminométhyl-cyclopentan-1-carboxylique ;
N-[2-(2'-(1H-tétrazol-5-yl)-phényl-benzo[b]-thiophén-5-yl-méthyl]-N-valéroyl-valine ;
Acide N-[3-bromo-2-(2'-carboxyphényl)-benzo[b]-thiophén-5-yl-méthyl]-N-valéroyl-1-aminométhyl-cyclopentane-1-carboxylique; et
Acide N-[2-(2'-(1H-tétrazol-5-yl)-phényl)-benzofuran-5-yl-méthyl]-N-valéroyl-1-aminométhyl-cyclohexane-1-carboxylique ;
N-[2-(2'-(tétrazol-5-yl)-phényl)-naphtalène-6-yl-méthyl]-N-valéroyl-valine ;
Acide N-[2-(2'-(tétrazol-5-yl)-phényl)-naphtalène-6-yl-méthyl]-N-valéroyl-1-aminométhyl-cyclopentane-1-carboxylique ;
Acide N-[2-(2'-(tétrazol-5-yl)-phényl)-naphtalène-6-yl-méthyl]-N-valéroyl-2-aminométhyl-2-éthyl-butyrique ;
Acide N-[2-(2'-(tétrazol-5-yl)-phényl)-naphtalène-6-yl-méthyl]-N-valéroyl-1-aminométhyl-cyclohexane-1-carboxylique ;
N-[2-(2'-(tétrazol-5-yl)-phényl)-quinoléin-6-yl-méthyl]-N-cyclopropylcarbonyl-valine ;
Acide N-[2-(2'-(tétrazol-5-yl)-phényl)-quinoléin-6-yl-méthyl]-N-cyclopropylcarbonyl-1-aminométhyl-cyclopentane-1-carboxylique ;
Acide N-[2-(2'-(tétrazol-5-yl)-phényl)-quinoléin-6-yl-méthyl]-N-cyclopropylcarbonyl-2-aminométhyl-2-éthylbutyrique ;
N-[2-(2'-(tétrazol-5-yl)-phényl)-quinoléin-6-yl-méthyl]-N-cyclopropylcarbonyl-valine ;
ou un de leurs sels.

15. Procédé selon la revendication 1, de préparation d'un composé de formule I choisi parmi les suivants :
Ester éthylique de l'acide N-[2-(2'-(1H-tétrazol-5-yl)-phényl)-naphtalène-6-ylméthyl]-N-valéroyl-1-aminométhyl-cyclopentane-1-carboxylique ;
Acide N-[2-(2'-(1H-tétrazol-5-yl)-phényl)-naphtalène-6-ylméthyl]-N-valéroyl-1-aminométhyl-cyclopentane-1-carboxylique;
Acide N-[2-(2'-(1H-tétrazol-5-yl)-phényl-naphtalène-6-ylméthyl]-N-valéroyl-3-amino-2,2-diméthylpropionique ;
N-[2-(2'-(1H-tétrazol-5-yl)-phényl)-naphtalène)-6-yl-méthyl]-N-butyryl-valine ; et
N-[2-(2'-(1H-tétrazol-5-yl)-phényl)-naphtalène)-6-ylméthyl]-N-valéroyl-valine;
ou un de leurs sels.

16. Procédé selon la revendication 1, de préparation d'un composé de formule I choisi parmi les suivants :
Ester éthylique de l'acide N-{3-bromo-2-[2'-(1H-tétrazol-5-yl)-phényl]-5-ylméthyl-benzofuran}-N-valéroyl-1-aminométhyl-cyclopentane-1-carboxylique ;
Ester éthylique de l'acide N-{3-bromo-2-[2'-(1H-tétrazol-5-yl)-phényl]-5-ylméthyl-benzofuran}-N-butyryl-1-aminométhyl-cyclopentane-1-carboxylique ;
Ester éthylique de l'acide N-{3-bromo-2-[2'-(1H-tétrazol-5-yl)-phényl]-5-ylméthyl-benzofuran}-N-caproyl-1-aminométhyl-cyclopentane-1-carboxylique ;
Ester éthylique de N-{3-bromo-2-[2'-(1H-tétrazol-5-yl)-phényl]-5-ylméthyl-benzofuran}-N-valéroyl-valine ;
Ester éthylique de N-{3-bromo-2-[2'-(1H-tétrazol-5-yl)phényl]-5-ylméthyl-benzofuran}-N-butyryl-valine ;
Ester éthylique de N-{3-bromo-2-[2'-(1H-tétrazol-5-yl)phényl]-5-ylméthyl-benzofuran}-N-caproyl-valine ;
Ester éthylique de N-{3-bromo-2-[2'-(1H-tétrazol-5-yl)phényl]-5-ylméthyl-benzofuran}-N-valéroyl-alanine ;
Acide N-{3-bromo-2-[2'-(1H-tétrazol-5-yl)phényl]-5-ylméthyl-benzofuran}-N-valéroyl-1-aminométhyl-cyclopentane-1-carboxylique ;
Acide N-{3-bromo-2-[2'-(1H-tétrazol-5-yl)phényl]-5-ylméthyl-benzofuran}-N-butyryl-1-aminométhyl-cyclopentan-1-carboxylique ;
Acide N-{3-bromo-2-[2'-(1H-tétrazol-5-yl)phényl]-5-ylméthyl-benzofuran}-N-caproyl-1-aminométhyl-cyclopentane-1-carboxylique ;
N-{3-bromo-2-[2'-(1H-tétrazol-5-yl)phényl]-5-ylméthyl-benzofuran}-N-valéroyl-valine ;
N-{3-bromo-2-[2'-(1H-tétrazol-5-yl)phényl]-5-ylméthyl-benzofuran}-N-butyryl-valine ;
N-{3-bromo-2-[2'-(1H-tétrazol-5-yl)phényl]-5-ylméthyl-benzofuran}-N-caproyl-valine ; et
N-{3-bromo-2-[2'-(1H-tétrazol-5-yl)phényl]-5-ylméthyl-benzofuran}-N-valéroyl-alanine ;
ou un de leurs sels.

17. Procédé selon la revendication 1, de préparation d'un composé de formule I choisi parmi les suivants :
Ester éthylique de l'acide N-{3-bromo-2-[2'-(1H-tétrazol-5-yl)phényl]-benzofuran-5-ylméthyl}-N-valéroyl-1-aminométhyl-cyclopentane-1-carboxylique ;
Ester éthylique de l'acide N-{3-bromo-2-[2'-(1H-tétrazol-5-yl)-phényl]-benzofuran-5-ylméthyl}-N-butyryl-1-amino-méthylcyclopentane-1-carboxylique ;
Ester éthylique de l'acide N-{3-bromo-2-[2'-(1H-tétrazol-5-yl)-phényl]-benzofuran-5-ylméthyl}-N-caproyl-1-aminométhyl-cyclopentane-1-carboxylique ;
Ester éthylique de N-{3-bromo-2-[2'-(1H-tétrazol-5-yl)-phényl]-benzofuran-5-ylméthyl}-N-valéroyl-valine ;
Ester éthylique de N-{3-bromo-2-[2'-(1H-tétrazol-5-yl)-phényl]-benzofuran-5-ylméthyl}-N-butyryl-valine ;
Ester éthylique de N-{3-bromo-2-[2'-(1H-tétrazol-5-yl)-phényl]-benzofuran-5-ylméthyl}-N-caproyl-valine ;
Ester éthylique de N-{3-bromo-2-[2'-(1H-tétrazol-5-yl)-phényl]-benzofuran-5-ylméthyl}-N-valéroyl-alanine ;
Acide N-{3-bromo-2-(2'-(1H-tétrazol-5-yl)-phényl]-benzofuran-5-ylméthyl}-N-valéroyl-1-aminométhyl-cyclopentane-1-carboxylique ;
Acide N-{3-bromo-2-[2'-(1H-tétrazol-5-yl)-phényl]-benzofuran-5-ylméthyl}-N-butyryl-1-aminométhyl-cyclopentane-1-carboxylique ,
Acide N-{3-bromo-2-[2'-(1H-tétrazol-5-yl)-phényl]-benzofuran-5-ylméthyl}-N-caproyl-1-aminométhyl-cyclopentane-1-carboxylique ;
N-{3-bromo-2-[2'-(1H-tétrazol-5-yl)-phényl]-benzofuran-5-ylméthyl}-N-valéroyl-valine ;
N-{3-bromo-2-[2'-(1H-tétrazol-5-yl)-phényl]-benzofuran-5-ylméthyl}-N-butyryl-valine ;
N-{3-bromo-2-[2'-(1H-tétrazol-5-yl)-phényl]-benzofuran-5-ylméthyl}-N-caproyl-valine ; et
N-{3-bromo-2-[2'-(1H-tétrazol-5-yl)-phényl]-benzofuran-5-ylméthyl}-N-valéroyl-alanine,
ou un de leurs sels.

18. Procédé de préparation d'une préparation pharmaceutique, caractérisé en ce qu'on mélange un composé selon l'une des revendications 1 - 18 ou un de ses sels pharmaceutiquement acceptables avec le cas échéant des additifs et/ou supports pharmaceutiquement acceptables.

19. Application d'un composé selon l'un des revendications 1 - 17 ou d'un de ses sels pharmaceutiquement acceptables, à la préparation d'une préparation pharmaceutique pour le traitement de l'hypertension artérielle et de l'insuffisance cardiaque ainsi que du glaucome.
